# EUROPEAN PATENT SPECIFICATION

(11) **EP 3 908 291 B1**
(45) Date of publication and mention of the grant of the patent: **22.07.2026**
(21) Application number: 20737913.2
(22) Date of filing: 06.01.2020
(51) Int. Cl.: A61K 47/55, A61K 35/17, C12N 9/22, C07K 14/82, A61K 38/00, A61P 35/00, C12N 9/10, C12N 9/90, C07K 14/725, C12N 5/0783

(54) **SMALL MOLECULE DEGRADERS OF FKBP12 VIA RECRUITMENT OF VON HIPPEL-LINDAU E3 UBIQUITIN LIGASE (VHL) E3 UBIQUITIN LIGASE, AND USES IN DTAG SYSTEMS**
NIEDERMOLEKULARE DEGRADER VON FKBP12 ÜBER REKRUTIERUNG DER VON-HIPPEL-LINDAU-E3-UBIQUITIN-LIGASE (VHL) UND VERWENDUNG IN DTAG-SYSTEMEN
AGENTS DE DÉGRADATION DE PETITES MOLÉCULES DE FKBP12 PAR RECRUTEMENT DE L'UBIQUITINE LIGASE E3 DE VON HIPPEL-LINDAU (VHL), ET UTILISATIONS DANS DES SYSTÈMES DTAG

(30) Priority: 07.01.2019 US 201962789230 P
(43) Date of publication of application: 17.11.2021
(73) Proprietor: Dana-Farber Cancer Institute, Inc., Boston, MA 02215 (US)
(72) Inventor: GRAY, Nathanael S., Boston, Massachusetts 02130 (US); FERGUSON, Fleur M., Cambridge, Massachusetts 02139 (US); NABET, Behnam, Boston, Massachusetts 02135 (US); BUCKLEY, Dennis L., Cambrdige, Massachusetts 02139 (US)
(74) Representative: Haseltine Lake Kempner LLP
(86) International application number: PCT/US2020/012348
(87) International publication number: WO 2020/146250

(56) References cited:
- WO-A1-2017/024318
- WO-A1-2017/180417
- WO-A1-2018/148440
- WO-A1-2018/148443
- WO-A2-2017/024317
- WO-A2-2017/024317
- DANIEL P. BONDESON ET AL: "Lessons in PROTAC Design from Selective Degradation with a Promiscuous Warhead", CELL CHEMICAL BIOLOGY, vol. 25, no. 1, 1 January 2018 (2018-01-01), AMSTERDAM, NL, pages 78 - 87, XP055561974, ISSN: 2451-9456, DOI: 10.1016/j.chembiol.2017.09.010
- SU SHANG ET AL: "Potent and Preferential Degradation of CDK6 via Proteolysis Targeting Chimera Degraders", JOURNAL OF MEDICINAL CHEMISTRY, vol. 62, no. 16, 22 August 2019 (2019-08-22), US, pages 7575 - 7582, XP055882578, ISSN: 0022-2623, Retrieved from the Internet <URL:https://pubs.acs.org/doi/pdf/10.1021/acs.jmedchem.9b00871> DOI: 10.1021/acs.jmedchem.9b00871
- ASHTON C. LAI ET AL: "Modular PROTAC Design for the Degradation of Oncogenic BCR-ABL", ANGEWANDTE CHEMIE, vol. 55, no. 2, 11 January 2016 (2016-01-11), Hoboken, USA, pages 807 - 810, XP055734239, ISSN: 1433-7851, DOI: 10.1002/anie.201507634
- LI XIN ET AL: "Proteolysis-targeting chimera (PROTAC) for targeted protein degradation and cancer therapy", JOURNAL OF HEMATOLOGY & ONCOLOGY, vol. 13, no. 1, 13 May 2020 (2020-05-13), London UK, pages 50 - 1, XP093006857, ISSN: 1756-8722, Retrieved from the Internet <URL:https://jhoonline.biomedcentral.com/counter/pdf/10.1186/s13045-020-00885-3.pdf?pdf=button%20sticky> DOI: 10.1186/s13045-020-00885-3
- DATABASE REAXYS [online] Elsevier; 16 August 2018 (2018-08-16), BUCKLEY D: "TUNABLE ENDOGENOUS PROTEIN DEGRADATION WITH HETEROBIFUNCTIONAL COMPOUNDS - WO2018/148443 A1", XP055952184, Database accession no. XRN = 33551764
- DATABASE CAPLUS [online] 1 January 2017 (2017-01-01), BRADNER JAMES ;: "Using heterobifunctional compounds for targeted CAR protein degradation to attenuate adoptive immunotherapy associated adverse inflammation - WO 2017024318 A1", XP055952197, retrieved from ACS Database accession no. 2017:229794
- DATABASE CAPLUS [online] 1 January 2017 (2017-01-01), BRADNER JAMES ;: "Using heterobifunctional compounds for targeted CAR protein degradation to attenuate adoptive immunotherapy associated adverse inflammation - WO 2017024318 A1", XP055952509, retrieved from ACS Database accession no. 2017:229794
- DATABASE CAPLUS [online] 1 January 2017 (2017-01-01), BRADNER JAMES: "Methods to induce targeted protein degradation through bifunctional molecules - WO 2017024317 A2", XP055952510, retrieved from ACS Database accession no. 2017:229795
- PHILIPP OTTIS ET AL: "Assessing Different E3 Ligases for Small Molecule Induced Protein Ubiquitination and Degradation", ACS CHEMICAL BIOLOGY, vol. 12, no. 10, 5 September 2017 (2017-09-05), pages 2570 - 2578, XP055618965, ISSN: 1554-8929, DOI: 10.1021/acschembio.7b00485
- DATABASE CAPLUS [online] 1 January 2018 (2018-01-01), BRADNER JAMES: "Regulating CAR-cells against inflammatory side effects by targeted CAR protein degradation through the use of ubiquitin ligase binding heterobifunctional compounds - WO 2018148440 A1", XP055952511, retrieved from ACS Database accession no. 2018:1504369
- NABET, B ET AL.: "The dTAG system for immediate and target-specific protein degradation", NATURE CHEMICAL BIOLOGY, vol. 14, no. 5, May 2018 (2018-05-01), pages 431 - 441, XP036478801
- MANIACI, C ET AL.: "Homo-PROTACs: bivalent small-molecule dimerizers of the VHL E3 ubiquitin ligase to induce self-degradation", NATURE COMMUNICATIONS, 10 October 2017 (2017-10-10), pages 1 - 14, XP055476041

## Description

### RELATED APPLICATIONS

This application claims the benefit of priority under 35 U.S.C. § 119(e) to U.S. Provisional Application No: 62/789,230, filed January 7, 2019.

### BACKGROUND OF THE INVENTION

Genetic mechanisms for modulating abundance of gene products such as RNA interference and Clustered Regularly Interspaced Short Palindromic Repeats (CRISPR)-Cas9 genome editing have become powerful tools for determining the consequences of functional loss or gain of a target gene. However, these methodologies are limited from the standpoint of being able to assess acute changes in protein function, particularly with respect to proteins that are required for cell growth and survival. Chemical-based methodologies have been developed as well. Winter, et al., Science 348:1376-81(2015), reported construction of the first small molecule bifunctional degraders and use thereof to selectively degrade the bromodomain and extraterminal domain (BET) bromodomain transcriptional co-activator bromo domain-containing protein 2 (BRD2), BRD3 and BRD4 by a cell-permeable heterobifunctional degrader that bridges these co-activators to the E3 ubiquitin ligase cereblon (CRBN). This use of small molecule degraders has been shown to be advantageous in that it allows rapid and target-specific turnover without degradation of the heterobifunctional degraders. On the other hand, this approach has been found to be limited in that it requires the up-front identification of a target-selective ligand as a prerequisite for specific protein for degradation.

More recently, Nabet et al., Nat. Chem. Biol. 14:431-41(2018), reported a relatively generalizable approach that provides rapid degradation of allele-specific protein chimeras for biological investigation and early target validation. Nabet's degradation tag (dTAG) system is a hybrid chemical/biological system that leverages the potency of cell-permeable heterobifunctional degraders, including CRBN E3 ligase mediated degraders of FKBP12^{F36V} tagged proteins.

WO 2018/148443 A1 provides a means to modulate gene expression *in vivo* in a manner that avoids problems associated with CRISPR endogenous protein knock-out or knock-in strategies and strategies that provide for correction, or alteration, of single nucleotides. This includes inserting into the genome a nucleotide encoding a heterobifunctional compound targeting protein (dTAG) in-frame with the nucleotide sequence of a gene encoding an endogenously expressed protein of interest which, upon expression, produces an endogenous protein-dTAG hybrid protein. This allows for targeted protein degradation of the dTAG and the fused endogenous protein using a heterobifunctional compound.

WO 2017024318 A1 discloses heterobifunctional compounds for targeted CAR protein degradation to attenuate adoptive immunotherapy associated adverse inflammation.

WO 2017/024317 A2 provides bifunctional compounds which act as protein degradation inducing moieties, as well as nucleic acids, polypeptides, cells, and methods for highly regulated, targeted degradation of proteins through the use of the bifunctional compounds

P. Ottis et al, ACS Chemical Biology, vol. 12, no. 10, 2017, pages 2570-2578, discloses different E3 ligases for small molecule induced protein ubiquitination and degradation.

WO 2018/148440 A1 relates to compositions and methods for regulating chimeric antigen receptor immune effector cell, for example T-cell (CAR-T) therapy, to modulate associated adverse inflammatory responses, for example, cytokine release syndrome and tumor lysis syndrome, using targeted protein degradation

### SUMMARY OF THE INVENTION

The invention is as defined in the appended claims.

A first aspect of the present invention is directed to a bifunctional compound also referred to herein as a degrader, having a structure represented by formula I: 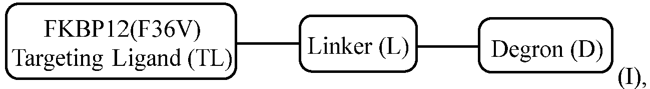
wherein the targeting ligand has a structure represented by formula TL-1: 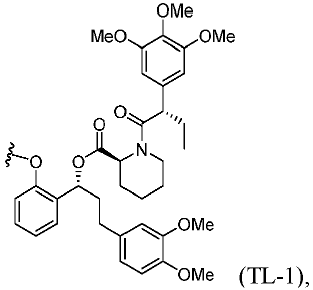 wherein the linker is an alkylene chain which may be interrupted by, and/or terminate (at either or both termini) at least one of -O-, -S-, -N(R')-, -C≡C-, -C(O)-, -C(O)O-, -OC(O)-, -OC(O)O-, -C(NOR')-, -C(O)N(R')-, -C(O)N(R')C(O)-, -C(O)N(R')C(O)N(R')-, -N(R')C(O)-,-N(R')C(O)N(R')-, -N(R')C(O)O-, -OC(O)N(R')-, -C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N(R')-,-N(R')S(O)N(R')-, C₃-C₁₂ carbocyclene, 3- to 12-membered heterocyclene, 5- to 12-membered heteroarylene or any combination thereof, wherein R' is H or C₁-C₆ alkyl, wherein the interrupting and the one or both terminating groups may be the same or different, or
a polyethylene glycol chain which may be interrupted by, and/or terminate (at either or both termini) at least one of -S-, -N(R')-, -C≡C-, -C(O)-, -C(O)O-, -OC(O)-, -OC(O)O-, - C(NOR')-, -C(O)N(R')-, -C(O)N(R')C(O)-, -C(O)N(R')C(O)N(R')-, -N(R')C(O)-,-N(R')C(O)N(R')-, -N(R')C(O)O-, -OC(O)N(R')-, -C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N(R')-,-N(R')S(O)N(R')-, C₃₋₁₂ carbocyclene, 3- to 12-membered heterocyclene, 5- to 12-membered heteroarylene or any combination thereof, wherein R' is H or C₁-C₆ alkyl, wherein the one or both terminating groups may be the same or different, or represented by structure L10: 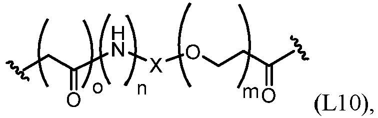
wherein m is an integer of 0-8;
X is absent or C1 to 14 alkyl;
n is 0 or 1; and
o is 0 or 1; and
wherein the degron has a structure represented by formula D1 or D2: 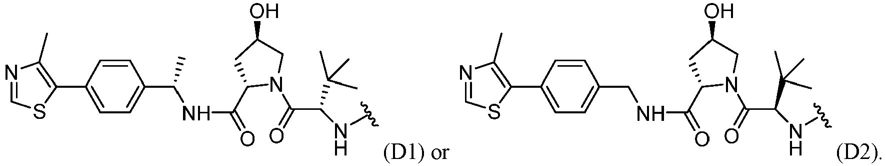 The targeting ligand represents a moiety that selectively binds a FKBP12(F36V)-tagged protein, the degron represents a ligand that selectively binds a Von Hippel-Lindau E3 ubiquitin ligase (VHL), and the linker represents a moiety that covalently connects the degron and the targeting ligand or a pharmaceutically acceptable salt or stereoisomer thereof.

Another aspect of the present invention is the bifunctional compound disclosed herein or a pharmaceutically acceptable salt or stereoisomer thereof, for use in a method of degrading a CAR protein, comprising:
administering to a subject an effective amount of the bifunctional compound, wherein the subject has previously been treated with allogeneic or autologous immune effector cells that express a nucleic acid encoding a fusion protein comprising the CAR and a dTAG FKBP12(F36V), wherein the fusion protein comprises, from N-terminus to C-terminus:
a) an extracellular ligand binding domain that binds a tumor associated antigen;
b) a transmembrane domain;
c) a cytoplasmic domain comprising at least one intracellular signaling domain; and
d) a dTAG FKBP12(F36V) of any one of SEQ ID NOs: 1-4 which is disposed at the N-terminus or between the extracellular binding domain and the transmembrane domain, provided that there is no disruption to antigen binding or insertion into the membrane; or at the C-terminus, between the transmembrane domain and the intracellular domain or between signaling domains when more than one is present, provided that there is no disruption to intracellular signaling or insertion into the membrane.

Another aspect of the present invention is the bifunctional compound disclosed herein for use in a method of identifying protein function, comprising genetically modifying a cell by introducing an exogenous nucleic acid comprising a sequence that encodes FKBP12(F36V) at a genetic locus of an endogenous protein, wherein the thus modified locus expresses the protein with FKBP12(F36V) as an in-frame N-terminal or C-terminal fusion; contacting the modified cell with the bifunctional compound; and detecting a change in the modified cell relative to an unmodified cell

Also disclosed herein is a degradation tag (dTAG) system comprising the protein FKBP12 (F36V) that binds the targeting ligand, or a nucleic acid encoding the dTAG. The dTAG is utilized as an expression product formed *in vivo* or *in vitro* that is in the form of a fusion protein with a protein of interest (that is intended to be degraded). The other component of the system is the bifunctional compound. Without intending to be bound by any particular theory of operation, when the dTAG and the degrader are brought into contact, the dTAG is bound by the TL, which brings the fusion protein into close proximity with the degron, which results in its ubiquitination and degradation by cellular proteasomes.

Also disclosed herein is the dTAG and the bifunctional compound for use in methods of using the dTag and the bifunctional compound, which may include both clinical and pre-clinical purposes.

In some embodiments, the method includes genetically modifying a cell by introducing an exogenous nucleic acid comprising a sequence that encodes a fusion protein comprising a mutated form of a protein that is endogenous to the cell and FKBP(F36V) fused in frame to the N-terminus or the C-terminus of the mutated protein; contacting the modified cells with a heterobifunctional compound comprising a targeting ligand that binds FKBP12(F36V) linked via a linker to a degron that binds VHL ubiquitin ligase; and determining a change in a property of the modified cell before and after said contacting with the heterobifunctional compound. In some aspects, the methods include modifying expression of a polynucleotide in a eukaryotic cell by introducing a nucleic acid encoding a dTAG.

*In vivo* methods may include genetically modifying a cell by introducing an exogenous nucleic acid comprising a sequence that encodes FKBP12(F36V) at a genetic locus of an endogenous protein, wherein the thus modified locus expresses the protein with FKBP12(F36V) as an in-frame N-terminal or C-terminal fusion; introducing the thus modified cells into a non-human animal such as a rodent; administering to the animal (*e.g.*, rodent) a heterobifunctional compound comprising a targeting ligand that binds FKBP12(F36V) covalently linked via a linker to a degron that binds VHL tumor suppressor; and detecting a change in a property of the non-human animal relative to an unmodified non-human animal. The cells may be autologous or non-autologous to the non-human animal. In some embodiments, the cell may be a human cell, *e.g.,* a human cancer cell line or a non-cancerous cell line (for other non-cancerous conditions).

Also disclosed herein are immune effector cells which have been genetically modified to express a chimeric antigen receptor protein (CAR)-dTAG fusion protein for use in methods, which may be used clinically, wherein the method includes administering to a subject a therapeutically effective number of the immune effector cells ,such as autologous or allogeneic T-cells (CAR-T cells), . In the event the patient experiences an adverse immune response (*e.g.*, cytokine release syndrome or neurotoxicity) as a result of the therapy, also disclosed herein is the heterobifunctional compound for use in the method, wherein the patient is administered the heterobifunctional compound which will result in degradation of the CAR-dTAG fusion protein.

Currently available molecules for degradation of FKBP12(F36V)-tagged proteins exclusively recruit the cereblon (CRBN) E3 ligase. They are limited in that the CRBN ligase does not ubiquitinate all proteins of interest, nor is it expressed or activated in all cell types. Thus, an overriding advantage of the present invention is that it overcomes limitations associated with CRBN-based dTAG systems, and thus expands upon the current methods and provides a more universal system for targeting and degrading target proteins and studying their effects on cells. For example, various oncogene expression products such as EWS/Friend leukemia integration 1 transcription factor (FLI1), are not amenable to degradation with CRBN-based dTAG systems but can be degraded by the present dTAG system.

The present invention provides several other advantages. The dTAG system disclosed herein possesses favorable pharmacokinetic (PK) properties as compared to dTAG systems based on CRBN E3 ligase recruitment, which greatly facilitates in vivo use. The present methods may also include pairing with a CRBN-based dTAG system which enables study of two proteins simultaneously in the same cell line or animal model. The dTAG VHL E3 ligase recruiting and CRBN E3 ligase recruiting degrader molecules (dTAG or endogenous degraders) can be paired and used to degrade two proteins in the same cell line or animal model. Such studies include combinatorial synergy studies. Further, the dTAG system of the present disclosure may be used to conduct high-throughput screening studies wherein the degraders function as a benchmark for effective degradation or as a reporter output (*e.g.*, GFP/RFP/luciferase). In yet other embodiments, the methods may also be used to provide tunable control of Clustered Regularly Interspaced Short Palindromic Repeats (CRISPR) associated protein 9 (Cas9).

### BRIEF DESCRIPTION OF THE DRAWINGS

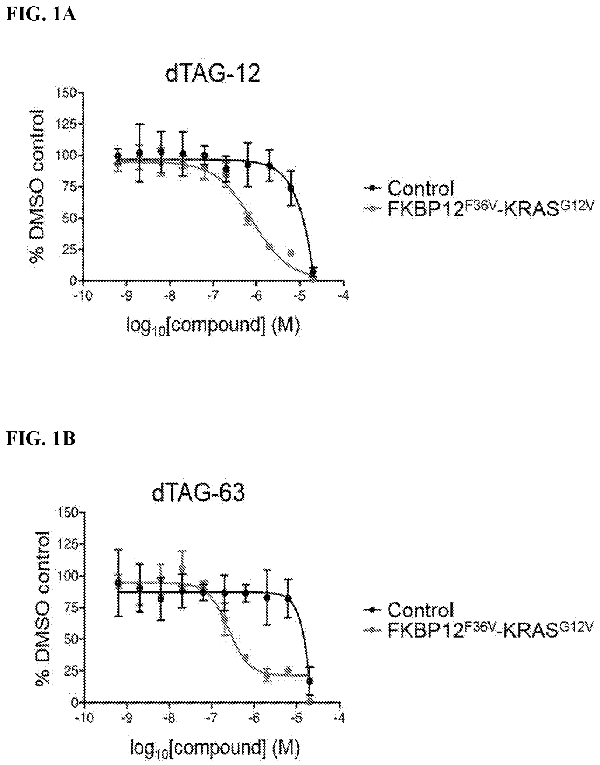

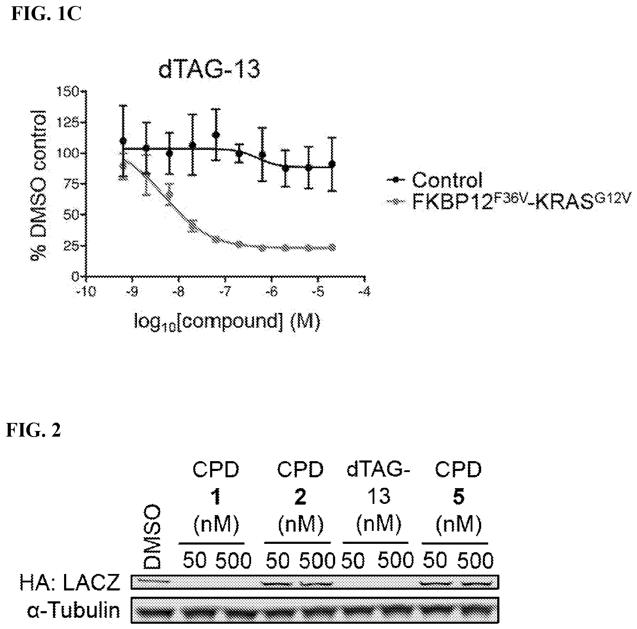

FIG. 1A.-FIG.1C are graphs that show the loss of cell viability upon degradation of mutant KRAS in NIH/3T3 cells (expressed in % DMSO control) as a function of the log of the concentration of dTAG-12 (FIG. 1A), dTAG-63 (FIG. 1B), and dTAG-13 (FIG. 1C) via ATPlite^{™} assay. Effective loss of viability is observed with dTAG-12, dTAG-63 and dTAG-13.
FIG. 2 is a photograph of an immunoblot that shows the rapid degradation of LACZ (control) in a PATU-8902 LACZ-FKBP12^{F36V} clone by inventive compounds **1** and dTAG-13. Lack of degradation was observed with negative control compounds **2** and **5.**

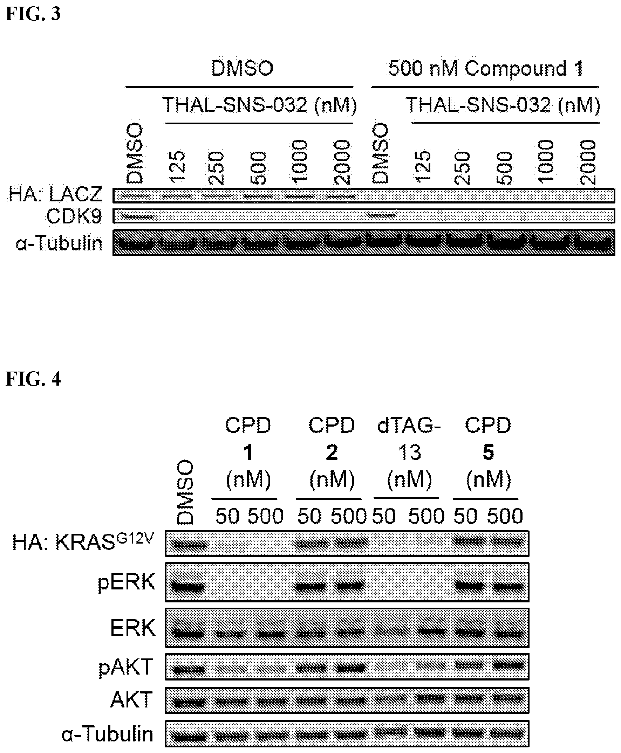

FIG. 3 is a photograph of an immunoblot that shows the degradation of LACZ (control) upon recruitment of VHL by inventive compound **1** in combination with degradation of CDK9 with THAL-SNS-032 upon recruitment of CRBN in a PATU-8902 LACZ-FKBP12^{F36V} clone.
FIG. 4 is a photograph of an immunoblot that shows the degradation of mutant KRAS in a PATU-8902 FKBP12^{F36V}-KRAS^{G12V}; *KRAS*^{*-*/*-*} clone leading to rapid loss of downstream signaling pathway activation by inventive compounds **1** and dTAG-13. Lack of degradation was observed with negative control compounds **2** and **5.**

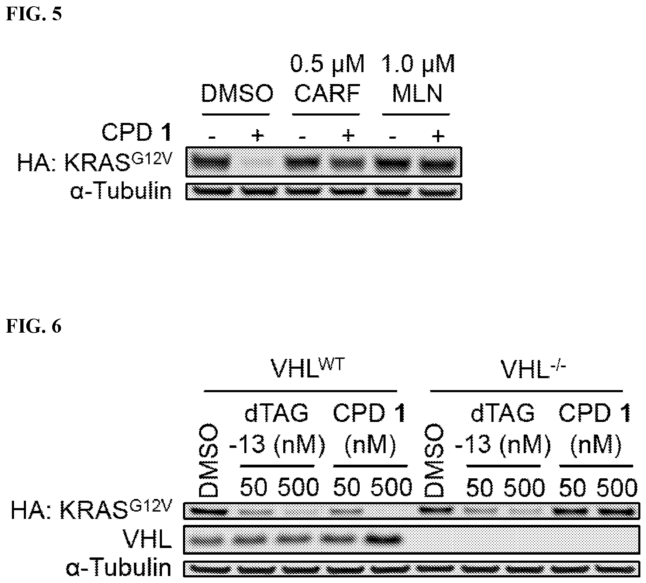

FIG. 5 is a photograph of an immunoblot that shows the degradation of mutant KRAS in a PATU-8902 FKBP12^{F36V}-KRAS^{G12V}; *KRAS*^{*-*/*-*} clone by inventive compound **1** that was rescued upon pre-treatment with Carflizomib (CARF) or MLN4924 (MLN).
FIG. 6 is a photograph of an immunoblot that shows the degradation of mutant KRAS in 293T^{WT} FKBP12^{F36V}-KRAS^{G12V} cells by inventive compound **1** and dTAG-13. Degradation of mutant KRAS was observed in 293T^{VHL-/-} FKBP12^{F36V}-KRAS^{G12V} cells by dTAG-13 but not by inventive compound **1.**

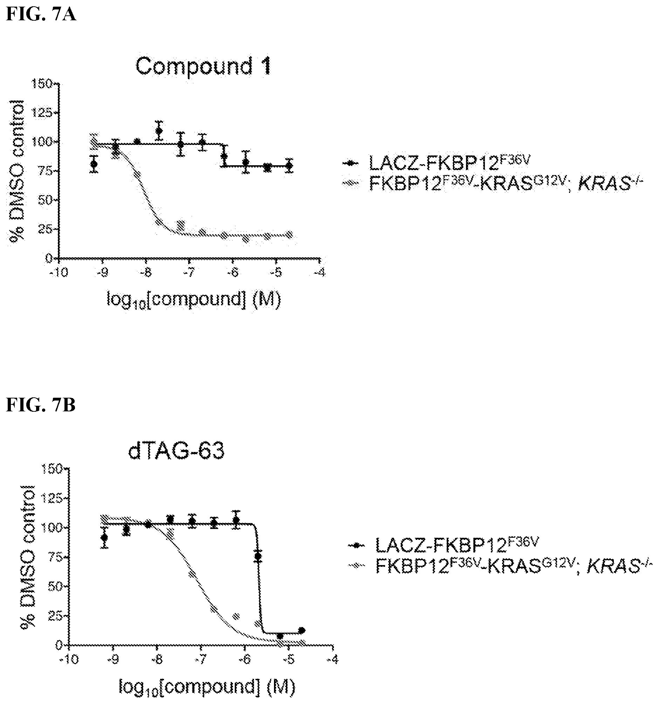

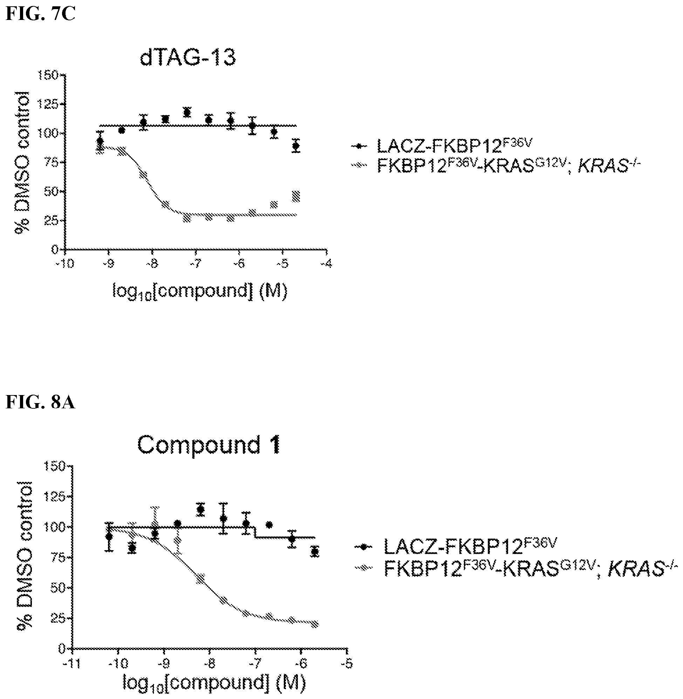

FIG. 7A-FIG. 7C are graphs that show loss of cell viability upon effective degradation of mutant KRAS in a PATU-8902 FKBP12^{F36V}-KRAS^{G12V}; *KRAS*^{*-*/*-*} clone (expressed in % DMSO control) as a function of the log of the concentration of compound **1** (FIG. 7A), dTAG-63 (FIG. 7B) and dTAG-13 (FIG. 7C) by Cell Tilter-Glo^{®} assay. PATU-8902 LACZ-FKBP12^{F36V} clone was used as a control showing that effective of degradation of LACZ did not impact cell viability. Effective loss of viability is observed with compound **1,** dTAG-63 and dTAG-13.

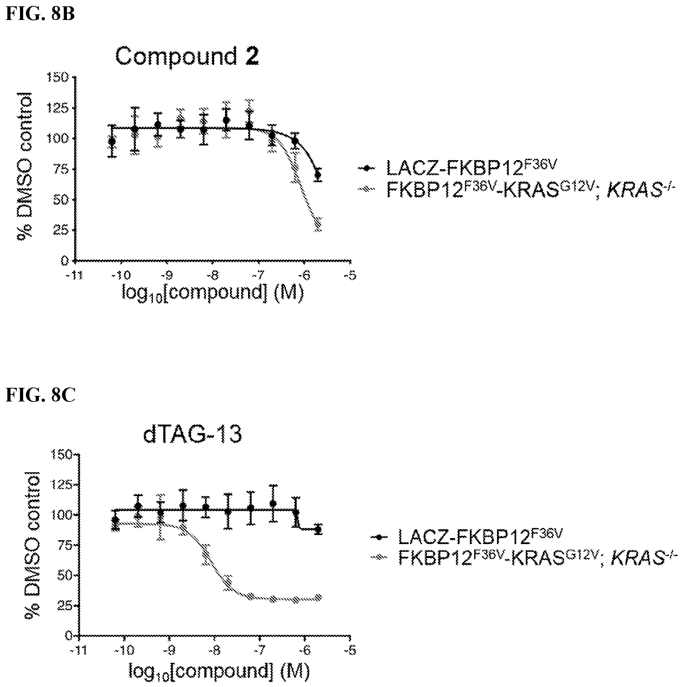

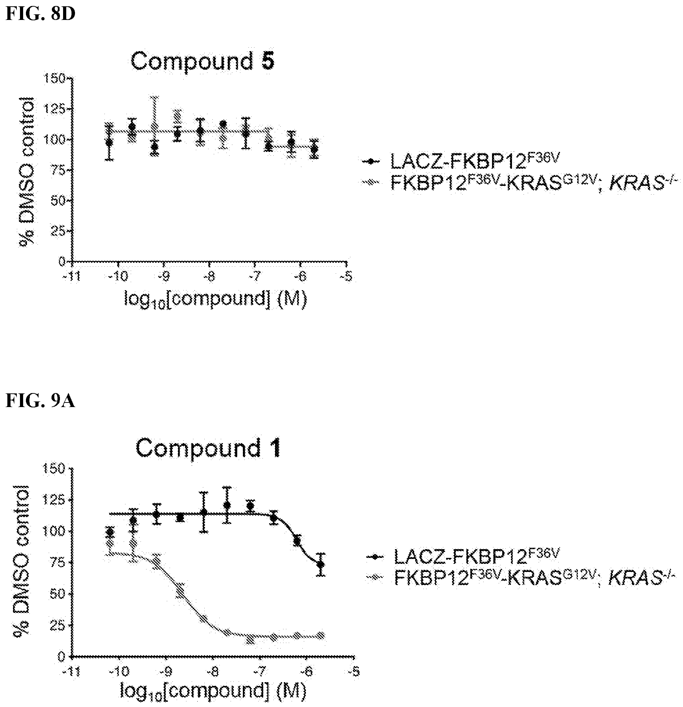

FIG. 8A-FIG. 8D are graphs that show loss of cell viability upon degradation of mutant KRAS in a PATU-8902 FKBP12^{F36V}-KRAS^{G12V}; *KRAS*^{*-*/*-*} clone (expressed in % DMSO control) as a function of the log of the concentration of inventive compounds **1** (FIG. 8A), **2** (FIG. 8B), and **5** (FIG. 8D)and dTAG-13 (FIG. 8C) by Cell Tilter-Glo^{®} assay. PATU-8902 LACZ-FKBP12^{F36V} clone was used as a control showing that effective of degradation of LACZ did not impact cell viability. Effective loss of viability is observed with compound **1** and dTAG-13, while little to no loss of viability were observed with negative control compounds **2** and **5.**

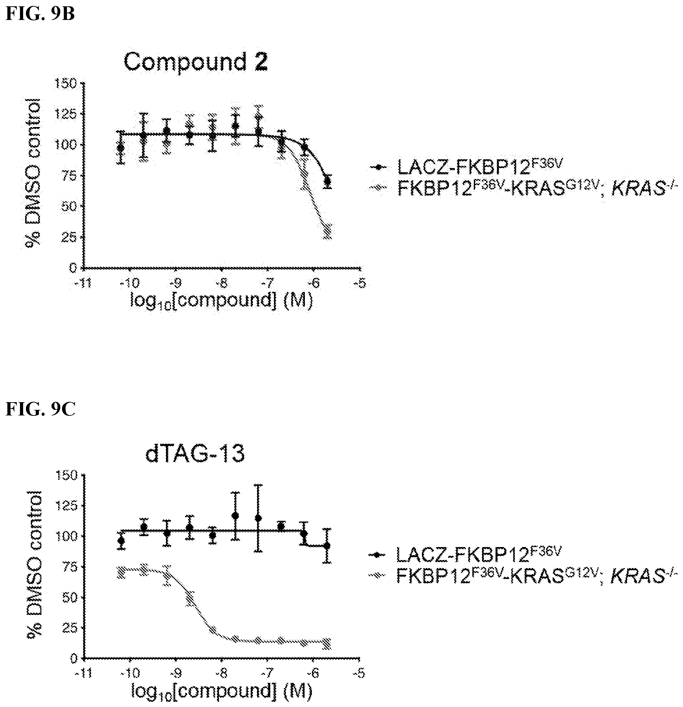

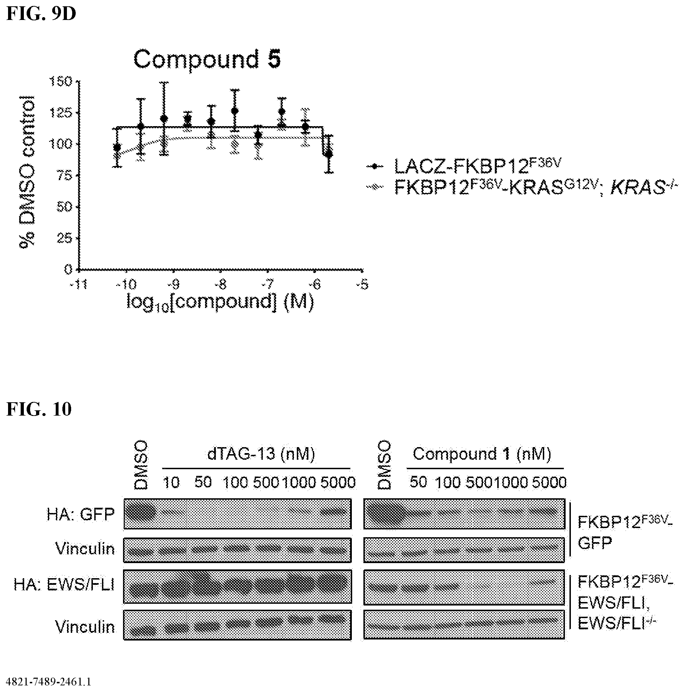

FIG. 9A-FIG. 9D are graphs that show that degradation of mutant KRAS leads to pronounced loss of cell transformation in a PATU-8902 FKBP12^{F36V}-KRAS^{G12V}; *KRAS*^{*-*/*-*} clone (expressed in % DMSO control) as a function of the log of the concentration of inventive compounds **1** (FIG. 9A), **2** (FIG. 9B), and **5** (FIG. 8D) and dTAG-13 (FIG. 9D) by Cell Tilter-Glo^{®} assay. PATU-8902 LACZ-FKBP12^{F36V} clone was used as a control showing that effective of degradation of LACZ did not impact cell transformation. Effective loss of viability is observed with compound **1** and dTAG-13, while little to no loss of viability were observed with negative control compounds **2** and **5.**
FIG. 10 is a photograph of an immunoblot that shows the degradation of EWS/FLI in EWS502 FKBP12^{F36V}-EWS/FLI; *EWS*/*FLI*^{*-*/*-*} cells by inventive compound **1** and not by dTAG-13. Degradation of GFP (control) is observed in EWS502 FKBP12^{F36V}-GFP cells inventive compound **1** and dTAG-13.

### DETAILED DESCRIPTION OF THE INVENTION

Unless defined otherwise, all technical and scientific terms used herein have the same meaning as is commonly understood by one of skill in the art to which the subject matter herein belongs. As used in the specification and the appended claims, unless specified to the contrary, the following terms have the meaning indicated in order to facilitate the understanding of the present invention.

As used in the description and the appended claims, the singular forms "a", "an", and "the" include plural referents unless the context clearly dictates otherwise. Thus, for example, reference to "a composition" includes mixtures of two or more such compositions, reference to "an inhibitor" includes mixtures of two or more such inhibitors, and the like.

Unless stated otherwise, the term "about" means within 10% (*e.g.*, within 5%, 2% or 1%) of the particular value modified by the term "about."

The transitional term "comprising," which is synonymous with "including," "containing," or "characterized by," is inclusive or open-ended and does not exclude additional, unrecited elements or method steps. By contrast, the transitional phrase "consisting of" excludes any element, step, or ingredient not specified in the claim. The transitional phrase "consisting essentially of" limits the scope of a claim to the specified materials or steps "and those that do not materially affect the basic and novel characteristic(s)" of the claimed invention.

With respect to compounds of the present invention, and to the extent the following terms are used herein to further describe them, the following definitions apply.

As used herein, the term "alkyl" refers to a saturated linear or branched-chain monovalent hydrocarbon radical. In one embodiment, the alkyl radical is a C₁-C₁₈ group. In other embodiments, the alkyl radical is a C₀ -C₆, C₀-C₅, C₀-C₃, C₁-C₁₂, C₁-C₈, C₁-C₆, C₁-C₅, C₁-C₄ or C₁-C₃ group (wherein C₀ alkyl refers to a bond). Examples of alkyl groups include methyl, ethyl, 1-propyl, 2-propyl, i-propyl, 1-butyl, 2-methyl-1-propyl, 2-butyl, 2-methyl-2-propyl, 1-pentyl, n-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, heptyl, octyl, nonyl, decyl, undecyl and dodecyl. In some embodiments, an alkyl group is a C₁-C₃ alkyl group.

As used herein, the term "alkylene" refers to a straight or branched divalent hydrocarbon chain linking the rest of the molecule to a radical group, consisting solely of carbon and hydrogen, containing no unsaturation and having from one to 12 carbon atoms, for example, methylene, ethylene, propylene, n-butylene, and the like. The alkylene chain may be attached to the rest of the molecule through a single bond and to the radical group through a single bond. In some embodiments, the alkylene group contains one to 8 carbon atoms (C₁-C₈ alkylene). In other embodiments, an alkylene group contains one to 5 carbon atoms (C₁-C₅ alkylene). In other embodiments, an alkylene group contains one to 4 carbon atoms (C₁-C₄ alkylene). In other embodiments, an alkylene contains one to three carbon atoms (C₁-C₃ alkylene). In other embodiments, an alkylene group contains one to two carbon atoms (C₁-C₂ alkylene). In other embodiments, an alkylene group contains one carbon atom (C₁ alkylene).

As used herein, the term "alkenyl" refers to a linear or branched-chain monovalent hydrocarbon radical with at least one carbon-carbon double bond. An alkenyl includes radicals having "cis" and "trans" orientations, or alternatively, "E" and "Z" orientations. In one example, the alkenyl radical is a C₂-C₁₈ group. In other embodiments, the alkenyl radical is a C₂-C₁₂, C₂-C₁₀, C₂-C₈, C₂-C₆ or C₂-C₃ group. Examples include ethenyl or vinyl, prop-1-enyl, prop-2-enyl, 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl and hexa-1,3-dienyl.

The terms "alkoxyl" or "alkoxy" as used herein refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, and -O-alkynyl.

As used herein, the term "alkoxylene" refers to a saturated monovalent aliphatic radicals of the general formula (-O-CₙH₂ₙ-) where n represents an integer *(e.g.,* 1, 2, 3, 4, 5, 6, or 7) and is inclusive of both straight-chain and branched-chain radicals. The alkoxylene chain may be attached to the rest of the molecule through a single bond and to the radical group through a single bond. In some embodiments, the alkoxylene group contains one to 3 carbon atoms (-O-C₁-C₃ alkoxylene). In other embodiments, an alkoxylene group contains one to 5 carbon atoms (-O-C₁-C₅ alkoxylene).

As used herein, the term "cyclic group" broadly refers to any group that used alone or as part of a larger moiety, contains a saturated, partially saturated or aromatic ring system *e.g.*, carbocyclic (cycloalkyl, cycloalkenyl), heterocyclic (heterocycloalkyl, heterocycloalkenyl), aryl and heteroaryl groups. Cyclic groups may have one or more (*e.g.*, fused) ring systems. Thus, for example, a cyclic group can contain one or more carbocyclic, heterocyclic, aryl or heteroaryl groups.

As used herein, the term "carbocyclic" (also "carbocyclyl") refers to a group that used alone or as part of a larger moiety, contains a saturated, partially unsaturated, or aromatic ring system having 3 to 20 carbon atoms, that is alone or part of a larger moiety (*e.g.*, an alkcarbocyclic group). The term carbocyclyl includes mono-, bi-, tri-, fused, bridged, and spiro-ring systems, and combinations thereof. In one embodiment, carbocyclyl includes 3 to 15 carbon atoms (C₃-C₁₅). In one embodiment, carbocyclyl includes 3 to 12 carbon atoms (C₃-C₁₂). In another embodiment, carbocyclyl includes C₃-C₈, C₃-C₁₀ or C₅-C₁₀. In another embodiment, carbocyclyl, as a monocycle, includes C₃-C₈, C₃-C₆ or C₅-C₆. In some embodiments, carbocyclyl, as a bicycle, includes C₇-C₁₂. In another embodiment, carbocyclyl, as a spiro system, includes C₅-C₁₂. Representative examples of monocyclic carbocyclyls include cyclopropyl, cyclobutyl, cyclopentyl, 1-cyclopent-1-enyl, 1-cyclopent-2-enyl, 1-cyclopent-3-enyl, cyclohexyl, perdeuteriocyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, phenyl, and cyclododecyl; bicyclic carbocyclyls having 7 to 12 ring atoms include [4,3], [4,4], [4,5], [5,5], [5,6] or [6,6] ring systems, such as for example bicyclo[2.2.1]heptane, bicyclo[2.2.2]octane, naphthalene, and bicyclo[3.2.2]nonane. Representative examples of spiro carbocyclyls include spiro[2.2]pentane, spiro[2.3]hexane, spiro[2.4]heptane, spiro[2.5]octane and spiro[4.5]decane. The term carbocyclyl includes aryl ring systems as defined herein. The term carbocycyl also includes cycloalkyl rings (*e.g.*, saturated or partially unsaturated mono-, bi-, or spiro-carbocycles). The term carbocyclic group also includes a carbocyclic ring fused to one or more (*e.g.,* 1, 2 or 3) different cyclic groups (*e.g.*, aryl or heterocyclic rings), where the radical or point of attachment is on the carbocyclic ring.

As used herein, the term "heterocyclyl" refers to a "carbocyclyl" that used alone or as part of a larger moiety, contains a saturated, partially unsaturated or aromatic ring system, wherein one or more (*e.g.,* 1, 2, 3, or 4) carbon atoms have been replaced with a heteroatom *(e.g.,* O, N, N(O), S, S(O), or S(O)₂). The term heterocyclyl includes mono-, bi-, tri-, fused, bridged, and spiro-ring systems, and combinations thereof. In some embodiments, a heterocyclyl refers to a 3 to 15 membered heterocyclyl ring system. In some embodiments, a heterocyclyl refers to a 3 to 12 membered heterocyclyl ring system. In some embodiments, a heterocyclyl refers to a saturated ring system, such as a 3 to 12 membered saturated heterocyclyl ring system. In some embodiments, a heterocyclyl refers to a heteroaryl ring system, such as a 5 to 14 membered heteroaryl ring system. The term heterocyclyl also includes C₃-C₈ heterocycloalkyl, which is a saturated or partially unsaturated mono-, bi-, or spiro-ring system containing 3-8 carbons and one or more (1, 2, 3 or 4) heteroatoms.

In some embodiments, a heterocyclyl group includes 3-12 ring atoms and includes monocycles, bicycles, tricycles and spiro ring systems, wherein the ring atoms are carbon, and one to 5 ring atoms is a heteroatom such as nitrogen, sulfur or oxygen. In some embodiments, heterocyclyl includes 3- to 7-membered monocycles having one or more heteroatoms selected from nitrogen, sulfur or oxygen. In some embodiments, heterocyclyl includes 4- to 6-membered monocycles having one or more heteroatoms selected from nitrogen, sulfur or oxygen. In some embodiments, heterocyclyl includes 3-membered monocycles. In some embodiments, heterocyclyl includes 4-membered monocycles. In some embodiments, heterocyclyl includes 5-6 membered monocycles. In some embodiments, the heterocyclyl group includes 0 to 3 double bonds. In any of the foregoing embodiments, heterocyclyl includes 1, 2, 3 or 4 heteroatoms. Any nitrogen or sulfur heteroatom may optionally be oxidized (*e.g.,* NO, SO, SO₂), and any nitrogen heteroatom may optionally be quaternized (*e.g.*, [NR₄]⁺Cl⁻, [NR₄]⁺OH⁻). Representative examples of heterocyclyls include oxiranyl, aziridinyl, thiiranyl, azetidinyl, oxetanyl, thietanyl, 1,2-dithietanyl, 1,3-dithietanyl, pyrrolidinyl, dihydro-1H-pyrrolyl, dihydrofuranyl, tetrahydropyranyl, dihydrothienyl, tetrahydrothienyl, imidazolidinyl, piperidinyl, piperazinyl, morpholinyl, thiomorpholinyl, 1,1-dioxo-thiomorpholinyl, dihydropyranyl, tetrahydropyranyl, hexahydrothiopyranyl, hexahydropyrimidinyl, oxazinanyl, thiazinanyl, thioxanyl, homopiperazinyl, homopiperidinyl, azepanyl, oxepanyl, thiepanyl, oxazepinyl, oxazepanyl, diazepanyl, 1,4-diazepanyl, diazepinyl, thiazepinyl, thiazepanyl, tetrahydrothiopyranyl, oxazolidinyl, thiazolidinyl, isothiazolidinyl, 1,1-dioxoisothiazolidinonyl, oxazolidinonyl, imidazolidinonyl, 4,5,6,7-tetrahydro[2H]indazolyl, tetrahydrobenzoimidazolyl, 4,5,6,7-tetrahydrobenzo[d]imidazolyl, 1,6-dihydroimidazol[4,5-d]pyrrolo[2,3-b]pyridinyl, thiazinyl, thiophenyl, oxazinyl, thiadiazinyl, oxadiazinyl, dithiazinyl, dioxazinyl, oxathiazinyl, thiatriazinyl, oxatriazinyl, dithiadiazinyl, imidazolinyl, dihydropyrimidyl, tetrahydropyrimidyl, 1-pyrrolinyl, 2-pyrrolinyl, 3-pyrrolinyl, indolinyl, thiapyranyl, 2H-pyranyl, 4H-pyranyl, dioxanyl, 1,3-dioxolanyl, pyrazolinyl, pyrazolidinyl, dithianyl, dithiolanyl, pyrimidinonyl, pyrimidindionyl, pyrimidin-2,4-dionyl, piperazinonyl, piperazindionyl, pyrazolidinylimidazolinyl, 3-azabicyclo[3.1.0]hexanyl, 3,6-diazabicyclo[3.1.1]heptanyl, 6-azabicyclo[3.1.1]heptanyl, 3-azabicyclo[3.1.1]heptanyl, 3-azabicyclo[4.1.0]heptanyl, azabicyclo[2.2.2]hexanyl, 2-azabicyclo[3.2.1]octanyl, 8-azabicyclo[3.2.1]octanyl, 2-azabicyclo[2.2.2]octanyl, 8-azabicyclo[2.2.2]octanyl, 7-oxabicyclo[2.2.1]heptane, azaspiro[3.5]nonanyl, azaspiro[2.5]octanyl, azaspiro[4.5]decanyl, 1-azaspiro[4.5]decan-2-only, azaspiro[5.5]undecanyl, tetrahydroindolyl, octahydroindolyl, tetrahydroisoindolyl, tetrahydroindazolyl, 1,1-dioxohexahydrothiopyranyl. Examples of 5-membered heterocyclyls containing a sulfur or oxygen atom and one to three nitrogen atoms are thiazolyl, including thiazol-2-yl and thiazol-2-yl N-oxide, thiadiazolyl, including 1,3,4-thiadiazol-5-yl and 1,2,4-thiadiazol-5-yl, oxazolyl, for example oxazol-2-yl, and oxadiazolyl, such as 1,3,4-oxadiazol-5-yl, and 1,2,4-oxadiazol-5-yl. Example 5-membered ring heterocyclyls containing 2 to 4 nitrogen atoms include imidazolyl, such as imidazol-2-yl; triazolyl, such as 1,3,4-triazol-5-yl; 1,2,3-triazol-5-yl, 1,2,4-triazol-5-yl, and tetrazolyl, such as 1H-tetrazol-5-yl. Representative examples of benzo-fused 5-membered heterocyclyls are benzoxazol-2-yl, benzthiazol-2-yl and benzimidazol-2-yl. Example 6-membered heterocyclyls contain one to three nitrogen atoms and optionally a sulfur or oxygen atom, for example pyridyl, such as pyrid-2-yl, pyrid-3-yl, and pyrid-4-yl; pyrimidyl, such as pyrimid-2-yl and pyrimid-4-yl; triazinyl, such as 1,3,4-triazin-2-yl and 1,3,5-triazin-4-yl; pyridazinyl, in particular pyridazin-3-yl, and pyrazinyl.

Thus, the term heterocyclic embraces N-heterocyclyl groups which as used herein refer to a heterocyclyl group containing at least one nitrogen and where the point of attachment of the heterocyclyl group to the rest of the molecule is through a nitrogen atom in the heterocyclyl group. Representative examples of N-heterocyclyl groups include 1-morpholinyl, 1-piperidinyl, 1-piperazinyl, 1-pyrrolidinyl, pyrazolidinyl, imidazolinyl and imidazolidinyl. The term heterocyclic also embraces C-heterocyclyl groups which as used herein refer to a heterocyclyl group containing at least one heteroatom and where the point of attachment of the heterocyclyl group to the rest of the molecule is through a carbon atom in the heterocyclyl group. Representative examples of C-heterocyclyl radicals include 2-morpholinyl, 2- or 3- or 4-piperidinyl, 2-piperazinyl, and 2- or 3-pyrrolidinyl. The term heterocyclic also embraces heterocyclylalkyl groups which as disclosed above refer to a group of the formula -R^{c}-heterocyclyl where R^{c} is an alkylene chain. The term heterocyclic also embraces heterocyclylalkoxy groups which as used herein refer to a radical bonded through an oxygen atom of the formula -O-R^{c}-heterocyclyl where R^{c} is an alkylene chain.

As used herein, the term "aryl" used alone or as part of a larger moiety (*e.g.*, "aralkyl", wherein the terminal carbon atom on the alkyl group is the point of attachment, *e.g.*, a benzyl group),"aralkoxy" wherein the oxygen atom is the point of attachment, or "aroxyalkyl" wherein the point of attachment is on the aryl group) refers to a group that includes monocyclic, bicyclic or tricyclic, carbon ring system, that includes fused rings, wherein at least one ring in the system is aromatic. In some embodiments, the aralkoxy group is a benzoxy group. The term "aryl" may be used interchangeably with the term "aryl ring". In one embodiment, aryl includes groups having 6-18 carbon atoms. In another embodiment, aryl includes groups having 6-10 carbon atoms. Examples of aryl groups include phenyl, naphthyl, anthracyl, biphenyl, phenanthrenyl, naphthacenyl, 1,2,3,4-tetrahydronaphthalenyl, 1H-indenyl, 2,3-dihydro-1H-indenyl, naphthyridinyl, and the like, which may be substituted or independently substituted by one or more substituents described herein. A particular aryl is phenyl. In some embodiments, an aryl group includes an aryl ring fused to one or more *(e.g.,* 1, 2 or 3) different cyclic groups *(e.g.,* carbocyclic rings or heterocyclic rings), where the radical or point of attachment is on the aryl ring.

Thus, the term aryl embraces aralkyl groups (*e.g.*, benzyl) which as disclosed above refer to a group of the formula -R^{c}-aryl where R^{c} is an alkylene chain such as methylene or ethylene. In some embodiments, the aralkyl group is an optionally substituted benzyl group. The term aryl also embraces aralkoxy groups which as used herein refer to a group bonded through an oxygen atom of the formula -O-R^{c}-aryl where R^{c} is an alkylene chain such as methylene or ethylene.

As used herein, the term "heteroaryl" used alone or as part of a larger moiety (*e.g.*, "heteroarylalkyl" (also "heteroaralkyl"), or "heteroarylalkoxy" (also "heteroaralkoxy"), refers to a monocyclic, bicyclic or tricyclic ring system having 5 to 14 ring atoms, wherein at least one ring is aromatic and contains at least one heteroatom. In one embodiment, heteroaryl includes 5-6 membered monocyclic aromatic groups where one or more ring atoms is nitrogen, sulfur or oxygen that is independently optionally substituted. In another embodiment, heteroaryl includes 5-6 membered monocyclic aromatic groups where one or more ring atoms is nitrogen, sulfur or oxygen. Representative examples of heteroaryl groups include thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, imidazopyridyl, pyrazinyl, pyridazinyl, triazinyl, tetrazinyl, tetrazolo[1,5-b]pyridazinyl, purinyl, deazapurinyl, benzoxazolyl, benzofuryl, benzothiazolyl, benzothiadiazolyl, benzotriazolyl, benzoimidazolyl, indolyl, 1,3-thiazol-2-yl, 1,3,4-triazol-5-yl, 1,3-oxazol-2-yl, 1,3,4-oxadiazol-5-yl, 1,2,4-oxadiazol-5-yl, 1,3,4-thiadiazol-5-yl, 1H-tetrazol-5-yl, 1,2,3-triazol-5-yl, and pyrid-2-yl N-oxide. The term "heteroaryl" also includes groups in which a heteroaryl is fused to one or more cyclic (*e.g.*, carbocyclyl, or heterocyclyl) rings, where the radical or point of attachment is on the heteroaryl ring. Nonlimiting examples include indolyl, indolizinyl, isoindolyl, benzothienyl, benzothiophenyl, methylenedioxyphenyl, benzofuranyl, dibenzofuranyl, indazolyl, benzimidazolyl, benzodioxazolyl, benzthiazolyl, quinolyl, isoquinolyl, cinnolinyl, phthalazinyl, quinazolinyl, quinoxalinyl, 4H-quinolizinyl, carbazolyl, acridinyl, phenazinyl, phenothiazinyl, phenoxazinyl, tetrahydroquinolinyl, tetrahydroisoquinolinyl and pyrido[2,3-b]-1,4-oxazin-3(4H)-one. A heteroaryl group may be mono-, bi- or tri-cyclic. In some embodiments, a heteroaryl group includes a heteroaryl ring fused to one or more *(e.g.,* 1, 2 or 3) different cyclic groups *(e.g.,* carbocyclic rings or heterocyclic rings), where the radical or point of attachment is on the heteroaryl ring, and in some embodiments wherein the point of attachment is a heteroatom contained in the heterocyclic ring.

The term heteroaryl also embraces N-heteroaryl groups which as used herein refers to a heteroaryl group, as defined above, and which contains at least one nitrogen atom and where the point of attachment of the N-heteroaryl group to the rest of the molecule is through a nitrogen atom in the heteroaryl group. The term heteroaryl further embraces C-heteroaryl groups which as used herein refer to a heteroaryl group as defined above and where the point of attachment of the heteroaryl group to the rest of the molecule is through a carbon atom in the heteroaryl group. The term heteroaryl further embraces heteroarylalkyl groups which as disclosed above refer to a group of the formula --R^{c}-heteroaryl, wherein R^{c} is an alkylene chain as defined above. The term heteroaryl further embraces heteroaralkoxy (or heteroarylalkoxy) groups which as used herein refer to a group bonded through an oxygen atom of the formula --O--R^{c}-heteroaryl, where R^{c} is an alkylene group as defined above.

Any of the groups described herein may be substituted or unsubstituted. As used herein, the term "substituted" broadly refers to all permissible substituents with the implicit proviso that such substitution is in accordance with permitted valence of the substituted atom and the substituent, and that the substitution results in a stable compound, i.e. a compound that does not spontaneously undergo transformation such as by rearrangement, cyclization, elimination, etc. Representative substituents include halogens, hydroxyl groups, and any other organic groupings containing any number of carbon atoms, *e.g.,* 1-14 carbon atoms, and which may include one or more *(e.g.,* 1, 2, 3, or 4) heteroatoms such as oxygen, sulfur, and nitrogen grouped in a linear, branched, or cyclic structural format.

Representative examples of substituents may thus include alkyl, substituted alkyl (*e.g.,* C1-C6, C1-5, C1-4, C1-3, C1-2, C1), alkoxy (*e.g.,* C1-C6, C1-5, C1-4, C1-3, C1-2, C1), substituted alkoxy (*e.g.,* C1-C6, C1-5, C1-4, C1-3, C1-2, C1), haloalkyl (*e.g.,* CF₃), alkenyl (*e.g.,* C2-C6, C2-5, C2-4, C2-3, C2), substituted alkenyl (*e.g.*, C2-C6, C2-5, C2-4, C2-3, C2), alkynyl (*e.g.,* C2-C6, C2-5, C2-4, C2-3, C2), substituted alkynyl (*e.g.,* C2-C6, C2-5, C2-4, C2-3, C2), cyclic (*e.g.,* C3-C12, C5-C6), substituted cyclic (*e.g.,* C3-C12, C5-C6), carbocyclic (*e.g.,* C3-C12, C5-C6), substituted carbocyclic (*e.g.,* C3-C12, C5-C6), heterocyclic (*e.g.,* C3-C12, C5-C6), substituted heterocyclic (*e.g.,* C3-C12, C5-C6), aryl (*e.g.,* benzyl and phenyl), substituted aryl (*e.g.,* substituted benzyl or phenyl), heteroaryl (*e.g.,* pyridyl or pyrimidyl), substituted heteroaryl (*e.g.,* substituted pyridyl or pyrimidyl), aralkyl (*e.g.,* benzyl), substituted aralkyl (*e.g.,* substituted benzyl), halo, hydroxyl, aryloxy (*e.g.,* C6-C12, C6), substituted aryloxy (*e.g.,* C6-C12, C6), alkylthio (*e.g.,* C1-C6), substituted alkylthio (*e.g.,* C1-C6), arylthio (*e.g.,* C6-C12, C6), substituted arylthio (e.g., C6-C12, C6), cyano, carbonyl, substituted carbonyl, carboxyl, substituted carboxyl, amino, substituted amino, amido, substituted amido, thio, substituted thio, sulfinyl, substituted sulfinyl, sulfonyl, substituted sulfonyl, sulfinamide, substituted sulfinamide, sulfonamide, substituted sulfonamide, urea, substituted urea, carbamate, substituted carbamate, amino acid, and peptide groups.

The term "binding" as it relates to interaction between the targeting ligand and the targeted protein, which in this invention is a FKBP12(F36V)-tagged protein, typically refers to an inter-molecular interaction that is substantially specific or selective in that binding of the targeting ligand with other proteinaceous entities present in the cell is functionally insignificant.

As used herein, "FKBP12(F36V)-tagged protein" refers to a target protein of interest.

The term "binding" as it relates to interaction between the degron and the E3 ubiquitin ligase, typically refers to an inter-molecular interaction that may or may not exhibit an affinity level that equals or exceeds that affinity between the targeting ligand and the target protein, but nonetheless wherein the affinity is sufficient or selective to achieve recruitment of the ligase to the targeted degradation and the selective degradation of the targeted protein.

The present invention is directed to a bifunctional compound having a structure represented by formula I: 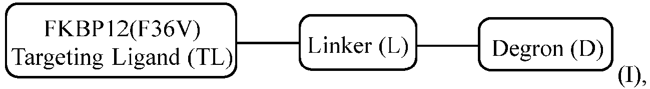
wherein the targeting ligand has a structure represented by formula TL-1: 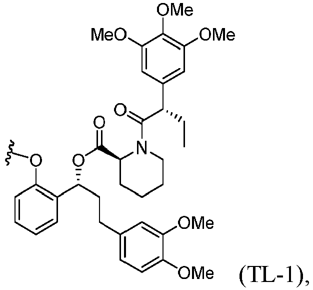
wherein the linker is an alkylene chain which may be interrupted by, and/or terminate (at either or both termini) at least one of -O-, -S-, -N(R')-, -C≡C-, -C(O)-, -C(O)O-, -OC(O)-, -OC(O)O-, -C(NOR')-, -C(O)N(R')-, -C(O)N(R')C(O)-, -C(O)N(R')C(O)N(R')-, -N(R')C(O)-,-N(R')C(O)N(R')-, -N(R')C(O)O-, -OC(O)N(R')-, -C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N(R')-,-N(R')S(O)N(R')-, C₃-C₁₂ carbocyclene, 3- to 12-membered heterocyclene, 5- to 12-membered heteroarylene or any combination thereof, wherein R' is H or C₁-C₆ alkyl, wherein the interrupting and the one or both terminating groups may be the same or different, or
a polyethylene glycol chain which may be interrupted by, and/or terminate (at either or both termini) at least one of -S-, -N(R')-, -C≡C-, -C(O)-, -C(O)O-, -OC(O)-, -OC(O)O-,-C(NOR')-, -C(O)N(R')-, -C(O)N(R')C(O)-, -C(O)N(R')C(O)N(R')-, -N(R')C(O)-,-N(R')C(O)N(R')-, -N(R')C(O)O-, -OC(O)N(R')-, -C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N(R')-,-N(R')S(O)N(R')-, C₃₋₁₂ carbocyclene, 3- to 12-membered heterocyclene, 5- to 12-membered heteroarylene or any combination thereof, wherein R' is H or C₁-C₆ alkyl, wherein the one or both terminating groups may be the same or different, or represented by structure L10: 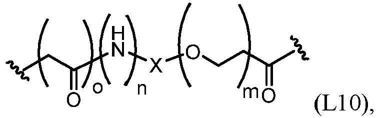
wherein m is an integer of 0-8;
X is absent or C1 to 14 alkyl;
n is 0 or 1; and
o is 0 or 1; and
wherein the degron has a structure represented by formula D1 or D2: 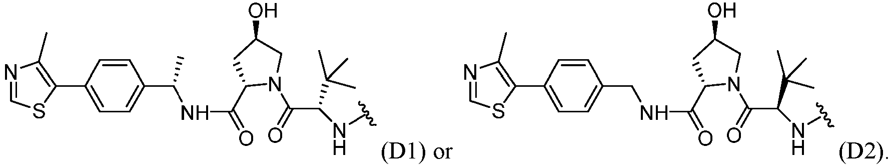
The targeting ligand represents a moiety that selectively binds a FKBP12(F36V)-tagged protein, the degron represents a ligand that selectively binds a Von Hippel-Lindau E3 ubiquitin ligase (VHL), and the linker represents a moiety that connects covalently the degron and the targeting ligand, or a pharmaceutically acceptable salt or stereoisomer thereof.

Bifunctional compounds of formula I may be in the form of a pharmaceutically acceptable salt. As used herein, the term "pharmaceutically acceptable" in the context of a salt refers to a salt of the compound that does not abrogate the biological activity or properties of the compound, and is relatively non-toxic, *i.e.,* the compound in salt form may be administered to a subject without causing undesirable biological effects (such as dizziness or gastric upset) or interacting in a deleterious manner with any of the other components of the composition in which it is contained. The term "pharmaceutically acceptable salt" refers to a product obtained by reaction of the compound of the present invention with a suitable acid or a base. Examples of pharmaceutically acceptable salts of the compounds of this invention include those derived from suitable inorganic bases such as Li, Na, K, Ca, Mg, Fe, Cu, Al, Zn and Mn salts. Examples of pharmaceutically acceptable, nontoxic acid addition salts are salts of an amino group formed with inorganic acids such as hydrochloride, hydrobromide, hydroiodide, nitrate, sulfate, bisulfate, phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, 4-methylbenzenesulfonate or p-toluenesulfonate salts and the like. Certain compounds of the invention can form pharmaceutically acceptable salts with various organic bases such as lysine, arginine, guanidine, diethanolamine or metformin.

In some embodiments, the bifunctional compounds of formula I is an isotopic derivative in that it has at least one desired isotopic substitution of an atom, at an amount above the natural abundance of the isotope, *i.e.,* enriched. In one embodiment, the compound includes deuterium or multiple deuterium atoms. Substitution with heavier isotopes such as deuterium, *i.e.* ²H, may afford certain therapeutic advantages resulting from greater metabolic stability, for example, increased *in vivo* half-life or reduced dosage requirements, and thus may be advantageous in some circumstances.

Bifunctional compounds of the present invention may have at least one chiral center and thus may be in the form of a stereoisomer, which as used herein, embraces all isomers of individual compounds that differ only in the orientation of their atoms in space. The term stereoisomer includes mirror image isomers (enantiomers which include the (R-) or (S-) configurations of the compounds), mixtures of mirror image isomers (physical mixtures of the enantiomers, and racemates or racemic mixtures) of compounds, geometric (cis/trans or E/Z, R/S) isomers of compounds and isomers of compounds with more than one chiral center that are not mirror images of one another (diastereoisomers). The chiral centers of the compounds may undergo epimerization *in vivo*; thus, for these compounds, administration of the compound in its (R-) form is considered equivalent to administration of the compound in its (S-) form. Accordingly, the compounds of the present invention may be made and used in the form of individual isomers and substantially free of other isomers, or in the form of a mixture of various isomers, *e.g.*, racemic mixtures of stereoisomers.

In addition, the bifunctional compounds of formula I embrace N-oxides, crystalline forms (also known as polymorphs), active metabolites of the compounds having the same type of activity, tautomers, and unsolvated as well as solvated forms with pharmaceutically acceptable solvents such as water, ethanol, and the like, of the compounds. The solvated forms of the conjugates presented herein are also considered to be disclosed herein.

### Targeting Ligands

The FKBP12(F36V)-tagged protein targeting ligand has a structure represented by formula TL-1:

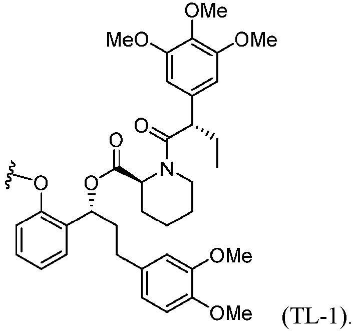

The compounds of the present invention have a structure represented by formula I-1: 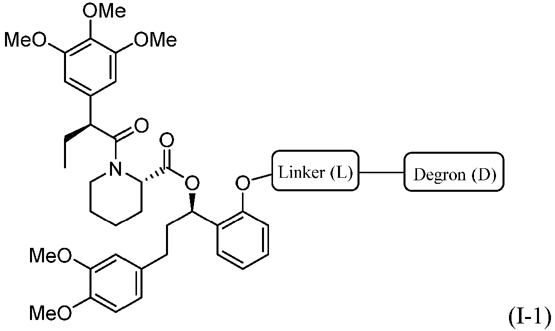 or a pharmaceutically acceptable salt or stereoisomer thereof.

### Linkers

The linker ("L") provides a covalent attachment the targeting ligand and the degron. The structure of linker may not be critical, provided it does not substantially interfere with the activity of the targeting ligand or the degron.

In some embodiments, the linker is an alkylene chain (*e.g.*, having 2-20 alkylene units), which may be interrupted by, and/or terminate at least one of -O-, -S-, -N(R')-, -C≡C-, -C(O)-, -C(O)O-, -OC(O)-, -OC(O)O-, -C(NOR')-, -C(O)N(R')-, -C(O)N(R')C(O)-,-C(O)N(R')C(O)N(R')-, -N(R')C(O)-, -N(R')C(O)N(R')-, -N(R')C(O)O-, -OC(O)N(R')-,-C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-,-S(O)N(R')-, -N(R')S(O)₂N(R')-, -N(R')S(O)N(R')-, C₃-C₁₂ carbocyclene, 3- to 12-membered heterocyclene, 5- to 12-membered heteroarylene or any combination thereof, wherein R' is H or C₁-C₆ alkyl, wherein the interrupting and the one or both terminating groups may be the same or different.

In some embodiments the linker may be C₁-C₁₀ alkylene chain terminating in NH-group wherein the nitrogen is also bound to the degron.

In certain embodiments, the linker is an alkylene chain having 1-12 alkylene units and interrupted by or terminating in

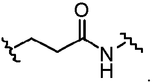

"Carbocyclene" refers to a bivalent carbocycle radical, which is optionally substituted.

"Heterocyclene" refers to a bivalent heterocyclyl radical which may be optionally substituted.

"Heteroarylene" refers to a bivalent heteroaryl radical which may be optionally substituted.

In other embodiments, the linker is a polyethylene glycol chain having 1-8 PEG units and terminating in

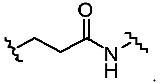

Representative examples of alkylene-based linkers that may be suitable for use in the present invention include straight alkylene chains, *e.g.*: 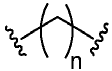 (L1), wherein n is an integer of 1-12 ("of" meaning inclusive), *e.g.,* 1-12, 1-11, 1-10, 1-9, 1-8, 1-7, 1-6, 1-5, 1-4, 1-3, 1-2, 2-10, 2-9, 2-8, 2-7, 2-6, 2-5, 2-4, 2-3, 3-10, 3-9, 3-8, 3-7, 3-6, 3-5, 3-4, 4-10, 4-9, 4-8, 4-7, 4-6, 4-5, 5-10, 5-9, 5-8, 5-7, 5-6, 6-10, 6-9, 6-8, 6-7, 7-10, 7-9, 7-8, 8-10, 8-9, 9-10 and 1, 2, 3, 4, 5, 6, 7, 8, 9 and 10, examples of which include: 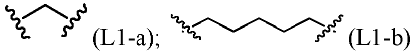 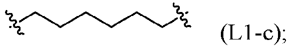 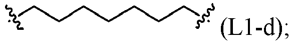 and 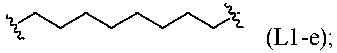 alkylene chains terminating in various functional groups (as described above), examples of which are as follows: 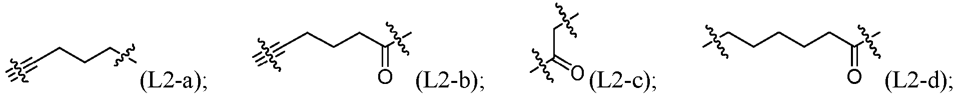 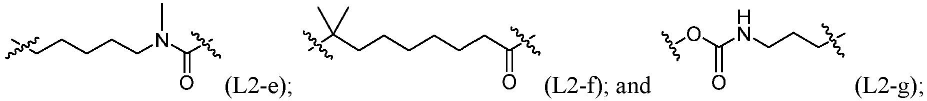 alkylene chains interrupted with various functional groups (as described above), examples of which are as follows: 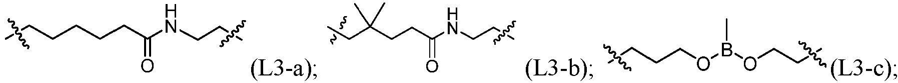 and 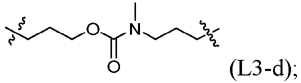 alkylene chains interrupted or terminating with heterocyclene groups, *e.g.*, 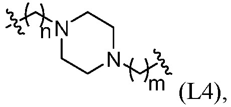 wherein m and n are independently integers from 0-10, examples of which include: 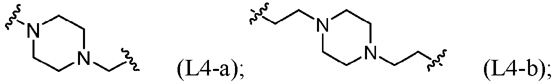 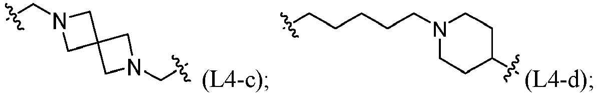 and 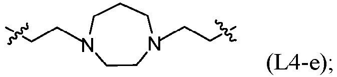 alkylene chains interrupted by amide, heterocyclene and/or aryl groups, examples of which include: 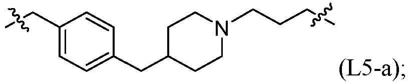 and 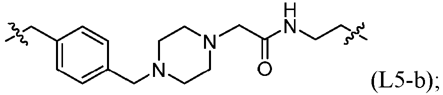 alkylene chains interrupted by heterocyclene and aryl groups, and a heteroatom, examples of which include: 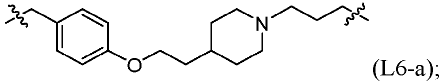 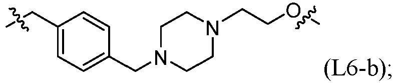 and 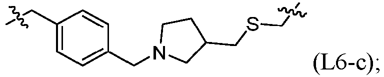 and
alkylene chains interrupted by or terminating in a heteroatom such as N, O or B, *e.g.*, 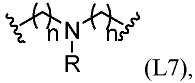 wherein each n independently is an integer of 1-10, *e.g.,* 1-9, 1-8, 1-7, 1-6, 1-5, 1-4, 1-3, 1-2, 2-10, 2-9, 2-8, 2-7, 2-6, 2-5, 2-4, 2-3, 3-10, 3-9, 3-8, 3-7, 3-6, 3-5, 3-4, 4-10, 4-9, 4-8, 4-7, 4-6, 4-5, 5-10, 5-9, 5-8, 5-7, 5-6, 6-10, 6-9, 6-8, 6-7, 7-10, 7-9, 7-8, 8-10, 8-9, 9-10, and 1, 2, 3, 4, 5, 6, 7, 8, 9 and 10, and R is H, or C₁ to C₄ alkyl, an example of which is 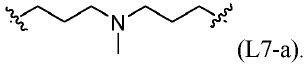 In other embodiments, the linker may be a polyethylene glycol chain, which may be interrupted by, and/or terminate (at either or both termini) at least one of -S-, -N(R')-, -C≡C-, -C(O)-,-C(O)O-, -OC(O)-, -OC(O)O-, -C(NOR')-, -C(O)N(R')-, -C(O)N(R')C(O)-,-C(O)N(R')C(O)N(R')-, -N(R')C(O)-, -N(R')C(O)N(R')-, -N(R')C(O)O-, -OC(O)N(R')-,-C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-,-S(O)N(R')-, -N(R')S(O)₂N(R')-, -N(R')S(O)N(R')-, C₃₋₁₂ carbocyclene, 3- to 12-membered heterocyclene, 5- to 12-membered heteroarylene or any combination thereof, wherein R' is H or C₁-C₆ alkyl, wherein the one or both terminating groups may be the same or different.

Representative examples of polyethylene glycol chains that may be suitable for use as linkers include: 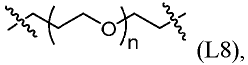 wherein n is an integer of 1-10, examples of which include: 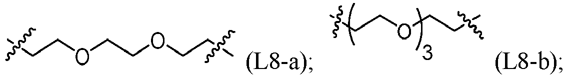 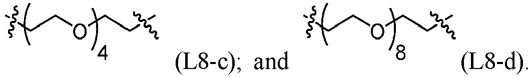 In some embodiments, the polyethylene glycol chain may terminate in a functional group, examples of which are as follows: 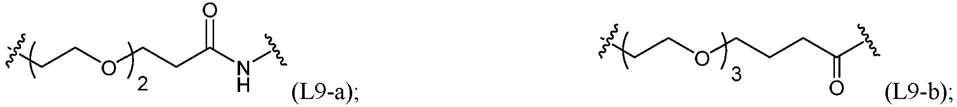 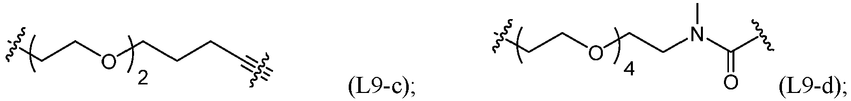 and

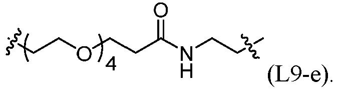

In some embodiments, the compound of formula (I) includes a linker that is represented by structure L7: 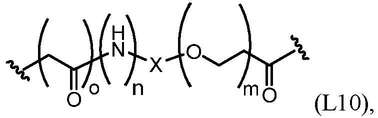
wherein m is an integer of 0-8;
X is absent or C1 to 14 alkyl;
n is 0 or 1; and
o is 0 or 1.

In some embodiments, the linker is represented by any one of the following structures:

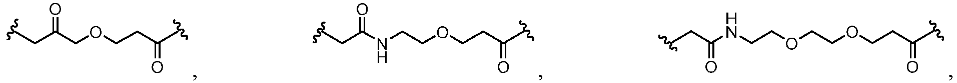

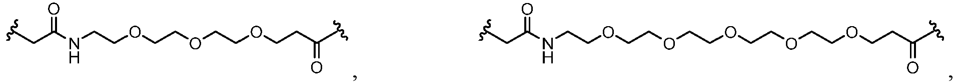

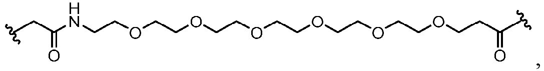

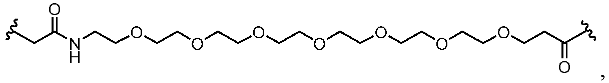

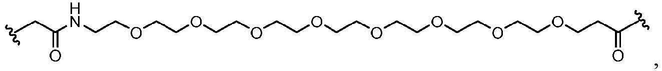

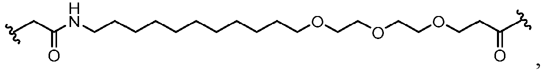

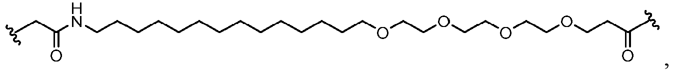

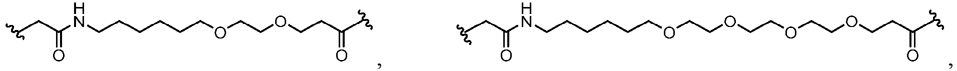

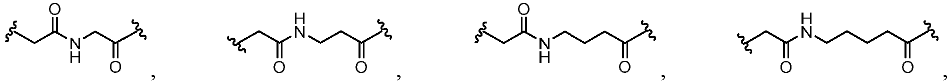

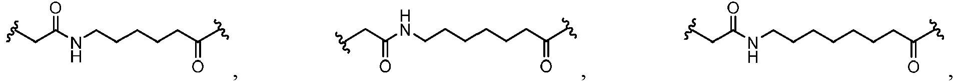

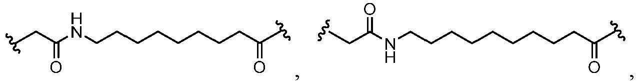

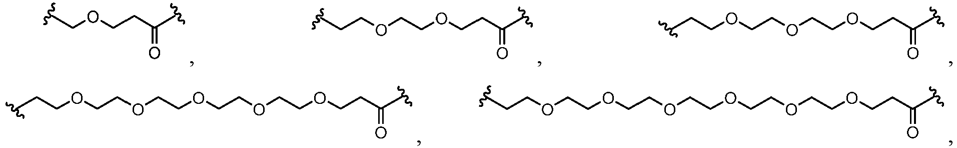

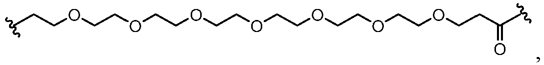

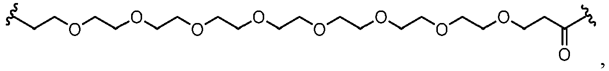

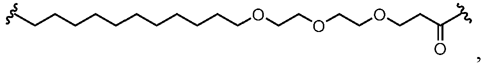

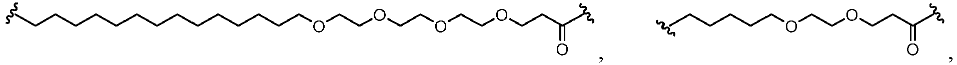

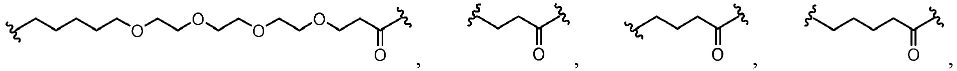

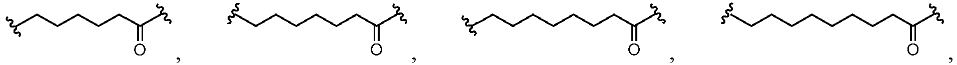

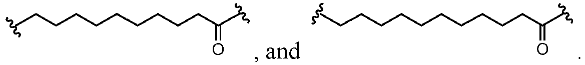

Thus, in some embodiments, the bifunctional compounds of the invention may be represented by formula I-2: 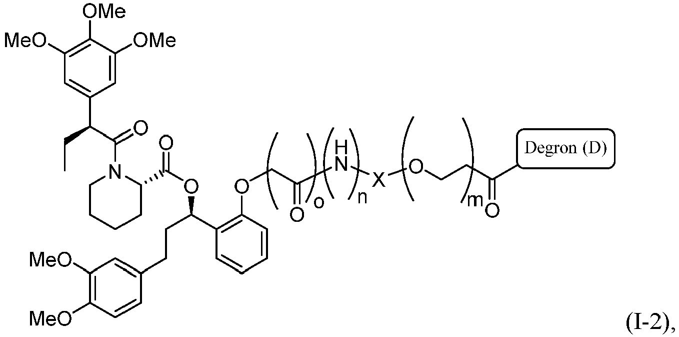
wherein m is an integer of 0-8;
X is absent or C1 to C14 alkyl;
n is 0 or 1; and
o is 0 or 1, or a pharmaceutically acceptable salt or stereoisomer thereof.

### Degrons

The Degron ("D") is a functional moiety that binds a Von Hippel-Lindau (VHL) tumor suppressor.

In the present invention, the bifunctional compound of formula I includes a degron that binds VHL that has a structure represented by formula D1 or D2:

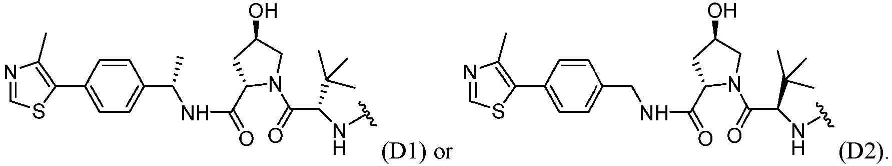

Thus, in some embodiments, the compound of the present invention is represented by any structures generated by the combination of structures TL-1, L1-L10 and the structures of the degrons described herein, which are D1 and D2, or a pharmaceutically acceptable salt or stereoisomer thereof.

In some embodiments, the compound of the present invention is represented by any one of the following structures: 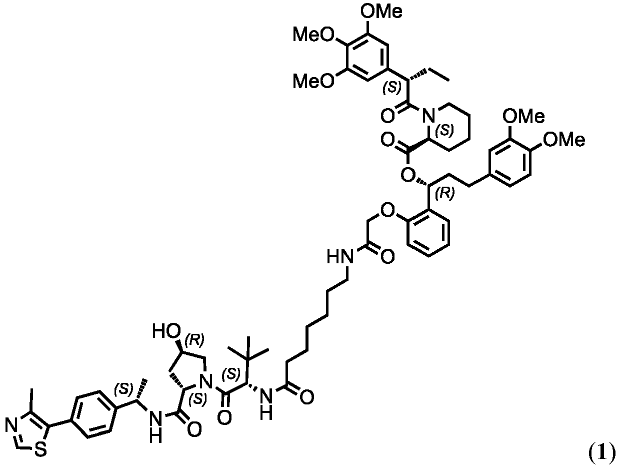 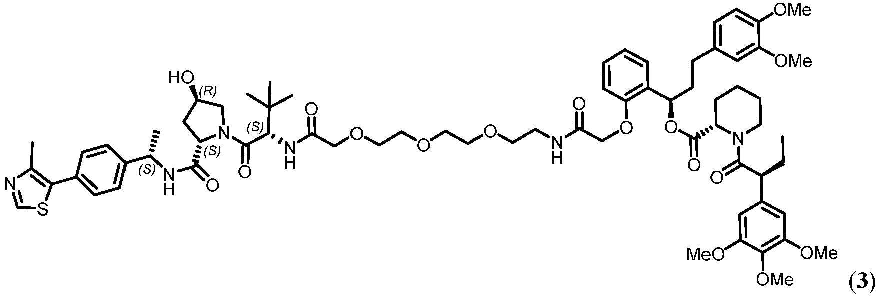 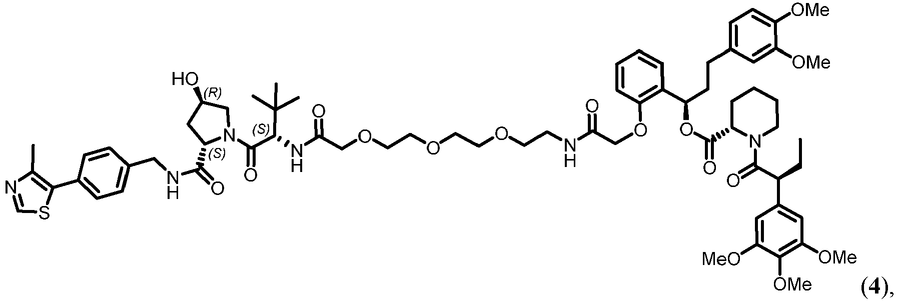 and pharmaceutically acceptable salts and stereoisomers thereof.

### Methods of Synthesis

Also disclosed herein is a method for making a bifunctional compound of formula I or a pharmaceutically acceptable salt or stereoisomer thereof. Broadly, the inventive bifunctional compounds and their pharmaceutically-acceptable salts and stereoisomers thereof may be prepared by any process known to be applicable to the preparation of chemically related compounds. The methods of making the bifunctional compounds of the present invention will be better understood in connection with the synthetic schemes that are described in various working examples.

Also disclosed herein is a degradation tag (dTAG) system comprising the protein FKBP12 (F36V) that binds the targeting ligand or a nucleic acid encoding the dTAG. The dTAG is utilized as an expression product formed *in vivo* or *in vitro* that is in the form of a fusion protein with a protein of interest (that is intended to be degraded). The other component of the system is the heterobifunctional compound (degrader) described herein. When the dTAG and the degrader are brought into contact, the dTAG is bound by the TL, and is brought into close proximity with the degron. The fusion protein, which is the dTAG-target protein, is ubiquitinated and then degraded by cellular proteasomes.

### The heterobifunctional compound targeting protein (dTAG)

The dTAG disclosed herein is a modified or mutant FKBP12 which contains one or more amino acid substitutions wherein the modified FKBP12 has an enlarged binding pocket for FKBP12 ligands. The one or more mutations include a mutation of the phenylalanine (F) at amino acid position 36 to valine (V) (F36V) (as counted without the methionine start codon) (referred to herein as FKBP12* or FKBP*, used interchangeably herein) (*see,* Clackson et al., PNAS 95:10437-42 (1998)).

In some embodiments, the dTAG disclosed herein has an amino acid sequence designated SEQ ID NO: 1:

MGVQVETISPGDGRTFPKRGQTCVVHYTGMLEDGKKVDSSRDRNKPFKFMLGKQEVIR
GWEEGVAQMSVGQRAKLTISPDYAYGATGHPGIIPPHATLVFDVELLKLE.

In some embodiments, the nucleic acid molecule that encodes the dTAG of the present invention has a sequence designated as SEQ ID NO: 2:

ATGGGAGTGCAGGTGGAAACCATCTCCCCAGGAGACGGGCGCACCTTCCCCAAGCG
CGGCCAGACCTGCGTGGTGCACTACACCGGGATGCTTGAAGATGGAAAGAAAGTTG

ATTCCTCCCGGGACAGAAACAAGCCCTTTAAGTTTATGCTAGGCAAGCAGGAGGTG
ATCCGAGGCTGGGAAGAAGGGGTTGCCCAGATGAGTGTGGGTCAGAGAGCCAAACT
GACTATATCTCCAGATTATGCCTATGGTGCCACTGGGCACCCAGGCATCATCCCACC
ACATGCCACTCTCGTCTTCGATGTGGAGCTTCTAAAACTGGAA.

In some embodiments, the dTAG disclosed herein has an amino acid sequence designated SEQ ID NO: 3:

GVQVETISPGDGRTFPKRGQTCVVHYTGMLEDGKKVDSSRDRNKPFKFMLGKQEVIRG
WEEGVAQMSVGQRAKLTISPDYAYGATGHPGIIPPHATLVFDVELLKLE.

In some embodiments, the nucleic acid molecule that encodes the dTAG disclosed herein has a sequence designated as SEQ ID NO: 4:

GGAGTGCAGGTGGAAACCATCTCCCCAGGAGACGGGCGCACCTTCCCCAAGCGCGG
CCAGACCTGCGTGGTGCACTACACCGGGATGCTTGAAGATGGAAAGAAAGTTGATT
CCTCCCGGGACAGAAACAAGCCCTTTAAGTTTATGCTAGGCAAGCAGGAGGTGATC
CGAGGCTGGGAAGAAGGGGTTGCCCAGATGAGTGTGGGTCAGAGAGCCAAACTGAC
TATATCTCCAGATTATGCCTATGGTGCCACTGGGCACCCAGGCATCATCCCACCACA
TGCCACTCTCGTCTTCGATGTGGAGCTTCTAAAACTGGAA.

**In** some embodiments, the dTAG strategy can be utilized to produce a stably expressed, endogenous protein-dTAG hybrid in vivo, or as the case may be *ex vivo* or *in vitro,* by genomic insertion of the dTAG nucleic acid sequence either 5'- or 3' in-frame with the nucleic acid sequence encoding the protein of interest. Also disclosed herein is the heterobifunctional compound for use in a method of binding the dTAG and degrading the endogenous protein-dTAG hybrid. Following the insertion of the in-frame dTAG nucleic acid sequence, the cell expresses the endogenous protein-dTAG hybrid, the modulation of the activity of the endogenous protein-dTAG hybrid is allowed through the administration of a heterobifunctional compound that is capable of binding the dTAG and thus degrading the endogenous protein-dTAG hybrid. In one embodiment, the activity of the endogenous protein-dTAG hybrid is reduced.

### Proteins of Interest

As described herein, the dTAG system can be utilized to produce a stably expressed, endogenous protein-dTAG hybrid *in vivo,* or as the case may be *ex vivo* or *in vitro,* by genomic insertion of the dTAG nucleic acid sequence either 5'- or 3' in-frame with the nucleic acid sequence encoding the protein of interest. Also disclosed herein is the heterobifunctional compound for use in a method of binding the dTAG and degrading the endogenous protein-dTAG hybrid. Following the insertion of the in-frame dTAG nucleic acid sequence, the cell expresses the endogenous protein-dTAG hybrid, and modulation of the activity of the endogenous protein-dTAG hybrid is allowed through the administration of a heterobifunctional compound that is capable of binding the dTAG and thus degrading the endogenous protein-dTAG hybrid. In one embodiment, the activity of the endogenous protein-dTAG hybrid is reduced.

In some embodiments, a nucleic acid encoding a dTAG can be genomically inserted in-frame with a gene encoding a protein that is involved in a disorder. Non-limiting examples of particular genes involved in disorders that may be targeted for dTAG insertion include by way of non-limiting example, alpha-1 antitrypsin (A1AT), apolipoprotein B (APOB), angiopoietin-like protein 3 (ANGPTL3), proprotein convertase subtilisin/kexin type 9 (PCSK9), apolipoprotein C3 (APOC3), catenin (CTNNB1), low density lipoprotein receptor (LDLR), C-reactive protein (CRP), apolipoprotein a (Apo(a)), Factor VII, Factor XI, antithrombin III (SERPINC1), phosphatidylinositol glycan class A (PIG-A), C5, alpha-1 antitrypsin (SERPINA1), hepcidin regulation (TMPRSS6), (delta-aminolevulinate synthase 1 (ALAS-1), acylCaA:diacylglycerol acyltransferase (DGAT), miR-122, miR-21, miR-155, miR-34a, prekallikrein (KLKB1), connective tissue growth factor (CCN2), intercellular adhesion molecule 1 (ICAM-1), glucagon receptor (GCGR), glucocorticoid receptor (GCCR), protein tyrosine phosphatase (PTP-1B), c-Raf kinase (RAF1), fibroblast growth factor receptor 4 (FGFR4), vascular adhesion molecule-1 (VCAM-1), very late antigen-4 (VLA-4), transthyretin (TTR), survival motor neuron 2 (SMN2), growth hormone receptor (GHR), dystrophia myotonic protein kinase (DMPK), cellular nucleic acid-binding protein (CNBP or ZNF9), clusterin (CLU), eukaryotic translation initiation factor 4E (eIF-4e), MDM2, MDM4, heat shock protein 27 (HSP 27), signal transduction and activator of transcription 3 protein (STAT3), vascular endothelial growth factor (VEGF), kinesin spindle protein (KIF11), hepatitis B genome, the androgen receptor (AR), Atonal homolog 1 (ATOH1), vascular endothelial growth factor receptor 1 (FLT1), retinoschism 1 (RS1), retinal pigment epithelium-specific 65 kDa protein (RPE65), Rab escort protein 1 (CHM), and the sodium channel, voltage gated, type X, alpha subunit (PN3 or SCN10A). Additional proteins of interest that may be targeted by dTAG insertion include proteins associated with gain of function mutations, for example, cancer causing proteins.

### Methods of Use

**In** some aspects, the methods of the disclosed herein are conducted *in vitro.* In some embodiments, the method may include genetically modifying a cell by introducing an exogenous nucleic acid comprising a sequence that encodes FKBP12(F36V) at a genetic locus of an endogenous protein, wherein the thus modified locus expresses the protein with FKBP12(F36V) as an in-frame N-terminal or C-terminal fusion; contacting the modified cells with the bifunctional compound of formula I; and detecting a change in a property of the modified cell relative to an unmodified cell to identify protein function.

**In** some other embodiments, the method includes genetically modifying a cell by introducing an exogenous nucleic acid comprising a sequence that encodes a fusion protein comprising a mutated form of a protein that is endogenous to the cell and FKBP(F36V) fused to the N-terminus or the C-terminus of the mutated protein; contacting the modified cells with the bifunctional compound of formula I; and determining a change in a property of the modified cell before and after said contacting with the heterobifunctional compound.

Also disclosed herein is the bifunctional compound of formula I for use in *in vivo* methods which may include genetically modifying a cell by introducing an exogenous nucleic acid comprising a sequence that encodes FKBP12(F36V) at a genetic locus of an endogenous protein, wherein the thus modified locus expresses the protein with FKBP12(F36V) as an in-frame N-terminal or C-terminal fusion; introducing the thus modified cells into a rodent; and administering to the rodent the bifunctional compound of formula I; and detecting a change in a property of the rodent relative to an unmodified rodent. Thus, the bifunctional compounds for use and methods described herein may be used to validate a potential protein target associated with a disease state.

In some embodiments, the cell may be a human cell, *e.g.,* a human cancer cell line or a non-cancerous cell line (for other non-cancerous conditions).

In some embodiments, the heterobifunctional compound of formula I is for use in methods disclosed herein which may also be used to provide tunable control of constitutively expressed Clustered Regularly Interspaced Short Palindromic Repeats (CRISPR) associated protein 9 (Cas9) via the degradation of the Cas9-dTAG fused proteins with the heterobifunctional compound of formula I.

As described above, the heterobifunctional compound is for use in the methods disclosed herein which are based on the genomic insertion of a dTAG in-frame with a gene expressing an endogenous protein of interest. As contemplated herein, the 5'- or 3' in-frame insertion of a nucleic acid sequence encoding a dTAG results, upon expression of the resultant nucleic acid sequence, in an endogenous protein-dTAG hybrid protein that can be targeted for degradation by the administration of a specific heterobifunctional compound.

In-frame insertion of the nucleic acid sequence encoding the dTAG can be performed or achieved by any known and effective genomic editing processes. In one aspect, the heterobifunctional compound is for use in a method of the present disclosure that utilizes the CRISPR-Cas9 system to produce knock-in endogenous protein-dTAG fusion proteins that are produced from the endogenous locus and are readily degraded in a ligand-dependent, reversible, and dose-responsive, fashion. In certain embodiments, the CRISPR-Cas9 system is employed in order to insert an expression cassette for dTAG present in a homologous recombination (HR) "donor" sequence with the dTAG nucleic acid sequence serving as a "donor" sequence inserted into the genomic locus of a protein of interest during homologous recombination following CRISPR-Cas endonucleation. The HR targeting vector contains homology arms at the 5' and 3' end of the expression cassette homologous to the genomic DNA surrounding the targeting gene of interest locus. By fusing the nucleic acid sequence encoding the dTAG in frame with the target gene of interest, the resulting fusion protein contains a dTAG that is targeted by a heterobifunctional compound.

The present disclosure also provides a heterobifunctional compound for use in methods that provide for insertion of an exogenous dTAG sequence (also called a "donor sequence" or "donor" or "transgene") in frame with the target gene of interest, and the resulting fusion protein contains a dTAG that is targeted by a heterobifunctional compound. It will be readily apparent that the donor sequence need not be identical to the genomic sequence where it is placed. A donor sequence can contain a non-homologous sequence flanked by two regions of homology to allow for efficient HR at the location of interest. Additionally, donor sequences can comprise a vector molecule containing sequences that are not homologous to the region of interest in cellular chromatin. A donor molecule can contain several, discontinuous regions of homology to cellular chromatin. For example, for targeted insertion of sequences not normally present in a region of interest, for example, the dTAGs of the present disclosure, said sequences can be present in a donor nucleic acid molecule and flanked by regions of homology to sequence in the region of interest. Alternatively, a donor molecule may be integrated into a cleaved target locus via non-homologous end joining (NHEJ) mechanisms. *See, e.g.,* U.S. Patent Application Publications 2011/0207221 A1 and U.S. 2013/0326645 A1.

The donor dTAG encoding sequence for insertion can be DNA or RNA, single-stranded and/or double-stranded and can be introduced into a cell in linear or circular form. *See, e.g.,* U.S. Patent Application Publications 2010/0047805 A1, 2011/0281361 A1, and 2011/0207221 A1. The donor sequence may be introduced into the cell in circular or linear form. If introduced in linear form, the ends of the donor sequence can be protected (*e.g.*, from exonucleolytic degradation) by methods known to those of skill in the art.

The donor polynucleotide encoding a dTAG can be introduced into a cell as part of a vector molecule having additional sequences such as, for example, CRISPR-Cas sequences, replication origins, promoters and genes encoding antibiotic resistance. Moreover, donor polynucleotides can be introduced as naked nucleic acid, as nucleic acid complexed with an agent such as a liposome or poloxamer, or can be delivered by viruses (*e.g.*, adenovirus, AAV, herpesvirus, retrovirus, lentivirus and integrase defective lentivirus (IDLV)).

The present invention takes advantage of well-characterized insertion strategies, for example the CRISPR-Cas9 system. In general, the "CRISPR system" refers collectively to transcripts and other elements involved in the expression of or directing the activity of CRISPR-associated ("Cas") genes, including sequences encoding a Cas gene, a tracr (trans-activating CRISPR) sequence (*e.g.*, tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus. (*See, e.g.,* Ruan *et al.,* Sci. Rep. 5:14253 (2015); Park *et al.,* PLoS ONE 9(4):e95101 (20140)).

In some embodiments, the CRISPR/Cas nuclease or CRISPR/Cas nuclease system includes a non-coding RNA molecule (guide) RNA, which sequence-specifically binds to DNA, and a Cas protein (*e.g.,* Cas9), with nuclease functionality (*e.g.,* two nuclease domains). Further included is the donor nucleotide encoding a dTAG for in-frame insertion into the genomic locus of the protein of interest.

In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes.

In some embodiments, a Cas nuclease and gRNA (including a fusion of crRNA specific for the target sequence and fixed tracrRNA), and a donor sequence encoding a dTAG are introduced into the cell. In general, target sites at the 5' end of the gRNA target the Cas nuclease to the target site, *e.g.,* the gene, using complementary base pairing. In some embodiments, the target site is selected based on its location immediately 5' of a protospacer adjacent motif (PAM) sequence, such as typically NGG, or NAG. In this respect, the gRNA is targeted to the desired sequence by modifying the first 20 nucleotides of the guide RNA to correspond to the target DNA sequence.

In some embodiments, the CRISPR system induces DSBs at the target site, followed by homologous recombination of the donor sequence encoding a dTAG into the genomic locus of a protein of interest, as discussed herein. In other embodiments, Cas9 variants, deemed "nickases" are used to nick a single strand at the target site. In some aspects, paired nickases are used, *e.g.,* to improve specificity, each directed by a pair of different gRNAs targeting sequences such that upon introduction of the nicks simultaneously, a 5' overhang is introduced.

In general, a CRISPR system is characterized by elements that promote the formation of a CRISPR complex at the site of a target sequence. Typically, in the context of formation of a CRISPR complex, "target sequence" generally refers to a sequence to which a guide sequence is designed to have complementarity, where hybridization between the target sequence and a guide sequence promotes the formation of a CRISPR complex, and wherein insertion of the donor sequence encoding a dTAG is to take place. Full complementarity is not necessarily required, provided there is sufficient complementarity to cause hybridization and promote formation of a CRISPR complex.

Typically, in the context of an endogenous CRISPR system, formation of the CRISPR complex (comprising the guide sequence hybridized to the target sequence and complexed with one or more Cas proteins) results in cleavage of one or both strands in or near (*e.g.*, within 1, 2, 3, 4, 5, 6, 7, 8, 9, 10, 20, 50, or more base pairs from) the target sequence. Without wishing to be bound by theory, the tracr sequence, which may comprise or consist of all or a portion of a wildtype tracr sequence (*e.g.*, about or more than about 20, 26, 32, 45, 48, 54, 63, 67, 85, or more nucleotides of a wild-type tracr sequence), may also form part of the CRISPR complex, such as by hybridization along at least a portion of the tracr sequence to all or a portion of a tracr mate sequence that is operably linked to the guide sequence. In some embodiments, the tracr sequence has sufficient complementarity to a tracr mate sequence to hybridize and participate in formation of the CRISPR complex.

As with the target sequence, in some embodiments, complete complementarity is not necessarily needed. In some embodiments, the tracr sequence has at least 50%, 60%, 70%, 80%, 90%, 95% or 99% of sequence complementarity along the length of the tracr mate sequence when optimally aligned. In some embodiments, one or more vectors driving expression of one or more elements of the CRISPR system are introduced into the cell such that expression of the elements of the CRISPR system direct formation of the CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. In some embodiments, CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (*e.g.*, each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises a regulatory element operably linked to an enzyme-coding sequence encoding a CRISPR RNA-guided endonuclease. In some embodiments, a vector comprises a regulatory element operably linked to an enzyme-coding sequence encoding the CRISPR enzyme, such as a Cas protein. Non-limiting examples of Cas proteins include Cas1, Cas IB, Cas2, Cas3, Cas4, Cas5, Cas6, Cas7, Cas8, Cas9 (also known as Csn1 and Csx12), Cas1O, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx1O, Csx16, CsaX, Csx3, Csx1, Csx15, Csf1, Csf2, Csf3, Csf4, Cpf1, homologs thereof, or modified versions thereof. (*See,* WO 2015/200334). These enzymes are known; for example, the amino acid sequence of S. pyogenes Cas9 protein may be found in the SwissProt database under accession number Q99ZW2.

Cas proteins generally comprise at least one RNA recognition or binding domain. Such domains can interact with guide RNAs (gRNAs, described in more detail below). Cas proteins can also comprise nuclease domains, for example endonuclease domains (*e.g.*, DNase or RNase domains), DNA binding domains, helicase domains, protein-protein interaction domains, dimerization domains, and other domains. A nuclease domain possesses catalytic activity for nucleic acid cleavage. Cleavage includes the breakage of the covalent bonds of a nucleic acid molecule. Cleavage can produce blunt ends or staggered ends, and it can be single-stranded or double-stranded.

Any Cas protein that induces a nick or double-strand break into a desired recognition site can be used in the methods and compositions disclosed herein.

In general, a guide sequence is any polynucleotide sequence having sufficient complementarity with a target polynucleotide sequence to hybridize with the target sequence and direct sequence-specific binding of the CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more.

Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (*e.g.*, the Burrows Wheeler Aligner), ClustalW, Clustal X, BLAT, Novoalign (Novocraft Technologies, ELAND (Illumina, San Diego, Calif.), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of the CRISPR complex to a target sequence may be assessed by any suitable assay. For example, the components of the CRISPR system sufficient to form the CRISPR complex, including the guide sequence to be tested, may be provided to the cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of the CRISPR complex, including the guide sequence to be tested and a control guide sequence different from the test guide sequence, and comparing binding or rate of cleavage at the target sequence between the test and control guide sequence reactions.

A guide sequence may be selected to target any target sequence. In some embodiments, the target sequence is a sequence within a genome of a cell, and in particular, a protein of interest targeted for controlled degradation through the engineering of an endogenous protein-dTAG hybrid. Exemplary target sequences include those that are unique in the target genome which provide for insertion of the dTAG donor nucleic acid in an in-frame orientation. In some embodiments, a guide sequence is selected to reduce the degree of secondary structure within the guide sequence. Secondary structure may be determined by any suitable polynucleotide folding algorithm.

As described herein, the CRISPR-Cas system is used to insert a nucleic acid sequence encoding a dTAG in-frame with the genomic sequence encoding a protein of interest in a eukaryotic, for example, human cell. In some embodiments, the method comprises allowing the CRISPR complex to bind to the genomic sequence of the targeted protein of interest to effect cleavage of the genomic sequence, wherein the CRISPR complex comprises the CRISPR enzyme complexed with a guide sequence hybridized to a target sequence within said target polynucleotide, wherein said guide sequence is linked to a tracr mate sequence which in turn hybridizes to a tracr sequence.

In some aspects, the methods include modifying expression of a polynucleotide in a eukaryotic cell by introducing a nucleic acid encoding a dTAG.

In some aspects, the polypeptides of the CRISPR-Cas system and donor sequence are administered or introduced to the cell. The nucleic acids typically are administered in the form of an expression vector, such as a viral expression vector. In some aspects, the expression vector is a retroviral expression vector, an adenoviral expression vector, a DNA plasmid expression vector, or an AAV expression vector. In some aspects, one or more polynucleotides encoding CRISPR-Cas system and donor sequence delivered to the cell. In some aspects, the delivery is by delivery of more than one vectors.

Methods of delivering nucleic acid sequences to cells as described herein are described, for example, in U.S. Patent Nos. 8,586,526; 6,453,242; 6,503,717; 6,534,261; 6,599,692; 6,607,882; 6,689,558; 6,824,978; 6,933,113; 6,979,539; 7,013,219; and 7,163,824.

The various polynucleotides as described herein may also be delivered using vectors containing sequences encoding one or more of compositions described herein. Any vector systems may be used including, but not limited to, plasmid vectors, retroviral vectors, lentiviral vectors, adenovirus vectors, poxvirus vectors; herpesvirus vectors and adeno-associated virus vectors, etc. *See,* also, U.S. Patent Nos. 6,534,261; 6,607,882; 6,824,978; 6,933,113; 6,979,539; 7,013,219; and 7,163,824.

Methods of non-viral delivery of nucleic acids include lipofection, nucleofection, microinjection, biolistics, virosomes, liposomes, immunoliposomes, polycation or lipid:nucleic acid conjugates, naked DNA, artificial virions, and agent-enhanced uptake of DNA. Lipofection is described in *e.g.,* U.S. Patent Nos. 5,049,386, 4,946,787; and 4,897,355) and lipofection reagents are sold commercially (*e.g.*, Transfectam^{™} and Lipofectin^{™}). Cationic and neutral lipids that are suitable for efficient receptor-recognition lipofection of polynucleotides include those of Feigner, WO 1991/17424 and WO 1991/16024. Delivery can be to cells (*e.g., in vitro* or *ex vivo* administration) or target tissues (*e.g., in vivo* administration).

In some embodiments, RNA or DNA viral based systems for the delivery of nucleic acids are for use in delivery. Viral vectors in some aspects may be administered directly to patients (*in vivo*) or they can be used to treat cells *in vitro* or *ex vivo,* and then administered to patients. Viral-based systems in some embodiments include retroviral, lentivirus, adenoviral, adeno-associated and herpes simplex virus vectors for gene transfer. The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTR5 are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (*see, e.g.,* Buchscher et al., J. Virol. 66:2731-2739 (1992); Johann et al., J. Virol. 66:1635-1640 (1992); Sommerfelt et al., J. Virol. 176:58-69 (1992); Wilson et al., J. Virol. 63:2374-2378 (1989); Miller et al., J. Virol. 65:2220-2224 (1991); and PCT/US94/05700).

In applications in which transient expression is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, *e.g.,* in the in vitro production of nucleic acids and peptides, and for *in vivo* and *ex vivo* gene therapy procedures (*see, e.g.,* West et al., Virology 160:38-47 (1987); U.S. Patent No. 4,797,368; WO 1993/24641; Kotin, Human Gene Therapy 5:793-801 (1994); Muzyczka, J. Clin. Invest. 94:1351 (1994). Construction of recombinant AAV vectors is described in a number of publications, including U.S. Patent No. 5,173,414; Trashcan ditties et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:3822-3828 (1984).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-1023 (1995); Malech et al., Proc. Natl. Acad. Sci. USA 94(22):12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol. Immunother. 44(1):10-20 (1997); Dranoff et al., Hum. Gene Ther. 1:111-112 (1997)).

Vectors suitable for introduction of polynucleotides described herein also include non-integrating lentivirus vectors (IDLV). *See,* for example, Naldini et al., Proc. Natl. Acad. Sci. USA 93:11382-11388 (1996); Dull et al., J. Virol. 72:8463-8471 (1998); Zuffery et al., J. Virol. 72:9873-9880 (1998); Follenzi et al., Nat. Genet. 25:217-222 (2000); and U.S. Patent Application Publication 2009/0117617.

Recombinant adeno-associated virus vectors (rAAV) may also be used to deliver the compositions described herein. All vectors are derived from a plasmid that retains only the AAV inverted terminal repeats flanking the transgene expression cassette. Efficient gene transfer and stable transgene delivery are key features for this vector system. (Wagner et al., Lancet 351:9117 1702-3 (1998) and Kearns et al., Gene Ther. 9:748-55 (1996)). Other AAV serotypes, including AAV1, AAV3, AAV4, AAV5, AAV6, AAV8, AAV9 and AAVrh10, pseudotyped AAV such as AAV2/8, AAV2/5 and AAV2/6 and all variants thereof, can also be used in accordance with the present invention.

Replication-deficient recombinant adenoviral vectors (Ad) can be produced at high titer and readily infect a number of different cell types. Most adenovirus vectors are engineered such that a transgene replaces the Ad Ela, E1b, and/or E3 genes; subsequently the replication defective vector is propagated in human 293 cells that supply deleted gene function in trans. Ad vectors can transduce multiple types of tissues *in vivo,* including non-dividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-1089 (1998)). Additional examples of the use of adenovirus vectors for gene transfer in clinical trials include Rosenecker et al., Infection 24(1):5-10 (1996); Sterman et al., Hum. Gene Ther. 9(7):1083-1089 (1998); Welsh et al., Hum. Gene Ther. 2:205-218 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and w2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in trans by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, *e.g.,* heat treatment to which adenovirus is more sensitive than AAV.

The vector can be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751(1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (*e.g.*, FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Vectors can be delivered *in vivo* by administration to an individual subject, typically by systemic administration (*e.g.*, intravenous, intraperitoneal, intramuscular, intrathecal, intratracheal, subdermal, or intracranial infusion) or topical application, as described below. Alternatively, vectors can be delivered to cells *ex vivo,* such as cells explanted from an individual patient (*e.g.*, lymphocytes, bone marrow aspirates, and tissue biopsy) or universal donor hematopoietic stem cells, followed by reimplantation of the cells into a patient, usually after selection for cells which have incorporated the vector.

Vectors (*e.g.*, retroviruses, adenoviruses, liposomes, etc.) containing nucleases and/or donor constructs can also be administered directly to an organism for transduction of cells in vivo. Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

In some embodiments, the polypeptides of the CRISPR-Cas system are synthesized in situ in the cell as a result of the introduction of polynucleotides encoding the polypeptides into the cell. In some aspects, the polypeptides of the CRISP-Cas system could be produced outside the cell and then introduced thereto. Methods for introducing a CRISPR-Cas polynucleotide construct into animal cells are known and include, as non-limiting examples stable transformation methods wherein the polynucleotide construct is integrated into the genome of the cell, transient transformation methods wherein the polynucleotide construct is not integrated into the genome of the cell, and virus mediated methods, as described herein. Preferably, the CRISPR-Cas polynucleotide is transiently expressed and not integrated into the genome of the cell. In some embodiments, the CRISPR-Cas polynucleotides may be introduced into the cell by for example, recombinant viral vectors (*e.g.*, retroviruses, adenoviruses), liposome and the like. For example, in some aspects, transient transformation methods include microinjection, electroporation, or particle bombardment. In some embodiments, the CRISPR-Cas polynucleotides may be included in vectors, more particularly plasmids or virus, in view of being expressed in the cells.

In some embodiments, non-CRISPR-CAS viral and non-viral based gene transfer methods can be used to insert nucleic acids encoding a dTAG in frame in the genomic locus of a protein of interest in mammalian cells or target tissues. Such methods can be used to administer nucleic acids encoding components of a zing finger protein a zing finger nuclease (ZFN), transcription activator-like effector protein (TALE), and/or transcription activator-like effector nuclease (TALEN) system to cells in culture, or in a host organism including a donor sequence encoding a dTAG for in-frame insertion into the genomic locus of a protein of interest.

Non-viral vector delivery systems include DNA plasmids, RNA *(e.g.,* a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, *see* Anderson, Science 256:808-813 (1992); Nabel & Feigner, TIBTECH 11:211-217 (1993); Mitani & Caskey, TIBTECH 11:162-166 (1993); Dillon, TIBTECH 11:167-173 (1993); Miller, Nature 357:455-460 (1992); Van Brunt, Biotechnology 6(10):1149-1154 (1988); Vigne, Restor. Neurol. Neurosci. 8:35-36 (1995); Kremer & Perricaudet, Br. Med. Bull. 51(1):31-44 (1995); and Yu et al., Gene Ther. 1:13-26 (1994).

The preparation of lipid:nucleic acid complexes, including targeted liposomes such as immunolipid complexes, is well known to one of skill in the art (*see, e.g.,* Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5, (1994):647-654; Gao et al., Gene Therapy 2:710-722 (1995); Ahmad et al., Cancer Res. 52:4817-4820 (1992); and U.S. Patent Nos. 4,186,183, 4,217,344, 4,235,871, 4,261,975, 4,485,054, 4,501,728, 4,774,085, 4,837,028, and 4,946,787).

Additional methods of delivery include the use of packaging the nucleic acids to be delivered into EnGeneIC delivery vehicles (EDVs). These EDVs are specifically delivered to target tissues using bispecific antibodies where one arm of the antibody has specificity for the target tissue and the other has specificity for the EDV. The antibody brings the EDVs to the target cell surface and then the EDV is brought into the cell by endocytosis. Once in the cell, the contents are released (*see,* MacDiarmid et al., Nat. Biotechnol. 27(7):643 (2009).

### Chimeric antigen receptor protein (CAR)-dTAG fusion proteins

Also disclosed herein are immune effector cells which have been genetically modified to express a CAR-dTAG fusion protein for use in methods that may be used in connection with immunotherapy (*e.g.*, clinically), and include administering a subject with immune effector cells such as autologous or allogeneic T-cells (CAR-T cells) which have been genetically modified to express a CAR-dTAG fusion protein. Also disclosed herein is the heterobifunctional compound for use in the methods. In the event the patient experiences an adverse immune response (*e.g.*, cytokine release syndrome or neurotoxicity) as a result of the therapy, the patient may then be administered the heterobifunctional compound which will result in degradation of the CAR-dTAG fusion protein.

Genetically modified T cells expressing chimeric antigen receptors (CAR-T therapy) have shown to have therapeutic efficacy in a number of cancers, including lymphoma (Till et al., Blood 119:3940-50 (2012)), chronic lymphocytic leukemia (Porter et al., N. Engl. J. Med. 365:725-33 (2011)), acute lymphoblastic leukemia (Grupp et al., N. Engl. J. Med. 368:1509-18 (2013)) and neuroblastoma (Louis et al., Blood 118:6050-56 (2011)). Two autologous CAR-T cell therapies (Kymriah^{™} and Yescarta^{™}) have been approved by the FDA. In common, both are CD19-specific CAR-T cell therapies lysing CD19-positive targets (normal and malignant B lineage cells).

CAR-T therapy is not, however, without significant side effects. Although most adverse events with CAR-T are tolerable and acceptable, the administration of CAR-T cells has, in a number of cases, resulted in severe systemic inflammatory reactions, including cytokine release syndrome and tumor lysis syndrome (Xu et al., Leukemia Lymphoma 54:255-60 (2013)).

Cytokine release syndrome (CRS) is an inflammatory response clinically manifesting with fever, nausea, headache, tachycardia, hypotension, hypoxia, as well as cardiac and/or neurologic manifestations. Severe cytokine release syndrome is described as a cytokine storm, and can be fatal. CRS is believed to be a result of the sustained activation of a variety of cell types such as monocytes and macrophages, T cells and B cells, and is generally characterized by an increase in levels of TNFα and IFNγ within 1 to 2 hours of stimulus exposure, followed by increases in interleukin (IL)-6 and IL-10 and, in some cases, IL-2 and IL-8 (Doessegger et al., Nat. Clin. Transl. Immuno. 4:e39 (2015)).

Tumor lysis syndrome (TLS) is a metabolic syndrome that is caused by the sudden killing of tumor cells with chemotherapy, and subsequent release of cellular contents with the release of large amounts of potassium, phosphate, and nucleic acids into the systemic circulation. Catabolism of the nucleic acids to uric acid lease to hyperuricemia; the marked increase in uric acid excretion can result in the precipitation of uric acid in the renal tubules and renal vasoconstriction, impaired autoregulation, decreased renal flow, oxidation, and inflammation, resulting in acute kidney injury. Hyperphosphatemia with calcium phosphate deposition in the renal tubules can also cause acute kidney injury. High concentrations of both uric acid and phosphate potentiate the risk of acute kidney injury because uric acid precipitates more readily in the presence of calcium phosphate and vice versa that results in hyperkalemia, hyperphosphatemia, hypocalcemia, uremia, and acute renal failure. It usually occurs in patients with bulky, rapidly proliferating, treatment-responsive tumors (Wintrobe et al., Complications of hematopoietic neoplasms Wintrobe's Clinical Hematology, 11th ed., Lippincott Williams & Wilkins, Vol. II, 1919-44 (2003)).

The dramatic clinical activity of CAR-T cell therapy necessitates the need to implement safety strategies to rapidly reverse or abort the T cell responses in patients undergoing CRS or associated adverse events.

Accordingly, the present disclosure includes fusion proteins that are CARs containing the dTAG. The CARs of the present disclosure are further characterized in that they include an extracellular ligand binding domain capable of binding to an antigen, a transmembrane domain, and an intracellular domain in this order from the N-terminal side, wherein the intracellular domain includes at least one signaling domain. The dTAG can be located at the N-terminus or between the extracellular binding domain and the transmembrane domain, provided that there is no disruption to antigen binding or insertion into the membrane. Similarly, the dTAG can be located at the C-terminus, between the transmembrane domain and the intracellular domain or between signaling domains when more than one is present, provided that there is no disruption to intracellular signaling or insertion into the membrane. The dTAG is preferably located at the C-terminus.

In some embodiments, the fusion protein is the CAR used in tisagenlecleucel (Kymriah^{™}) immunotherapy plus the dTAG described herein. Tisagenlecleucel is genetically modified, antigen-specific, autologous T cells that target CD19. The extracellular domain of the CAR is a murine anti-CD19 single chain antibody fragment (scFv) from murine monoclonal FMC63 hybridoma. The intracellular domain of the CAR is a T cell signaling domain derived from human CD3ζ and a co-stimulatory domain derived from human 4-1BB (CD137). The transmembrane domain and a spacer, located between the scFv domain and the transmembrane domain, are derived from human CD8α. Kymriah^{™} (tisagenlecleucel) is approved for the treatment of patients up to 25 years of age with B-cell precursor acute lymphoblastic leukemia (ALL) that is refractory or in relapse (R/R) and for the treatment of adults with R/R diffuse large B-cell lymphoma (DLBCL), the most common form of non-Hodgkin's lymphoma, as well as high grade B-cell lymphoma and DLBCL arising from follicular lymphoma.

In some embodiments, the fusion protein is the CAR used in axicabtagene ciloleucel (Yescarta^{™}) immunotherapy plus the dTAG described herein. Axicabtagene ciloleucel is genetically modified, antigen-specific, autologous T cells that target CD19. The extracellular domain of the CAR is a murine anti-CD19 single chain antibody fragment (scFv). The intracellular domain of the CAR is two signaling domains, one derived from human CD3ζ and one derived from human CD28. Yescarta^{™} (axicabtagene ciloleucel) is approved for the treatment of adults with R/R large B cell lymphoma including DLBCL not otherwise specified, primary mediastinal large B-cell lymphoma, high grade B-cell lymphoma, and DLBCL arising from follicular lymphoma.

The present disclosure provides a nucleic acid encoding a CAR as described herein. The nucleic acid encoding the CAR can be easily prepared from an amino acid sequence of the specified CAR by a conventional method. A base sequence encoding an amino acid sequence can be readily obtained from, for example, the aforementioned amino acid sequences or publicly available reference sequences, for example, NCBI RefSeq IDs or accession numbers of GenBank, for an amino acid sequence of each domain, and the nucleic acid of the present disclosure can be prepared using a standard molecular biological and/or chemical procedure. RefSeq IDs for commonly used CAR domains are known in the art, for example, U.S. Patent No. 9,175,308, discloses a number of specific amino acid sequences particularly used as CAR transmembrane and intracellular signaling domains. As one example, based on the base sequence, a nucleic acid can be synthesized, and the nucleic acid of the present disclosure can be prepared by combining DNA fragments which are obtained from a cDNA library using a polymerase chain reaction (PCR).

Immune effector cells expressing the CAR of the present disclosure can be engineered by introducing the nucleic acid encoding a CAR described above into a cell. In one embodiment, the step is carried out *ex vivo.* For example, a cell can be transformed *ex vivo* with a vector carrying the nucleic acid of the present disclosure to produce a cell expressing the CAR of the present disclosure.

Representative examples of immune effector cells as described herein include cytotoxic lymphocytes, T-cells, cytotoxic T-cells, T helper cells, Th17 T-cells, natural killer (NK) cells, natural killer T (NKT) cells, mast cells, dendritic cells, killer dendritic cells, or B cells derived from a mammal, for example, a human cell, or a cell derived from a non-human mammal such as a monkey, a mouse, a rat, a pig, a horse, or a dog. For example, a cell collected, isolated, purified or induced from a body fluid, a tissue or an organ such as blood (peripheral blood, umbilical cord blood etc.) or bone marrow can be used. A peripheral blood mononuclear cell (PBMC), an immune cell (a dendritic cell, a B cell, a hematopoietic stem cell, a macrophage, a monocyte, a NK cell or a hematopoietic cell (a neutrophil, a basophil)), an umbilical cord blood mononuclear cell, a fibroblast, a precursor adipocyte, a hepatocyte, a skin keratinocyte, a mesenchymal stem cell, an adipose stem cell, various cancer cell strains, or a neural stem cell can be used. In the present disclosure, use of a T-cell, a precursor cell of a T-cell (a hematopoietic stem cell, a lymphocyte precursor cell etc.) or a cell population containing them is preferable. Representative examples of T-cells include CD8-positive T-cells, CD4-positive T-cells, regulatory T-cells, cytotoxic T-cells, and tumor infiltrating lymphocytes. The cell population containing a T-cell and a precursor cell of a T-cell includes a PBMC. The aforementioned cells may be collected from a living body, obtained by expansion culture of a cell collected from a living body, or established as a cell strain. When transplantation of the produced CAR-expressing cell or a cell differentiated from the produced CAR-expressing cell into a living body is desired, it is preferable to introduce the nucleic acid into a cell collected from the living body itself or a conspecific living body thereof. Thus, the immune effector cells may be autologous or allogeneic.

The cell expressing the CAR can be for use as a therapeutic agent for a disease. The therapeutic agent can be the cell expressing the CAR as an active ingredient, and may further include a suitable excipient. The disease against which the cell expressing the CAR is administered is not limited as long as the disease shows sensitivity to the cell. Representative examples of diseases treatable with cells expressing CARs of the present disclosure include a cancer (blood cancer (leukemia), solid tumor, etc.), an inflammatory disease/autoimmune disease (asthma, eczema), hepatitis, and an infectious disease, the cause of which is a virus such as influenza and HIV, a bacterium, or a fungus, for example, tuberculosis, MRSA, VRE, and deep mycosis. The cell expressing the CAR of the present disclosure that binds to an antigen possessed by a cell that is desired to be decreased or eliminated for treatment of the aforementioned diseases, that is, a tumor antigen, a viral antigen, a bacterial antigen or the like is administered for treatment of these diseases. The cell of the present disclosure can also be utilized for use in the prevention of an infectious disease after bone marrow transplantation or exposure to radiation, donor lymphocyte transfusion for the purpose of remission of recurrent leukemia, and the like. The therapeutic agent including the cell expressing the CAR as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, although the administration route is not limited.

In some embodiments, the antigen binding moiety portion of the CAR of the disclosure is designed for use to treat a particular cancer (*e.g.*, hematological cancer). For example, a CAR designed to target CD19 can be used to treat cancers and disorders including pre-B ALL (pediatric indication), adult ALL, mantle cell lymphoma, diffuse large B-cell lymphoma, and salvage post allogenic bone marrow transplantation.

In some embodiments, the antigen binding moiety portion of the CAR of the disclosure may be for use in the treatment of solid tumors (*e.g.*, sarcomas, carcinomas, and lymphomas).

When "an immunologically effective amount", "an anti-tumor effective amount", "a tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). In some embodiments, the CAR expressing cells described herein may be administered at a dosage of 10⁴ to 10⁹ cells/kg body weight, preferably 10⁵ to 10⁶ cells/kg body weight, including all integer and non-integer values within those ranges. T-cell compositions may also be administered multiple times at these dosages. The cells can be administered by using infusion techniques that are commonly known in immunotherapy (*see, e.g.,* Rosenberg et al., N. Eng. J. Med. 319:1676 (1988)). The optimal dosage and treatment regime for a particular patient can readily be determined by one skilled in the art of medicine by monitoring the patient for signs of disease and adjusting the treatment accordingly.

The administration of the CAR expressing cells may be carried out in any convenient manner, including by aerosol inhalation, injection, ingestion, transfusion, implantation or transplantation. The CAR expressing cells described herein may be administered to a patient subcutaneously, intradermally, intratumorally, intranodally, intramedullary, intramuscularly, by intravenous (*i.v*.) injection, or intraperitoneally. In one embodiment, the CAR expressing cells for use of the present disclosure are administered to a patient by intradermal or subcutaneous injection. In another embodiment, the CAR expressing cells for use of the present disclosure are preferably administered by *i.v*. injection. The CAR expressing cells may be injected directly into a tumor, lymph node, or site of infection.

Further features of CAR proteins, nucleic acids encoding CAR proteins, immune effector cells expressing CARs and methods of using CAR expressing cells for the treatment of diseases are disclosed in U.S. Patent Application Publication 2018/0169109 A1.

The term "subject" (or "patient") as used herein includes all members of the animal kingdom prone to or suffering from the indicated disease or disorder. In some embodiments, the subject is a mammal, *e.g.*, a human or a non-human mammal. The methods are also applicable to companion animals such as dogs and cats as well as livestock such as cows, horses, sheep, goats, pigs, and other domesticated and wild animals. A subject "in need of" treatment according to the present invention may be "suffering from or suspected of suffering from" a specific disease or disorder may have been positively diagnosed or otherwise presents with a sufficient number of risk factors or a sufficient number or combination of signs or symptoms such that a medical professional could diagnose or suspect that the subject was suffering from the disease or disorder. Thus, subjects suffering from, and suspected of suffering from, a specific disease or disorder are not necessarily two distinct groups.

The modes of administration (*e.g.*, oral, parenteral) may also be determined in accordance with the standard medical practice.

### Dosage Amounts

As used herein, the term, "therapeutically effective amount" or "effective amount" refers to an amount of the compound of formula I or a pharmaceutically acceptable salt or a stereoisomer thereof; or a composition including the compound of formula I or a pharmaceutically acceptable salt or a stereoisomer thereof, effective in producing the desired therapeutic response. The term "therapeutically effective amount" includes the amount of the compound of the application or a pharmaceutically acceptable salt or a stereoisomer thereof, when administered, may induce cereblon-mediated degradation of a protein of interest, including CARs, or in the case of CAR-T therapy may reducing or alleviate to some extent an adverse immune response, *e.g.*, cytokine release syndrome (CRS) or a metabolic syndrome, *e.g.*, tumor lysis syndrome (TLS).

In respect of the therapeutic amount of the compound of formula I, the amount of the compound used for the treatment of a subject is low enough to avoid undue or severe side effects, within the scope of sound medical judgment can also be considered. The therapeutically effective amount of the compound or composition will be varied with the particular condition being treated, the severity of the condition being treated or prevented, the duration of the treatment, the nature of concurrent therapy, the age and physical condition of the end user, the specific compound or composition employed and the particular pharmaceutically acceptable carrier utilized.

The total daily dosage of the compounds of this invention and usage thereof may be decided in accordance with standard medical practice, *e.g.,* by the attending physician using sound medical judgment. The specific therapeutically effective dose for any particular subject will depend upon a variety of factors including the disease or disorder being treated and the severity thereof (*e.g.*, its present status); the activity of the specific compound employed; the specific composition employed; the age, body weight, general health, sex and diet of the subject; the time of administration, route of administration, and rate of excretion of the specific compound employed; the duration of the treatment; drugs used in combination or coincidental with the specific compound employed; and like factors well known in the medical arts (*see,* for example, Goodman and Gilman's, The Pharmacological Basis of Therapeutics, 10th ed., A. Gilman, J. Hardman and L. Limbird, eds., McGraw-Hill Press (2001), at pages 155-173.

The compound of formula I may be effective over a wide dosage range. In some embodiments, the total daily dosage *(e.g.,* for adult humans) may range from about 0.001 to about 1600 mg, from 0.01 to about 1600 mg, from 0.01 to about 500 mg, from about 0.01 to about 100 mg, from about 0.5 to about 100 mg, from 1 to about 100-400 mg per day, from about 1 to about 50 mg per day, and from about 5 to about 40 mg per day, and in yet other embodiments from about 10 to about 30 mg per day. Individual dosage may be formulated to contain the desired dosage amount depending upon the number of times the compound is administered per day. By way of example, capsules may be formulated with from about 1 to about 200 mg of compound (*e.g.*, 1, 2, 2.5, 3, 4, 5, 10, 15, 20, 25, 50, 100, 150, and 200 mgs.

The compounds of the present invention or pharmaceutical compositions for use in methods of the present disclosure may entail administration of the compounds of this invention or pharmaceutical compositions thereof to the patient in a single dose or in multiple doses (*e.g.,* 1, 2, 3, 4, 5, 6, 7, 8, 10, 15, 20, or more doses). For example, the frequency of administration may range from once a day up to about once every eight weeks. In some embodiments, the frequency of administration ranges from about once a day for 1, 2, 3, 4, 5, or 6 weeks, and in other embodiments entails a 28-day cycle which includes daily administration for 3 weeks (21 days).

### Pharmaceutical Kits

The present compositions and genetically modified cells may be assembled into kits or pharmaceutical systems. Kits or pharmaceutical systems according to this aspect of the disclosure include a carrier or package such as a box, carton, tube or the like, having in close confinement therein one or more containers, such as vials, tubes, ampoules, or bottles, which contain the compound of the present invention or a pharmaceutical composition. The kits may include the bifunctional compound of formula I or a pharmaceutically acceptable salt or stereoisomer thereof, and optionally a separate container having disposed therein a nucleic acid molecule comprising a sequence that encodes FKBP12(F36V). The kits or pharmaceutical systems of the disclosure may also include printed instructions for using the compounds and compositions.

These and other aspects of the present invention will be further appreciated upon consideration of the following Examples, which are intended to illustrate certain particular embodiments of the invention but are not intended to limit its scope, as defined by the claims.

### EXAMPLES

Unless otherwise noted, reagents and solvents were obtained from commercial suppliers and were used without further purification. ¹H NMR spectra were recorded on 500 MHz Bruker Avance III spectrometer, and chemical shifts are reported in parts per million (ppm, δ) downfield from tetramethylsilane (TMS). Coupling constants (J) are reported in Hz. Spin multiplicities are described as s (singlet), br (broad singlet), d (doublet), t (triplet), q (quartet), and m (multiplet). Mass spectra were obtained on a Waters Acquity UPLC. Preparative HPLC was performed on a Waters Sunfire C18 column (19 mm × 50 mm, 5 µM) using a gradient of 15-95% methanol in water containing 0.05% trifluoroacetic acid (TFA) over 22 min (28 min run time) at a flow rate of 20 mL/min. Assayed compounds were isolated and tested as TFA salts. Purities of assayed compounds were in all cases greater than 95%, as determined by reverse-phase HPLC analysis.

### Example 1: Synthesis of (R)-3-(3,4-dimethoxyphenyl)-1-(2-(2-((7-(((S)-1-((2S,4R)-4-hydroxy-2-(((S)-1-(4-(4-methylthiazol-5-yl)phenyl)ethyl)carbamovyl)pyrrolidin-1-yl)-3,3-dimethyl-1-oxobutan-2-yl)amino)-7-oxoheptyl)amino)-2-oxoethoxy)phenyl)propyl (S)-1-((S)-2-(3,4,5-trimethoxyphenyl)butanoyl)piperidine-2-carboxylate (1).

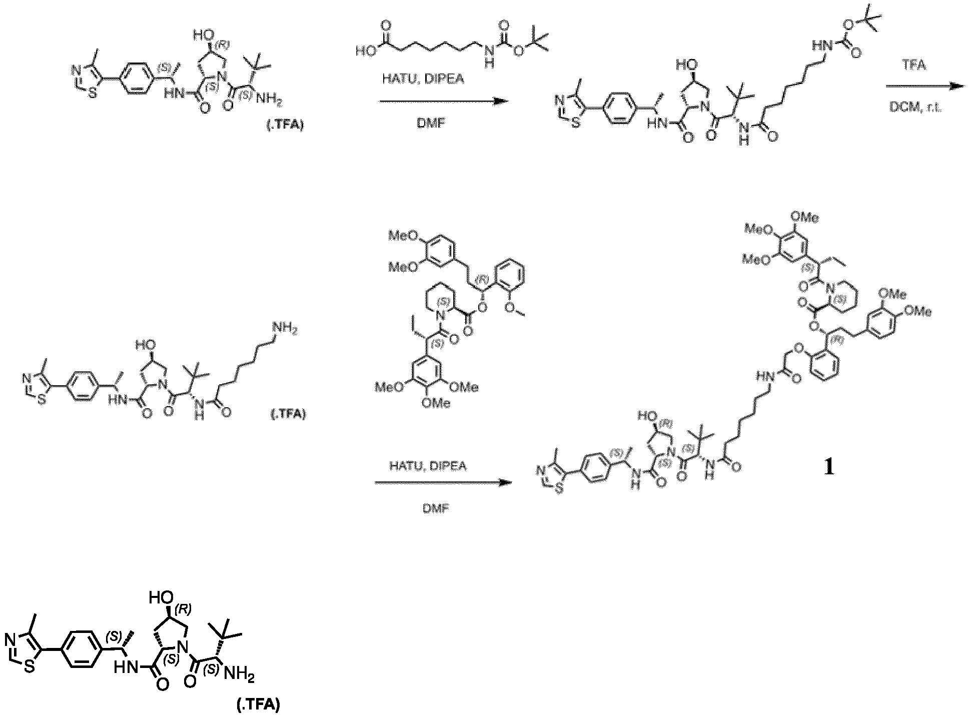

### (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide (A)

Compound A was prepared with minor modifications to the protocol described in Raina et. al., Proc. Natl. Acad. Sci. USA 113: 7124-7129 (2016). Specifically, trifluoroacetic acid (4N in DCM) was used in place of hydrochloric acid (4N in MeOH) to afford the title compound in quantitative yield.

¹H NMR (500 MHz, DMSO-*d*₆) δ 9.00 (s, 1H), 8.60 (dd, *J* = 38.0, 7.8 Hz, 1H), 8.01 (dd, *J* = 16.6, 5.4 Hz, 3H), 7.45 (dd, *J* = 8.4, 2.9 Hz, 2H), 7.39 (dd, *J* = 8.3, 4.9 Hz, 2H), 5.62 (s, 1H), 4.94 (td, *J* = 7.2, 2.6 Hz, 1H), 4.55 (td, *J = 9.5,* 7.5 Hz, 1H), 4.35 (s, 1H), 3.93 (d, *J* = 5.5 Hz, 1H), 3.68 (d, *J=* 11.1 Hz, 1H), 3.51 (dd, *J* = 11.0, 3.8 Hz, 1H), 2.46 (s, 3H), 2.17 - 2.07 (m, 1H), 1.84 - 1.75 (m, 1H), 1.42 - 1.36 (m, 3H), 1.04 (d, *J* = 2.9 Hz, 9H).

LC/MS (ESI⁺): *m*/*z* 445[M+H⁺].

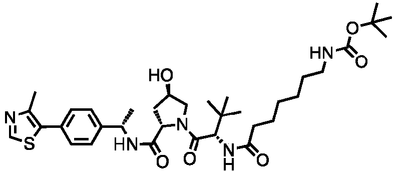

### tert-Butyl (7-(((S)-1-((2S,4R)-4-hydroxy-2-(((S)-1-(4-(4-methylthiazol-5-yl)phenyl)ethyl) carbamoyl)pyrrolidin-1-yl)-3,3-dimethyl-1-oxobutan-2-yl)amino)-7-oxoheptyl)carbamate (B)

To a stirred solution of 7-((tert-butoxycarbonyl)amino)heptanoic acid (36 mg, 0.12 mmol), 1-[bis(dimethylamino)methylene]-1H-1,2,3-triazolo[4,5-b]pyridinium 3-oxid hexafluorophosphate (HATU) (46 mg, 0.12 mmol) and N,N-diisopropylethylamine (DIPEA) (35 µL) in DMF was added compound A (50 mg, 0.1 mmol). The reaction mixture was stirred at room temperature (r.t.) for 16 hours (hrs). The reaction mixture was diluted with saturated aqueous sodium bicarbonate (20 mL) and extracted with EtOAc (3 x 50 mL). The residue was purified by flash chromatography to afford the title compound. (45 mg, 67 %).

¹H NMR (500 MHz, Methanol-*d*₄) δ 8.89 (s, 1H), 8.56 (dd, *J* = 8.0, 4.7 Hz, 1H), 8.00 (s, 1H), 7.48 - 7.42 (m, 4H), 6.56 (s, 1H), 5.51 (s, 1H), 5.07 - 4.98 (m, 1H), 4.64 (d, *J= 8.9* Hz, 1H), 4.59 (dd, *J* = 9.0, 7.7 Hz, 1H), 4.45 (dp, *J* = 4.4, 2.0 Hz, 1H), 3.90 (dt, *J=* 11.2, 1.8 Hz, 1H), 3.80 - 3.74 (m, 2H), 3.05 (d, *J =* 7.0 Hz, 2H), 2.83 (s, 3H), 2.32 - 2.25 (m, 2H), 2.25 - 2.17 (m, 2H), 1.53 (d, *J=* 7.0 Hz, 3H), 1.45 (s, 9H), 1.41 - 1.30 (m, 8H), 1.06 (s, 9H).

LC/MS (ESI⁺): *m*/*z* 672[M+H⁺].

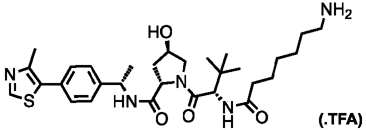

### (2S,4R)-1-((S)-2-(7-aminoheptanamido)-3,3-dimethylbutanoyl)-4-hydroxy-N-((S)-1-(4-(4-methylthiazol-5-yl)phenyl)ethyl)pyrrolidine-2-carboxamide (.TFA) (C)

A solution of compound B (45 mg, 0.067 mmol) in 4N TFA in DCM (5 mL) was stirred at r.t. for 2 hrs. The reaction mixture was concentrated *in vacuo* to afford the title compound (45 mg, quant), which was used without further purification.

LC/MS (ESI⁺): *m*/*z* 572[M+H⁺]

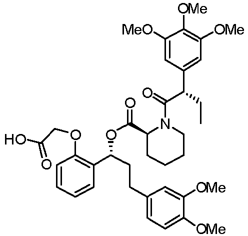

### 2-(2-((R)-3-(3,4-dimethoxyphenyl)-1-(((S)-1-((S)-2-(3,4,5-trimethoxyphenyl)butanoyl)piperidine -2-carbonyl)oxy)propyl)phenoxy)acetic acid (Ortho-AP acid) (D)

Ortho-AP acid **(D)** was prepared as described in Nabet et. al., Nat. Chem. Biol. 14:431-441 (2018).

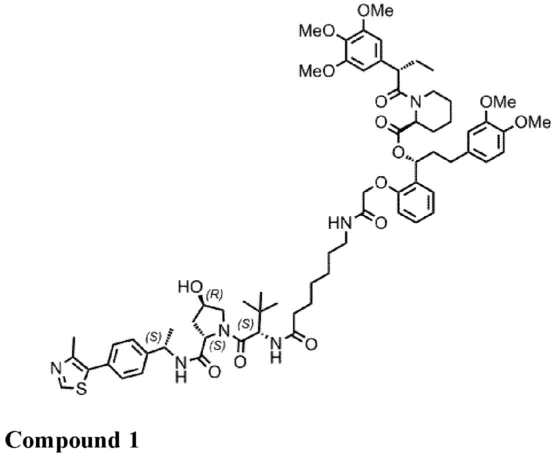

To a stirred solution of ortho-AP acid **(D)** (56 mg, 0.08 mmol), HATU (31 mg, 0.08 mmol), DIPEA (55 µL, 0.2 mmol) in DMF (2 mL) was added compound C (40 mg, 0.067 mmol). The reaction mixture was stirred for 16 hrs at r.t., filtered and purified by HPLC to afford the compound 1 as a TFA salt (34 mg, 41 %).

¹H NMR (500 MHz, DMSO-*d*₆) δ 8.99 (s, 1H), 8.37 (d, *J* = 7.8 Hz, 1H), 7.77 (dd, *J* = 9.3, 3.3 Hz, 1H), 7.70 (t, *J =* 5.8 Hz, 1H), 7.47 - 7.42 (m, 2H), 7.38 (d, *J =* 8.3 Hz, 2H), 7.21 (ddd, *J =* 8.5, 6.7, 2.4 Hz, 1H), 6.87 (d, *J =* 8.3 Hz, 1H), 6.83 - 6.79 (m, 2H), 6.75 (d, *J =* 2.0 Hz, 1H), 6.64 (dd, *J* = 8.2, 2.0 Hz, 1H), 6.62 (s, 1H), 6.56 (s, 2H), 6.03 (dd, *J* = 8.3, 4.9 Hz, 1H), 5.76 (s, 1H), 5.36 - 5.31 (m, 1H), 5.10 (d, *J* = 3.5 Hz, 1H), 4.92 (p, *J* = 7.3 Hz, 1H), 4.62 - 4.39 (m, 4H), 4.29 (d, *J =* 4.4 Hz, 1H), 4.06 (d, *J* = 13.6 Hz, 1H), 3.91 - 3.84 (m, 1H), 3.75 (s, 1H), 3.72 (s, 3H), 3.71 (s, 2H), 3.70 (s, 3H), 3.64 (d, *J =* 2.6 Hz, 1H), 3.61 (d, *J =* 5.6 Hz, 2H), 3.57 (s, 6H), 3.56 (s, 3H), 3.19 - 3.09 (m, 1H), 3.09 - 3.01 (m, 1H), 2.65 - 2.55 (m, 1H), 2.46 (s, 3H), 2.44 - 2.29 (m, 1H), 2.23 (dt, *J =* 14.5, 7.5 Hz, 1H), 2.16 (d, *J =* 13.2 Hz, 1H), 2.13 - 2.05 (m, 1H), 2.01 (dd, *J* = 12.4, 8.1 Hz, 1H), 1.97 - 1.86 (m, 1H), 1.79 (ddd, *J =* 18.5, 9.4, 5.1 Hz, 1H), 1.60 (qd, *J =* 15.0, 14.5, 9.6 Hz, 2H), 1.49 - 1.40 (m, 1H), 1.38 (d, *J =* 7.0 Hz, 3H), 1.33 (d, *J* = 6.9 Hz, 1H), 1.24 (s, 1H), 1.20 (d, *J* = 17.1 Hz, 6H), 0.94 (s, 9H), 0.81 (t, *J* = 7.3 Hz, 3H).

LC/MS (ESI⁺): *m*/*z* 1248[M+H⁺], 624[M+H⁺]/2.

### Example 2: Synthesis of (R)-3-(3,4-dimethoxyphenyl)-1-(2-(2-((7-(((S)-1-((2R,4S)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)amino)-7-oxoheptyl)amino)-2-oxoethoxy)phenyl)propyl (S)-1-((S)-2-(3,4,5-trimethoxyphenyl)butanoyl)piperidine-2-carboxylate (2).

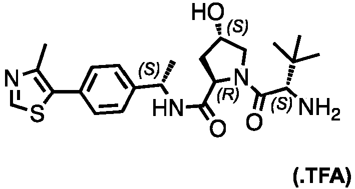

### (2R,4S)-1-((S)-2-amino-3,3-dimethylbutanoyl)-4-hydroxy-N-((S)-1-(4-(4-methylthiazol-5-yl)phenyl)ethyl)pyrrolidine-2-carboxamide (E)

Compound **E** was prepared in an analogous manor to compound A in Example 1 in quatitative yield.

¹H NMR (500 MHz, DMSO-*d*₆) δ 9.00 (s, 1H), 8.42 (d, *J* = 8.0 Hz, 1H), 8.09 (s, 2H), 7.48 - 7.44 (m, 4H), 4.95 (h, *J =* 7.1 Hz, 1H), 4.43 (ddd, *J =* 15.8, 8.5, 5.4 Hz, 2H), 3.92 (q, *J =* 5.5 Hz, 1H), 3.74 (dd, *J =* 10.9, 4.8 Hz, 1H), 3.58 (dd, *J =* 10.8, 3.3 Hz, 1H), 2.47 (s, 3H), 2.10 (ddd, *J =* 12.9, 8.3, 4.4 Hz, 1H), 2.01 - 1.94 (m, 1H), 1.38 (d, *J* = 7.0 Hz, 3H), 1.03 (s, 9H).

LC/MS (ESI⁺): *m*/*z* 445[M+H⁺].

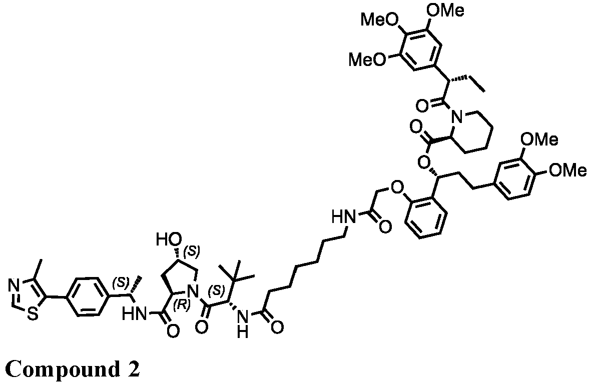

Compound **2** was prepared from intermediate **E** in an analogous manner to compound **1** in Example 1.

¹H NMR (500 MHz, DMSO-*d*₆) δ 8.98 (s, 1H), 8.03 (d, *J* = 8.0 Hz, 1H), 7.89 (d, *J* = 7.8 Hz, 1H), 7.68 (t, *J* = 5.8 Hz, 1H), 7.19 (ddd, *J* = 8.7, 6.6, 2.6 Hz, 1H), 6.86 (d, *J* = 8.3 Hz, 1H), 6.85 - 6.76 (m, 4H), 6.74 (d, *J =* 2.0 Hz, 1H), 6.65 - 6.60 (m, 2H), 6.56 (d, *J* = 2.7 Hz, 2H), 6.03 (dt, *J =* 8.5, 4.6 Hz, 1H), 5.32 (dd, *J =* 5.9, 2.5 Hz, 1H), 4.91 (h, *J =* 7.2, 6.5 Hz, 1H), 4.59 - 4.42 (m, 3H), 4.39 (dd, *J =* 8.0, 5.1 Hz, 2H), 4.31 (p, *J =* 5.2 Hz, 1H), 4.05 (d, *J =* 13.2 Hz, 1H), 3.86 (t, *J =* 7.2 Hz, 1H), 3.81 (dd, *J =* 10.5, 5.4 Hz, 1H), 3.74 (s, 2H), 3.71 (s, 3H), 3.69 (s, 3H), 3.64 (s, 1H), 3.56 (s, 6H), 3.55 (s, 3H), 3.50 (dd, *J* = 10.4, 4.2 Hz, 1H), 3.14 - 3.00 (m, 3H), 2.66 - 2.55 (m, 1H), 2.45 (*d, J =* 1.5 Hz, 3H), 2.43 - 2.30 (m, 1H), 2.24 (dt*, J =* 14.8, 7.7 Hz, 1H), 2.21 - 2.10 (m, 1H), 2.04 (ddd, *J* = 14.5, 8.1, 5.3 Hz, 1H), 2.00 - 1.85 (m, 3H), 1.67 - 1.48 (m, 3H), 1.47 - 1.32 (m, 2H), 1.31 (d, *J =* 7.0 Hz, 3H), 1.27 - 1.00 (m, 7H), 0.97 (s, 9H), 0.81 (t, *J =* 7.3 Hz, 3H).

LC/MS (ESI⁺): *m*/*z* 1248[M+H⁺], 624[M+H⁺]/2.

### Example 3: Synthesis of (R)-3-(3,4-dimethoxyphenyl)-1-(2-(((S)-16-((2S,4R)-4-hydroxy-2-(((S)-1-(4-(4-methylthiazol-5-yl)phenyl)ethyl)carbamoyl)pyrrolidine-1-carbonyl)-17,17-dimethyl-2,14-dioxo-6,9,12-trioxa-3,15-diazaoctadecyl)oxy)phenyl)propyl (S)-1-((S)-2-(3,4,5-trimethoxyphenyl)butanoyl)piperidine-2-carboxylate (3).

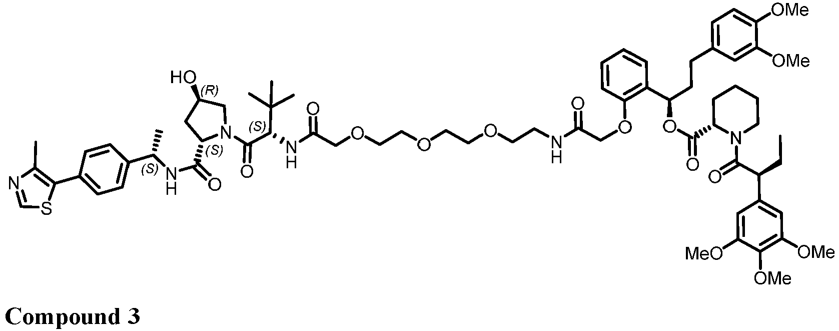

The title compound (3) was prepared from intermediate **A** in an analogous manner to compound 1 in Example 1.

LC/MS (ESI⁺): *m*/*z* 1310[M+H⁺], 655 [M+H⁺]/2.

### Example 4: Synthesis of (R)-3-(3,4-dimethoxyphenyl)-1-(2-(((S)-16-((2S,4R)-4-hydroxy-2-((4-(4-methylthiazol-5-yl)benzyl)carbamoyl)pyrrolidine-1-carbonyl)-17,17-dimethyl-2,14-dioxo-6,9,12-trioxa-3,15-diazaoctadecyl)oxy)phenyl)propyl (S)-1-((S)-2-(3,4,5-trimethoxyphenyl)butanoyl)piperidine-2-carboxylate (4).

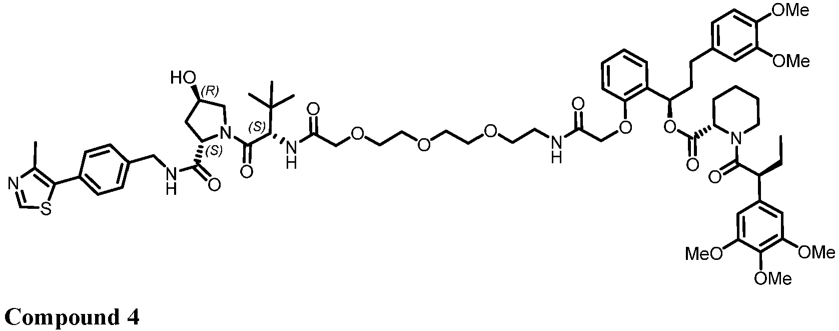

The title compound (**4**) was prepared from VHL ligand 1 (Galdeano et. al., J. Med. Chem. 57: 8657-8663 (2014)) in an analogous manner to compound 1 in Example 1.

LC/MS (ESI⁺): *m*/*z* 1296[M+H⁺], 648 [M+H⁺]/2.

### Example 5: Anti-proliferation in NIH/3T3 cells expressing FKBPI2^{F36v}-KRAS^{G12V} by ATPlite^{™} assay.

NIH/3T3 cells, that were mock transduced (control) or stably expressing with FKBP12^{F36v}-KRAS^{G12V}, were plated in 384-well format, allowed to adhere overnight, and treated with various dTAG molecules from compound stock plates using a JANUS^{®} Workstation pin tool for 72 hrs. To measure cell viability, ATPlite^{™} was added to wells for 15 minutes at room temperature and luminescence was measured on an EnVision^{™} 2104 Multilabel Plate Reader.

dTAG-12, dTAG-63 and dTAG-13 treatment (0.00063-20 µM) of NIH/3T3 cells expressing FKBP12^{F36v}-KRAS^{G12V} led to potent anti-proliferative effects (FIG. 1A-FIG. 1C). Limited anti-proliferative effects were observed on control NIH/3T3 cells at high doses with dTAG-12 and dTAG-63. These data indicate effective degradation of mutant KRAS by co-opting the CRBN or VHL E3 ligase machinery leads to potent anti-proliferative effects.

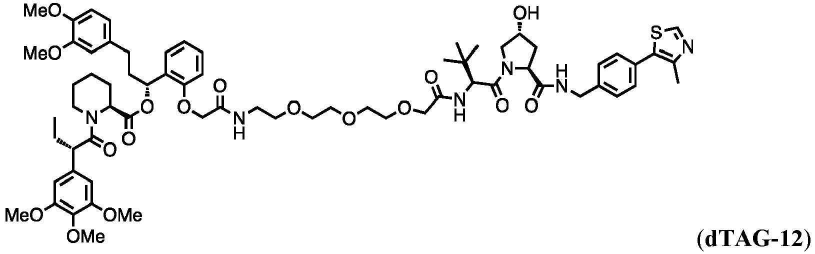

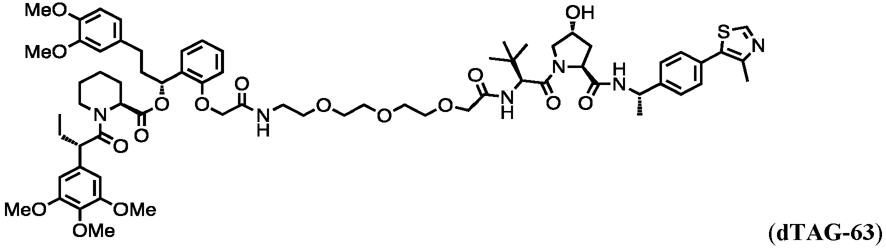

### Example 6: Immunoblotting of PATU-8902 LACZ-FKBP12^{F36v} clone.

PATU-8902 LACZ-FKBP12^{F36v} clone was plated in 6-well format, allowed to adhere overnight, and treated with 50 nM and 500 nM dTAG molecules for 4 hrs. Following treatment, cells were lysed in RIPA buffer on ice for 60 minutes. Lysates were clarified by centrifugation at 20,000 xg for 10 minutes at 4°C. Immunoblotting was performed to determine changes in protein levels with an Odyssey CLx Imager. The indicated antibodies were employed including HA (hemagglutinin) to monitor LACZ-FKBP12^{F36v} degradation and α-TUBULIN as a loading control.

Compound **1** and dTAG-13 4-hour treatment (50 or 500 nM) led to the potent degradation of LACZ-FKBP12^{F36v} (FIG. 2). Importantly, treatment with compound **2** and compound **5** (*see,* structure below), matched negative control molecules for compound 1 and dTAG-13 that cannot bind VHL or cereblon, respectively, had no effect on LACZ-FKBP12^{F36v} levels.

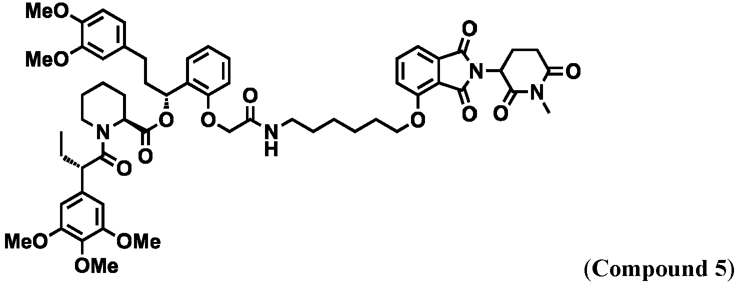

### Example 7: Immunoblotting of PATU-8902 LACZ-FKBP12^{F36v} clone.

PATU-8902 LACZ-FKBP12^{F36v} clone was plated in 6-well format, allowed to adhere overnight, and treated with DMSO and indicated doses of THAL-SNS-032 and/or 500 nM compound 1 for 24 hrs. Following treatment, cells were lysed in RIPA buffer on ice for 60 minutes. Lysates were clarified by centrifugation at 20,000 xg for 10 minutes at 4°C. Immunoblotting was performed to determine changes in protein levels with an Odyssey CLx Imager. The indicated antibodies were employed including HA to monitor LACZ-FKBP12^{F36v} degradation, CDK9 to monitor CDK9 degradation and α-TUBULIN as a loading control.

Compound **1** and THAL-SNS-032 led to the potent degradation of LACZ-FKBP12^{F36v} and CDK9, respectively (FIG. 3). Importantly, co-treatment of THAL-SNS-032 and compound **1,** effectively depleted LACZ-FKBP12^{F36v} and CDK9 equivalent to each compound alone, indicating that VHL and CRBN were effectively co-opted in concert to degrade both proteins.

### Example 8: Immunoblotting of PATU-8902 FKBP12^{F36v}-KRAS^{G12V}; KRAS^{-/-} clone.

PATU-8902 FKBP12^{F36V}-KRAS^{G12V}; *KRAS*^{*-*/*-*} clone was plated in 6-well format, allowed to adhere overnight, and treated with 50 nM and 500 nM dTAG molecules for 4 hrs. Following treatment, cells were lysed in RIPA buffer on ice for 60 minutes. Lysates were clarified by centrifugation at 20,000 xg for 10 minutes at 4°C. Immunoblotting was performed to determine changes in protein levels with an Odyssey CLx Imager. The indicated antibodies were employed including HA to monitor FKBP12^{F36V}-KRAS^{G12V} degradation, total and phosphorylated ERK to evaluate changes in ERK signaling, total and phosphorylated AKT to evaluate changes in AKT signaling and α-TUBULIN as a loading control.

Compound **1** and dTAG-13 treatment (50 or 500 nM) led to the potent degradation of FKBPI2^{F36v}-KRAS^{G12V} (FIG. 4). Degradation of FKBP12^{F36v}-KRAS^{G12V} upon compound **1** and dTAG-13 treatment led pronounced diminished levels of pERK and pAKT. Importantly, treatment with compound **2** and compound **5,** matched negative control molecules for compound **1** and dTAG-13 that cannot bind VHL or cereblon, respectively, had no effect on FKBP12^{F36V}-KRAS^{G12V}, pERK or pAKT levels. Together, these results indicate that mutant KRAS is amenable to degradation by co-opting CRBN and VHL E3 ligases, leading to rapid loss of downstream signaling pathways.

### Example 9: Immunoblotting of PATU-8902 FKBP12^{F36V}-KRAS^{G12V}; KRAS^{-/-} clone.

PATU-8902 FKBP12^{F36V}-KRAS^{G12V}; *KRAS*^{*-*/*-*} clone was plated in 6-well format, allowed to adhere overnight, and treated with DMSO, Carflizomib (CARF) or MLN4924 (MLN) prior to treatment with DMSO or compound **1** for 4 hrs. Following treatment, cells were lysed in RIPA buffer on ice for 60 minutes. Lysates were clarified by centrifugation at 20,000 xg for 10 minutes at 4°C. Immunoblotting was performed to determine changes in protein levels with an Odyssey CLx Imager. The indicated antibodies were employed including HA to monitor FKBP12^{F36V}-KRAS^{G12V} degradation and α-TUBULIN as a loading control.

500 nM Compound **1** treatment led to the potent degradation of FKBP12^{F36V}-KRAS^{G12V}, which was rescued upon pre-treatment with proteasome-inhibitor (carfilzomib, CARF) or Nedd8 activating enzyme inhibitor (MLN4924, MLN) (FIG. 5). Together, these results indicate that mutant KRAS is amenable to degradation by co-opting VHL E3 ligases, which requires the proteasome and activated E3 ligases.

### Example 10: Immunoblotting of 293T^{WT(wild-type)} or 293T^{VHL-/-} FKBP12^{F36V}-KRAS^{G12V} cells.

293T^{WT} or 293T^{VHL-/-} FKBP12^{F36V}-KRAS^{G12V} cells were plated in 6-well format, allowed to adhere overnight, and treated with 50 nM and 500 nM dTAG molecules for 24 hrs. Following treatment, cells were lysed in RIPA buffer on ice for 60 minutes. Lysates were clarified by centrifugation at 20,000 xg for 10 minutes at 4°C. Immunoblotting was performed to determine changes in protein levels with an Odyssey CLx Imager. The indicated antibodies were employed including HA to monitor FKBP12^{F36v}-KRAS^{G12V} degradation and α-TUBULIN as a loading control.

Compound 1 and dTAG-13 treatment (50 or 500 nM) led to the potent degradation of FKBPI2^{F36v}-KRAS^{G12V} in 293T^{WT} cells (FIG. 6). Importantly, dTAG-13 treatment led to the potent degradation of FKBPI2^{F36v}-KRAS^{G12V} in 293T^{VHL-/-} cells, while Compound 1 did not degrade FKBPI2^{F36v}-KRAS^{G12V} in 293T^{VHL-/-} cells. Together, these results indicate that Compound 1 requires VHL to degrade mutant KRAS.

### Example 11: Anti-proliferation measurements via Cell Tilter-Glo^{®} assay.

PATU-8902 LACZ-FKBP12^{F36v} or FKBP12^{F36V}-KRAS^{G12V}; *KRAS*^{*-*/*-*} clones were plated in 384-well format, allowed to adhere overnight, and treated with various dTAG molecules from compound stock plates using a JANUS^{®} Workstation pin tool for 72 hrs. To measure cell viability, Cell Tilter-Glo^{®} was added to wells for 15 minutes at r.t. and luminescence was measured on an EnVision^{™} 2104 Multilabel Plate Reader.

Compound 1, dTAG-63 and dTAG-13 treatment (0.00063-20 µM) led to potent anti-proliferative effects in a PATU-8902 FKBP12^{F36v}-KRAS^{G12V}; *KRAS*^{*-*/*-*} clone with limited effects on a LACZ-FKBP12^{F36v} control clone (FIG. 7A-FIG. 7C). These data indicate that effective degradation of mutant KRAS by co-opting the CRBN or VHL E3 ligase machinery leads to potent anti-proliferative effects.

### Example 12: Anti-proliferation measurements via Cell Tilter-Glo^{®} assay.

PATU-8902 LACZ-FKBP12^{F36v} or FKBP12^{F36v}-KRAS^{G12V}; *KRAS*^{*-*/*-*} clones were plated in 384-well format, allowed to adhere overnight, and treated with various dTAG molecules from compound stock plates using a JANUS^{®} Workstation pin tool for 72 hrs. To measure cell viability, Cell Tilter-Glo^{®} was added to wells for 15 minutes at r.t. and luminescence was measured on an EnVision^{™} 2104 Multilabel Plate Reader.

Compound **1** and dTAG-13 treatment (0.000063-2 µM) led to potent anti-proliferative effects in a PATU-8902 FKBP12^{F36V}-KRAS^{G12V}; *KRAS*^{*-*/*-*} clone with limited effects on a LACZ-FKBP12^{F36V} control clone (FIG. 8A-FIG. 8D). Treatment with compound **2** and compound **5,** are negative control molecules for compound **1** and dTAG-13 that cannot bind VHL or cereblon, respectively, had little to no toxicity. These data indicate that effective degradation of mutant KRAS by co-opting the CRBN or VHL E3 ligase machinery leads to potent anti-proliferative effects.

### Example 13: Cell transformation by growth in ultra-low attachment conditions by Cell Tilter-Glo^{®} assay.

PATU-8902 LACZ-FKBP12^{F36v} or FKBP12^{F36V}-KRAS^{G12V}; *KRAS*^{*-*/*-*} clones were plated in 384-well format in ultra-low attachment conditions, allowed to form 3D-spheroids overnight, and treated with various dTAG molecules from compound stock plates using a JANUS^{®} Workstation pin tool for 120 hrs. To measure cell transformation, Cell Tilter-Glo^{®} was added to wells for 15 minutes at room temperature and luminescence was measured on an EnVision^{™} 2104 Multilabel Plate Reader.

Compound **1** and dTAG-13 treatment (0.000063-2 µM) led to potent loss of cell transformation in a PATU-8902 FKBP12^{F36V}-KRAS^{G12V}; *KRAS*^{*-*/*-*} clone with limited effects on a LACZ-FKBP12^{F36v} control clone (FIG. 9A-FIG. 9D). Treatment with compound 2 and compound 5, negative control molecules for compound 1 and dTAG-13 that cannot bind VHL or cereblon, respectively, had limited toxicity at high doses. These data indicate that effective degradation of mutant KRAS by co-opting the CRBN or VHL E3 ligase machinery leads to pronounced loss of cell transformation.

### Example 14: Immunoblotting of EWS502 FKBP12^{F36V}-GFP and FKBP12^{F36V}-EWS/FLI; EWS/FLI^{-/-} cells.

EWS502 FKBP12^{F36V}-GFP and FKBP12^{F36V}-EWS/FLI; *EWS*/*FLI*^{*-*/*-*} cells were plated in 6-well format, allowed to adhere overnight, and treated with (10-5000 nM) dTAG-13 or (50-5000 nM) compound 1 for 24 hrs. Following treatment, cells were lysed in Cell Lysis Buffer (Cell Signaling Technology^{™}) on ice for 60 minutes. Lysates were clarified by centrifugation at 20,000 xg for 10 minutes at 4°C. Immunoblotting was performed to determine changes in protein levels upon incubation with the appropriate horseradish peroxidase-conjugated secondary antibodies. The indicated antibodies were employed including HA to monitor FKBP12^{F36V}-GFP or FKBP12^{F36V}-EWS/FLI degradation and Vinculin as a loading control.

dTAG-13 and compound 1 treatment led to the potent degradation of FKBP12^{F36V}-GFP (FIG. 10). However, only compound 1 treatment led to potent degradation of FKBP12^{F36V}-EWS/FLI. Together, these results indicate that EWS/FLI is amenable to degradation by co-opting VHL E3 ligases.

All publications cited in the specification, including patent publications and non-patent publications, are indicative of the level of skill of those skilled in the art to which this invention pertains.

## Claims

1. A bifunctional compound having a structure represented by formula I: 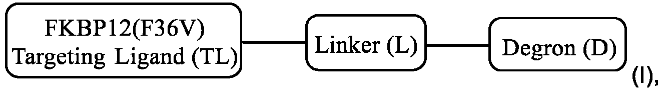
**or a pharmaceutically acceptable salt or stereoisomer thereof,**
**wherein the targeting ligand has a structure represented by formula TL-1:** 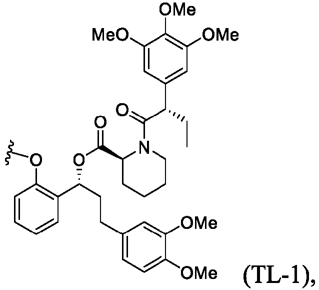
**wherein the linker is an alkylene chain which may be interrupted by, and/or terminate (at either or both termini) at least one of** -O-, -S-, -N(R')-, -C≡C-, -C(O)-, -C(O)O-, -OC(O)-, - OC(O)O-, -C(NOR')-, -C(O)N(R')-, -C(O)N(R')C(O)-, -C(O)N(R')C(O)N(R')-, -N(R')C(O)-, -N(R')C(O)N(R')-, -N(R')C(O)O-, -OC(O)N(R')-, -C(NR')-, -N(R')C(NR')-, -C(NR')N(R')-, -N(R')C(NR')N(R')-, -OB(Me)O-, -S(O)₂-, -OS(O)-, -S(O)O-, -S(O)-, -OS(O)₂-, - S(O)₂O-, -N(R')S(O)₂-, -S(O)₂N(R')-, -N(R')S(O)-, -S(O)N(R')-, -N(R')S(O)₂N(R')-, - N(R')S(O)N(R')-, C₃-C₁₂ **carbocyclene, 3- to 12-membered heterocyclene, 5- to 12-membered** heteroarylene or any combination thereof, wherein R' is H or C₁-C₆ alkyl, wherein the interrupting and the one or both terminating groups may be the same or different, or
a polyethylene glycol chain which may be interrupted by, and/or terminate (at either or both termini) at least one of -S-, -N(R')-, -C≡C-, -C(O)-, -C(O)O-, -OC(O)-, -OC(O)O-, - C(NOR')-, -C(O)N(R')-, -C(O)N(R')C(O)-, -C(O)N(R')C(O)N(R')-, -N(R')C(O)-, - N(R')C(O)N(R')-, -N(R')C(O)O-, -OC(O)N(R')-, -C(NR')-, -N(R')C(NR')-, -C(NR')N(R')-, - N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N(R')-, - N(R')S(O)N(R')-, C₃₋₁₂ carbocyclene, 3- to 12-membered heterocyclene, 5- to 12-membered heteroarylene or any combination thereof, wherein R' is H or C₁-C₆ alkyl, wherein the one or both terminating groups may be the same or different, or represented by structure L10: 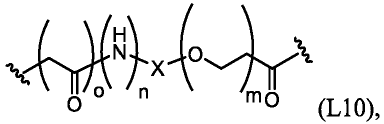
wherein m is an integer of 0-8;
X is absent or C1 to 14 alkyl;
n is 0 or 1; and
o is 0 or 1; and
wherein the degron has a structure represented by formula D1 or D2:

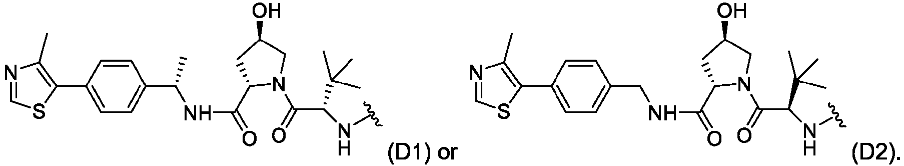

2. The bifunctional compound of claim 1, wherein the linker is represented by structure L10: 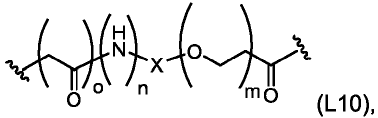
wherein m is an integer of 0-8;
X is absent or C1 to 14 alkyl;
n is 0 or 1; and
o is 0 or 1.

3. The bifunctional compound of claim 2, wherein the linker is represented by a structure selected from the group consisting of:

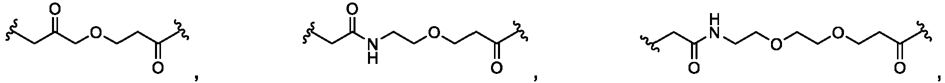

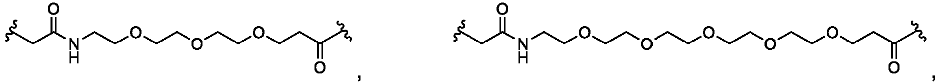

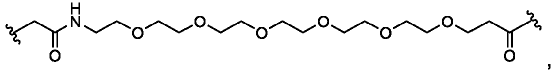

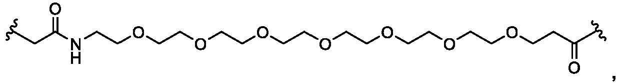

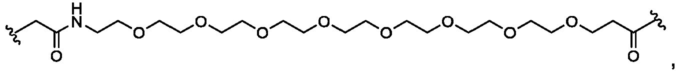

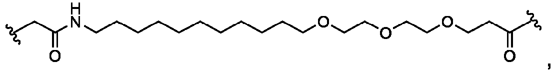

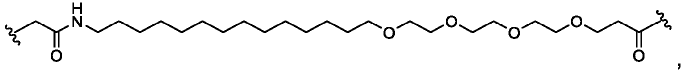

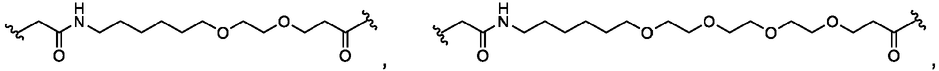

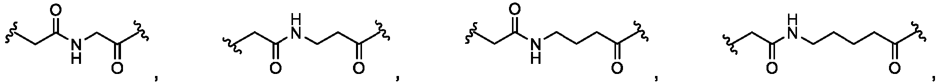

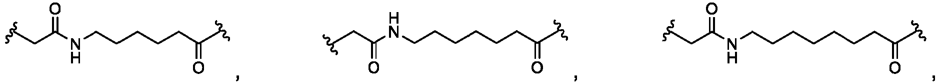

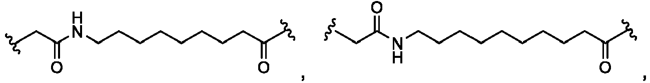

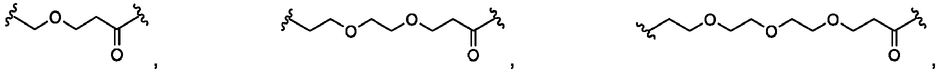

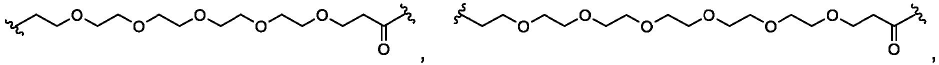

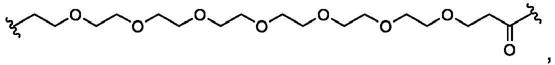

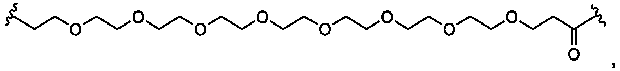

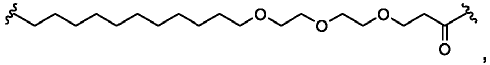

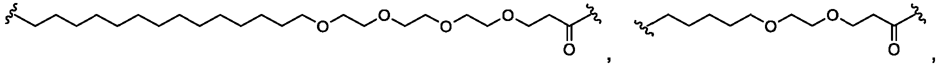

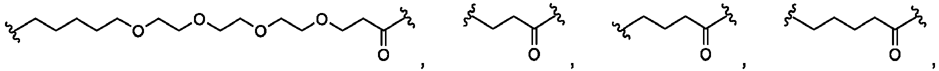

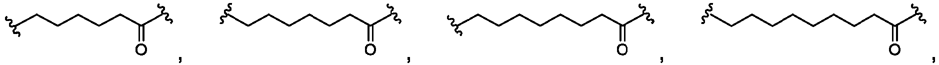

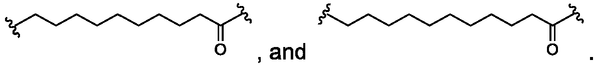

4. The bifunctional compound of any one of claims 1-3, wherein the degron has a structure represented by formula D1:

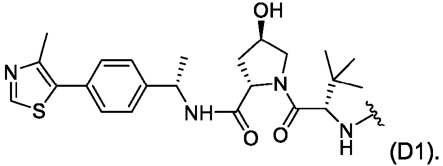

5. The bifunctional compounds of any one of claims 1-3, wherein the degron has a structure represented by formula D2:

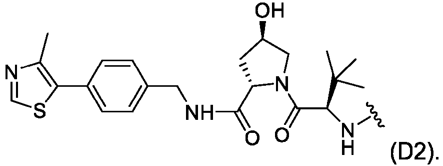

6. The bifunctional compound of claim 1, which is represented by a structure selected from the group consisting of: 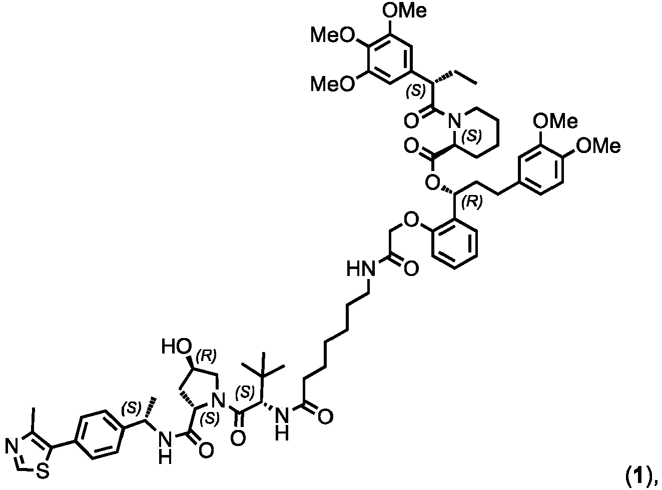 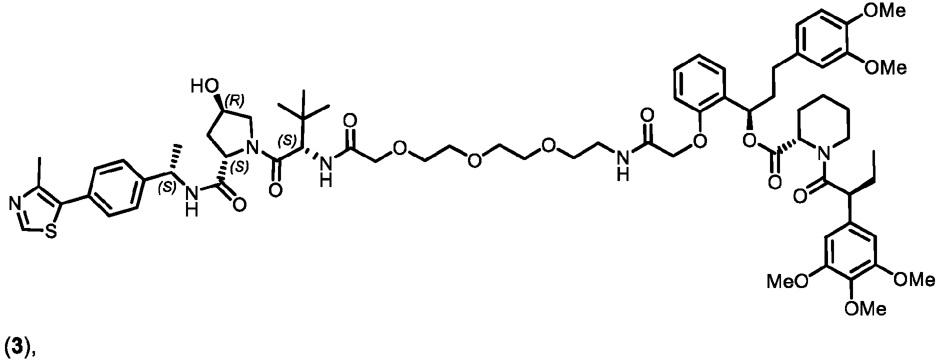 and 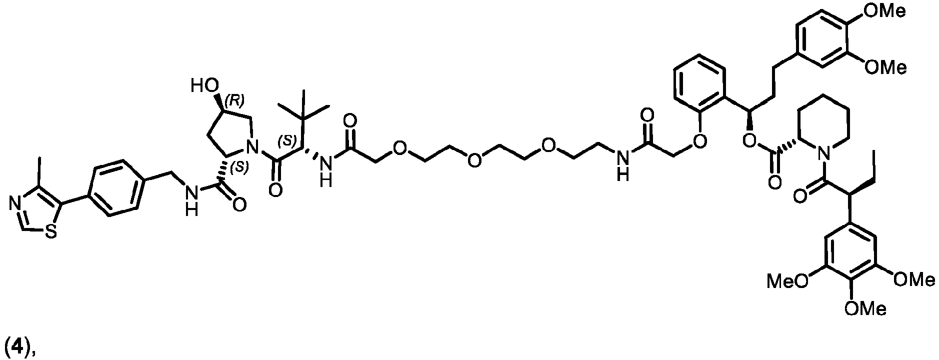 or a pharmaceutically acceptable salt and stereoisomer thereof.

7. The bifunctional compound of claim 6, which is: 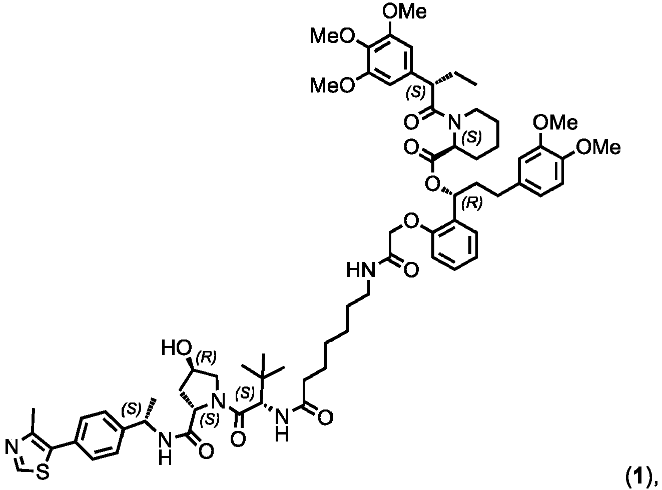 or a pharmaceutically acceptable salt and stereoisomer thereof.

8. The bifunctional compound of claim 6, which is: 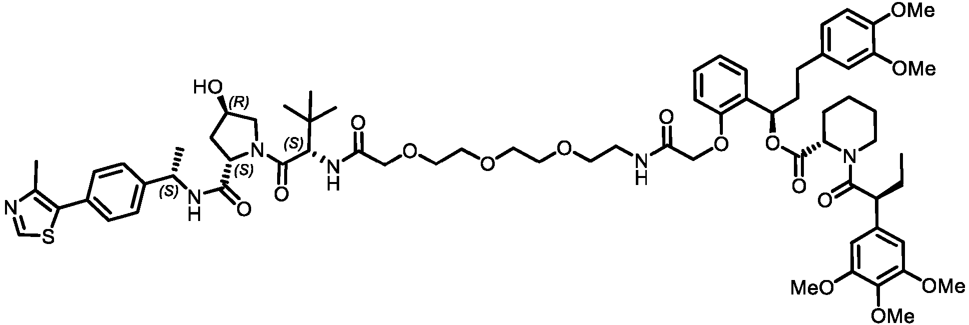 (3), or a pharmaceutically acceptable salt and stereoisomer thereof.

9. The bifunctional compound of claim 6, which is: 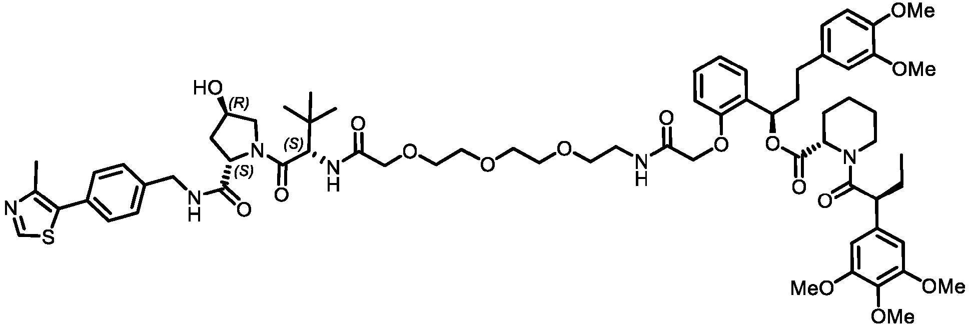 **(4),** or a pharmaceutically acceptable salt and stereoisomer thereof.

10. A pharmaceutical composition comprising a therapeutically effective amount of the bifunctional compound of any one of claims 1-9 or a pharmaceutically acceptable salt or stereoisomer thereof, and a pharmaceutically acceptable carrier.

11. The bifunctional compound of any one of claims 1-9 for use in a method of identifying protein function, comprising genetically modifying a cell by introducing an exogenous nucleic acid comprising a sequence that encodes FKBP12(F36V) at a genetic locus of an endogenous protein, wherein the thus modified locus expresses the protein with FKBP12(F36V) as an in-frame N-terminal or C-terminal fusion; contacting the modified cell with the bifunctional compound; and detecting a change in the modified cell relative to an unmodified cell, optionally wherein
the endogenous protein is mutated;
optionally wherein
the method is conducted *in vitro* or *in vivo* in a non-human animal,
further optionally wherein
the method is conducted *in vivo* in a murine model of disease or disorder; or
the cell is a human cancer cell line or a non-cancerous cell line.

12. The bifunctional compound of any one of claims 1-69 or a pharmaceutically acceptable salt or stereoisomer thereof, for use in a method of degrading a CAR protein, comprising:
administering to a subject an effective amount of the bifunctional compound, wherein the subject has previously been treated with allogeneic or autologous immune effector cells that express a nucleic acid encoding a fusion protein comprising the CAR and a dTAG FKBP12(F36V), wherein the fusion protein comprises, from N-terminus to C-terminus:
a) an extracellular ligand binding domain that binds a tumor associated antigen;
b) a transmembrane domain;
c) a cytoplasmic domain comprising at least one intracellular signaling domain; and
d) a dTAG FKBP12(F36V) of any one of SEQ ID NOs: 1-4 which is disposed at the N-terminus or between the extracellular binding domain and the transmembrane domain, provided that there is no disruption to antigen binding or insertion into the membrane; or at the C-terminus, between the transmembrane domain and the intracellular domain or between signaling domains when more than one is present, provided that there is no disruption to intracellular signaling or insertion into the membrane.

13. A compound, which is:

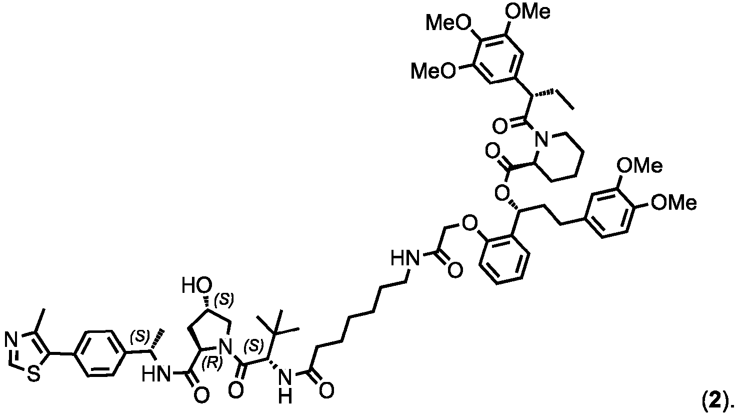

## Patentansprüche

1. Bifunktionale Verbindung, die eine Struktur aufweist, dargestellt durch Formel I: 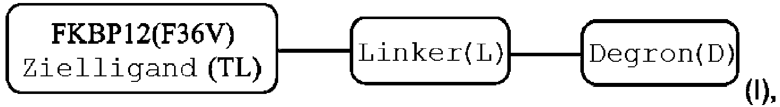
oder ein pharmazeutisch verträgliches Salz oder Stereoisomer davon,
wobei der Zielligand eine Struktur aufweist, dargestellt durch Formel TL-1: 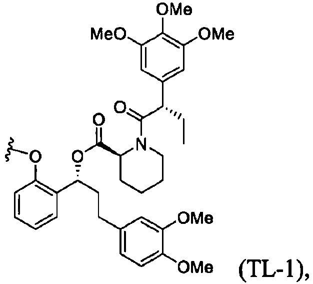
wobei der Linker eine Alkylenkette, die durch mindestens eines von -O-, -S-, -N(R')-, -C≡C-, -C(O)-, -C(O)O-, -OC(O)-, -OC(O)O-, -C(NOR')-, -C(O)N(R')-, -C(O)N(R')C(O)-, -C(O)N(R')C(O)N(R')-, -N(R')C(O)-, -N(R')C(O)N(R')-, -N(R')C(O)O-, -OC(O)N(R')-, - C(NR')-, -N(R')C(NR')-, -C(NR')N(R')-, -N(R')C(NR')N(R')-, - OB (Me) O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, - N (R')S (O)₂N(R') -, -N (R') S (O)N (R') -, C₃-C₁₂- Carbocyclen, 3- bis 12-gliedrigem Heterocyclen, 5- bis 12-gliedrigem Heteroarylen oder einer beliebigen Kombination davon unterbrochen und/oder (an einem Ende oder beiden Enden) beendet sein kann, wobei R' H oder C₁-C₆-Alkyl ist, wobei die unterbrechende Gruppe und die eine oder beide endständigen Gruppen gleich oder verschieden sein können,
oder eine Polyethylenglykolkette, die durch mindestens eines von -S-, -N(R')-, -C≡C-, -C(O)-, -C(O)O-, -OC(O)-, -OC(O)O-, - C(NOR')-, -C(O)N(R')-, -C(O)N(R')C(O)-, -C(O)N(R')C(O)N(R')-, - N(R')C(O)-, -N(R')C(O)N(R')-, -N(R')C(O)O-, -OC(O)N(R')-, - C(NR')-, -N(R')C(NR')-, -C(NR')N(R')-, -N(R')C(NR')N(R')-, - OB (Me) O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, - N(R')S(O)₂N(R')-, -N(R')S(O)N(R')-, C₃-₁₂- Carbocyclen, 3- bis 12-gliedrigem Heterocyclen, 5- bis 12-gliedrigem Heteroarylen oder eine beliebige Kombination davon unterbrochen und/oder (an einem Ende oder beiden Enden) beendet sein kann, wobei R' H oder C₁-C₆-Alkyl ist, wobei die eine oder beide endständigen Gruppen gleich oder verschieden sein können, oder dargestellt ist durch Struktur L10: 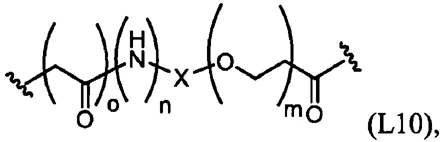
wobei m eine ganze Zahl von 0 bis 8 ist;
X fehlt oder C1-14-Alkyl ist;
n 0 oder 1 ist; und
o 0 oder 1 ist; und
wobei das Degron eine Struktur aufweist, dargestellt durch Formel D1 oder D2:

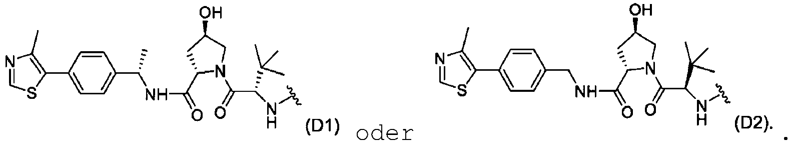

2. Bifunktionale Verbindung nach Anspruch 1, wobei der Linker durch Struktur L10 dargestellt ist: 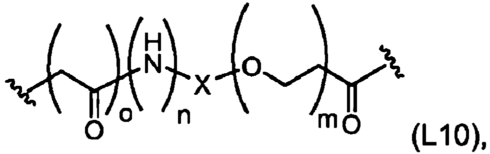
wobei m eine ganze Zahl von 0 bis 8 ist;
X fehlt oder C1-14-Alkyl ist;
n 0 oder 1 ist; und
o 0 oder 1 ist.

3. Bifunktionale Verbindung nach Anspruch 2, wobei der Linker durch eine Struktur dargestellt ist, ausgewählt aus der Gruppe, bestehend aus:

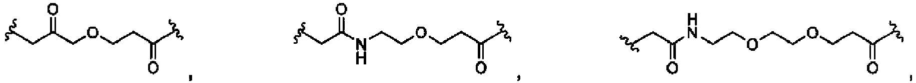

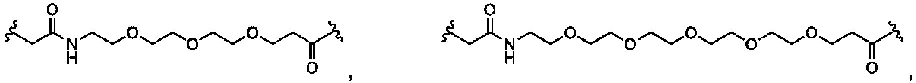

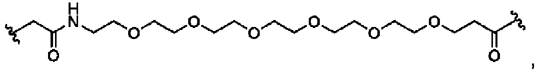

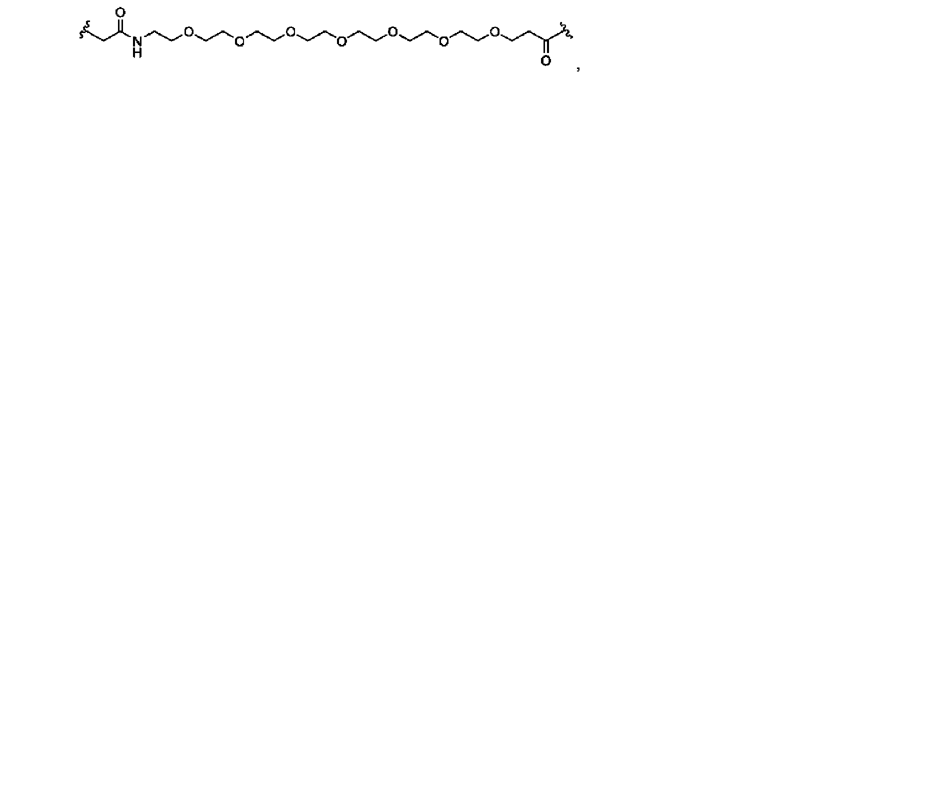

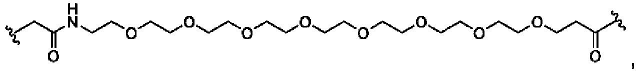

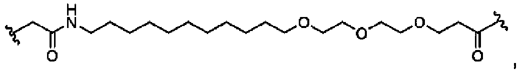

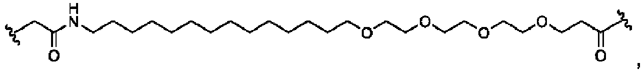

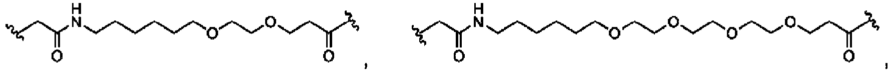

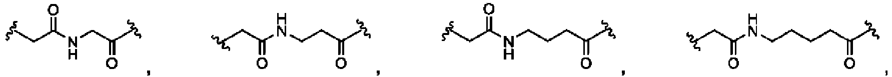

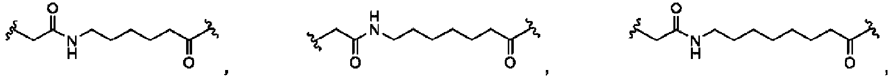

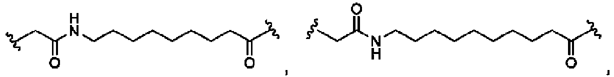

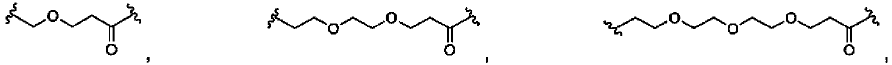

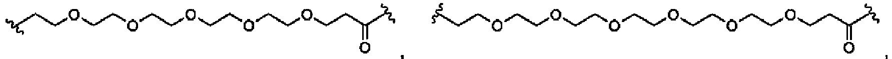

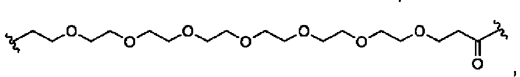

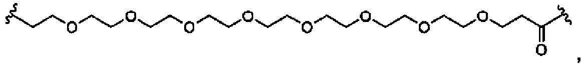

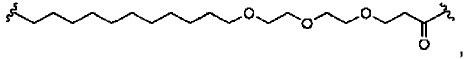

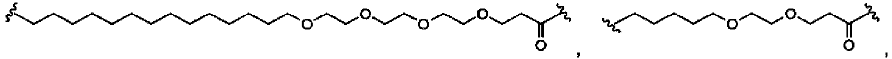

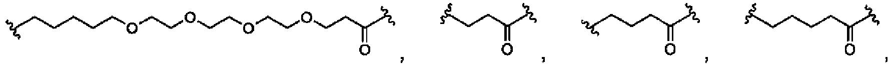

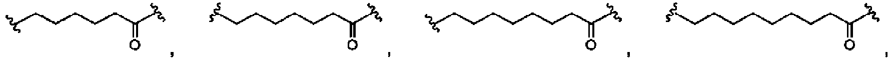

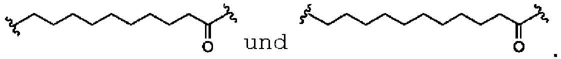

4. Bifunktionale Verbindung nach einem der Ansprüche 1 bis 3, wobei das Degron eine Struktur aufweist, dargestellt durch Formel D1:

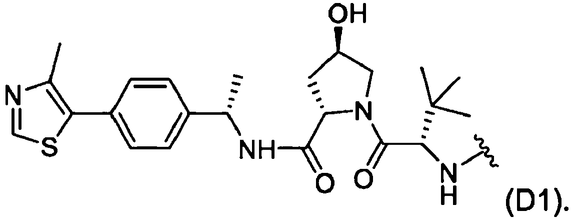

5. Bifunktionale Verbindungen nach einem der Ansprüche 1 bis 3, wobei das Degron eine Struktur aufweist, dargestellt durch Formel D2:

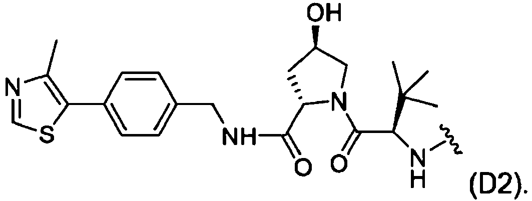

6. Bifunktionale Verbindung nach Anspruch 1, dargestellt durch eine Struktur, ausgewählt aus der Gruppe, bestehend aus: 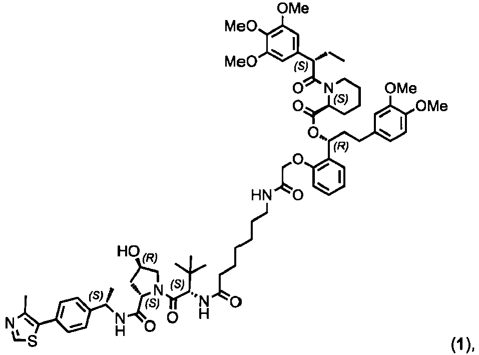 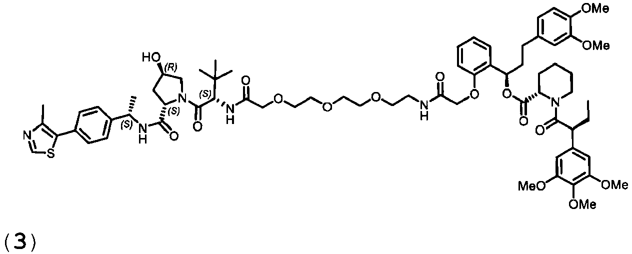 und 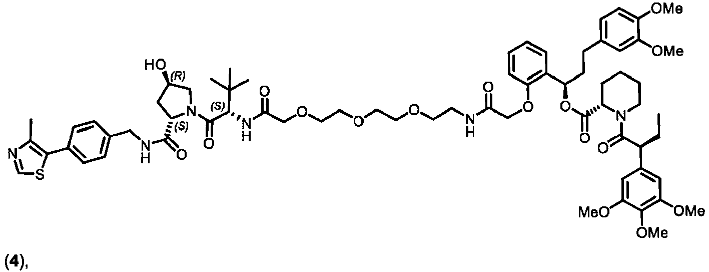 oder ein pharmazeutisch verträgliches Salz und Stereoisomer davon.

7. Bifunktionale Verbindung nach Anspruch 6, die Folgendes ist: 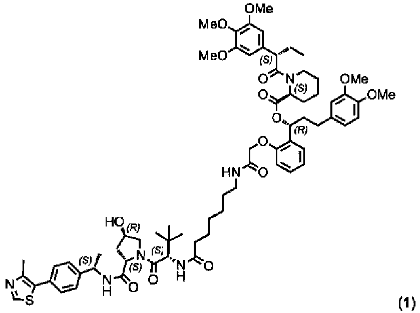 oder ein pharmazeutisch verträgliches Salz und Stereoisomer davon.

8. Bifunktionale Verbindung nach Anspruch 6, die Folgendes ist: 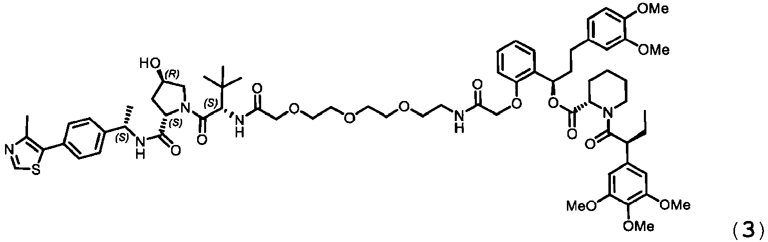 oder ein pharmazeutisch verträgliches Salz und Stereoisomer davon.

9. Bifunktionale Verbindung nach Anspruch 6, die Folgendes ist: 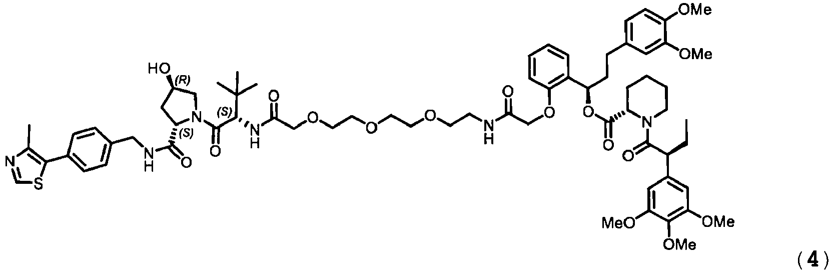 oder ein pharmazeutisch verträgliches Salz und Stereoisomer davon.

10. Pharmazeutische Zusammensetzung, umfassend eine therapeutisch wirksame Menge der bifunktionalen Verbindung nach einem der Ansprüche 1 bis 9 oder eines pharmazeutisch verträglichen Salzes oder Stereoisomers davon und einen pharmazeutisch verträglichen Träger.

11. Bifunktionale Verbindung nach einem der Ansprüche 1 bis 9 zur Verwendung in einem Verfahren zum Erkennen einer Proteinfunktion, umfassend gentechnisches Verändern einer Zelle durch Einführen einer exogenen Nukleinsäure, umfassend eine Sequenz, die FKBP12(F36V) an einem Genlocus eines endogenen Proteins codiert, wobei der derart modifizierte Locus das Protein mit FKBP12(F36V) als in-frame N-terminale oder C-terminale Fusion exprimiert; Inkontaktbringen der modifizierten Zelle mit der bifunktionalen Verbindung; und Nachweisen einer Veränderung der modifizierten Zelle relativ zu einer unmodifizierten Zelle, wobei optional
das endogene Protein mutiert ist;
wobei optional
das Verfahren *in vitro* oder *in vivo* an einem nicht humanen Tier durchgeführt wird,
wobei ferner optional
das Verfahren *in vivo* in einem murinen Modell der Krankheit oder Störung durchgeführt wird; oder
es sich bei der Zelle um eine humane Krebszelllinie oder eine nicht krebsartige Zelllinie handelt.

12. Bifunktionale Verbindung nach einem der Ansprüche 1 bis 69 oder ein pharmazeutisch verträgliches Salz oder Stereoisomer davon zur Verwendung in einem Verfahren zum Abbauen eines CAR-Proteins, umfassend:
Verabreichen einer wirksamen Menge der bifunktionalen Verbindung an ein Subjekt, wobei das Subjekt zuvor mit allogenen oder autologen Immuneffektorzellen behandelt wurde, die eine Nukleinsäure exprimieren, die ein Fusionsprotein codiert, umfassend das CAR und ein dTAG FKBP12(F36V), wobei das Fusionsprotein von dem N-Terminus zu dem C-Terminus Folgendes umfasst:
a) eine extrazelluläre Ligandenbindungsdomäne, die ein tumorassoziiertes Antigen bindet;
b) eine Transmembrandomäne;
c) eine cytoplasmatische Domäne, umfassend mindestens eine intrazelluläre Signaldomäne; und
d) ein dTAG FKBP12 (F36V) einer beliebigen von SEQ ID NO: 1-4, das an dem N-Terminus oder zwischen der extrazellulären Bindungsdomäne und der Transmembrandomäne angeordnet ist, mit der Maßgabe, dass die Antigenbindung oder -insertion in die Membran nicht beeinträchtigt ist; oder an dem C-Terminus zwischen der Transmembrandomäne und der intrazellulären Domäne oder zwischen Signaldomänen, wenn mehr als eine vorhanden ist, mit der Maßgabe, dass die intrazelluläre Signalierung oder eine Insertion in die Membran nicht beeinträchtigt ist.

13. Verbindung, die Folgendes ist:

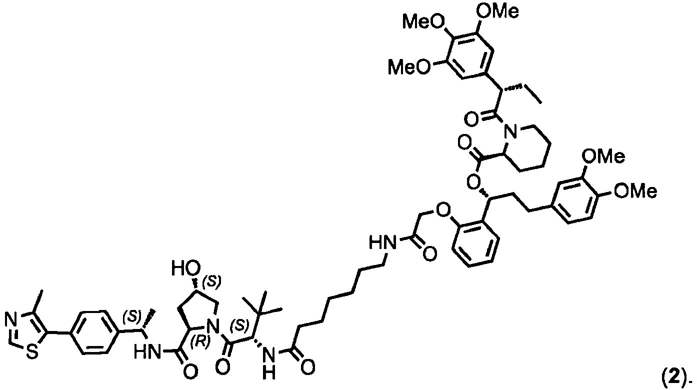

## Revendications

1. Composé bifonctionnel ayant une structure représentée par la formule I : 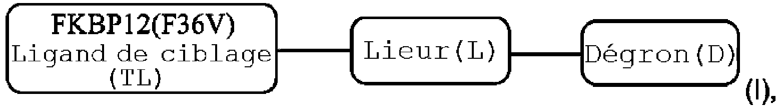
ou un sel ou stéréoisomère pharmaceutiquement acceptable de celui-ci,
dans lequel le ligand de ciblage a une structure représentée par la formule TL-1 : 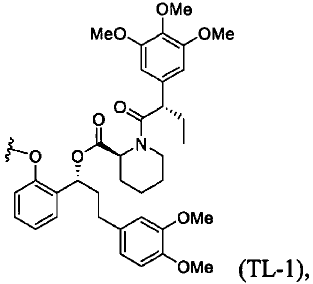
dans lequel le lieur est une chaîne alkylène qui peut être interrompue et/ou se terminer (à l'une ou aux deux extrémités) par au moins un des groupements suivants : -O-, -S-, -N(R')-, - C≡C-, -C(O)-, -C(O)O-, -OC(O)-, -OC(O)O-, -C(NOR')-, -C(O)N(R')-, -C(O)N(R')C(O)-, -C(O)N(R')C(O)N(R')-, -N(R')C(O)-, - N(R')C(O)N(R')-, -N(R')C(O)O-, -OC(O)N(R')-, -C(NR')-, - N(R')C(NR')-, -C(NR')N(R')-, -N(R')C(NR')N(R')-, -OB(Me)O-, - S(O)₂-, -OS(O)-, -S(O)O-, -S(O)-, -OS(O)₂-, -S(O)₂O-, - N(R')S(O)₂-, -S(O)₂N(R')-, -N(R')S(O)-, -S(O)N(R')-, - N(R') S(O)₂N(R')-, -N(R')S(O)N(R')-, carbocyclène en C₃-C₁₂, hétérocyclène à 3 à 12 chaînons, hétéroarylène à 5 à 12 chaînons ou toute combinaison de ceux-ci, dans lequel R' est H ou alkyle en C₁-C₆, dans lequel le groupe interrompant et l'un ou les deux groupes terminaux peuvent être identiques ou différents, ou une chaîne de polyéthylène glycol qui peut être interrompue et/ou se terminer (à l'une ou aux deux extrémités) par au moins un des groupements suivants : -S-, -N(R')-, -C≡C-, -C(O)-, -C(O)O-, - OC(O)-, -OC(O)O-, -C(NOR')-, -C(O)N(R')-, -C(O)N(R')C(O)-, - C(O)N(R')C(O)N(R')-, -N(R')C(O)-, -N(R')C(O)N(R')-, - N(R')C(O)O-, -OC(O)N(R')-, -C(NR')-, -N(R')C(NR')-, - C(NR')N(R')-, -N(R')C(NR')N(R')-, -OB(Me)O-, -S(O)₂-, -OS(O)-, -S(O)O-, -S(O)-, -OS(O)₂-, -S(O)₂O-, -N(R')S(O)₂-, -S(O)₂N(R')-, -N(R')S(O)-, -S(O)N(R')-, -N(R')S(O)₂N)R')-, -N(R')S(O)N(R')-, carbocyclène en C₃-₁₂, hétérocyclène à 3 à 12 chaînons, hétéroarylène à 5 à 12 chaînons ou toute combinaison de ceux-ci, où R' est H ou alkyle en C₁-C₆, dans lequel l'un ou les deux groupes terminaux peuvent être identiques ou différents, ou représenté par la structure L10 : 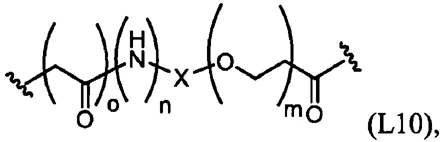
dans lequel m est un entier compris entre 0 et 8 ;
X est absent ou alkyle en C1 à 14 ;
n vaut 0 ou 1 ; et
o vaut 0 ou 1 ; et
dans lequel le dégron a une structure représentée par la formule D1 ou D2 :

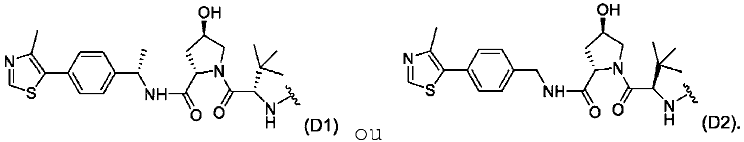

2. Composé bifonctionnel selon la revendication 1, dans lequel le lieur est représenté par la structure L10 : 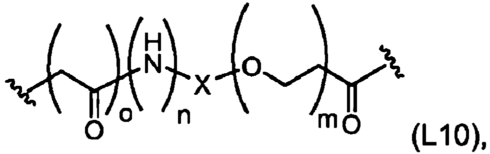
dans lequel m est un entier compris entre 0 et 8 ;
X est absent ou alkyle en C1 à 14 ;
n vaut 0 ou 1 ; et
o vaut 0 ou 1.

3. Composé bifonctionnel selon la revendication 2, dans lequel le lieur est représenté par une structure choisie dans le groupe constitué de :

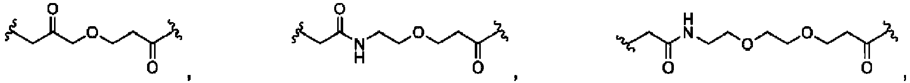

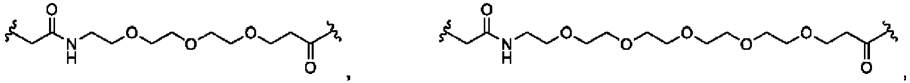

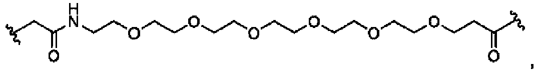

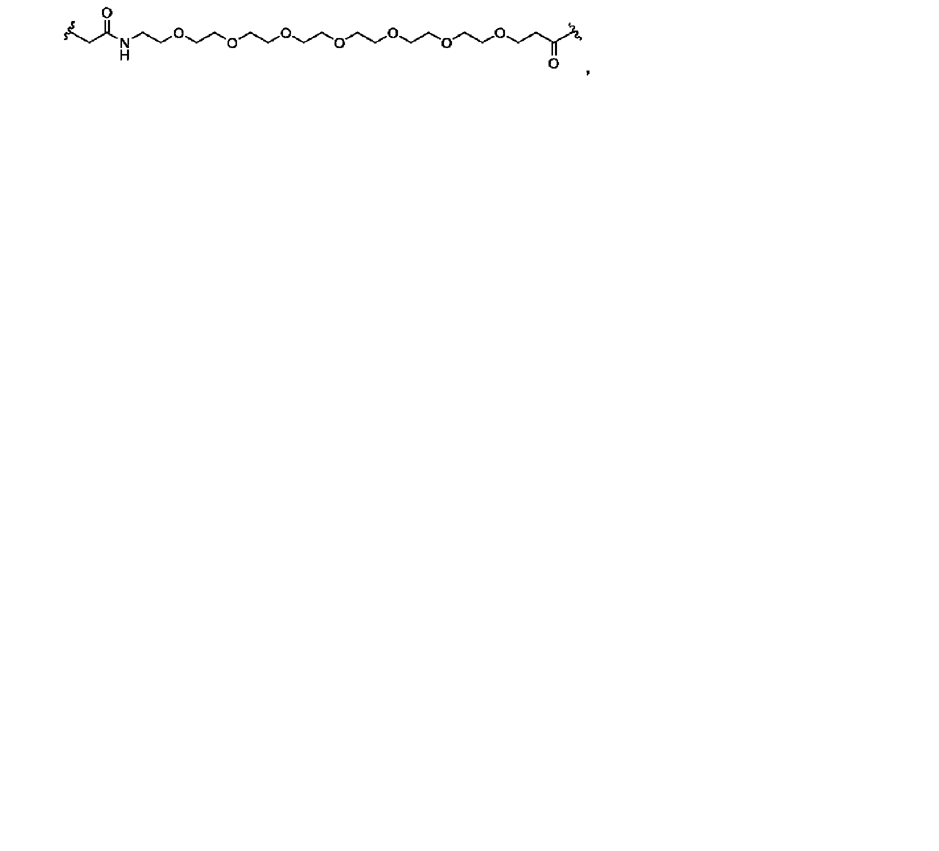

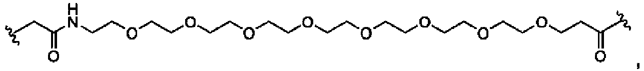

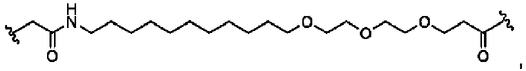

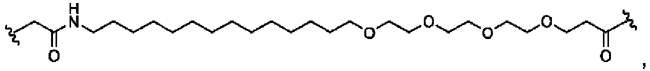

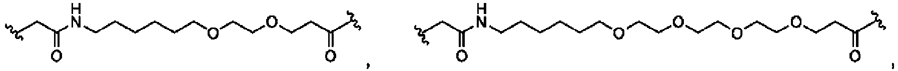

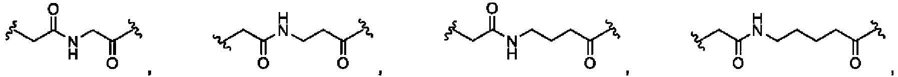

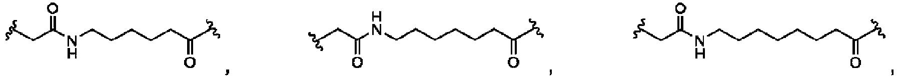

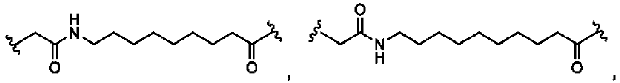

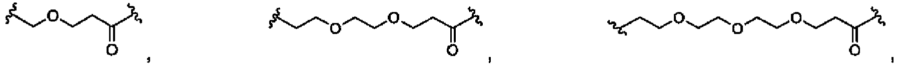

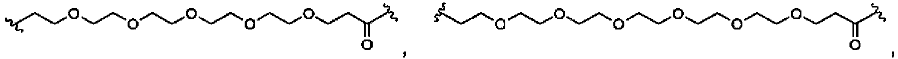

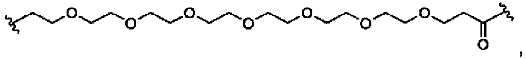

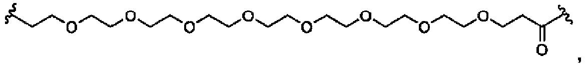

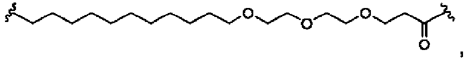

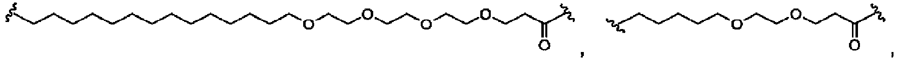

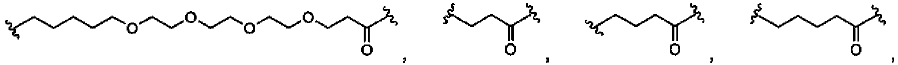

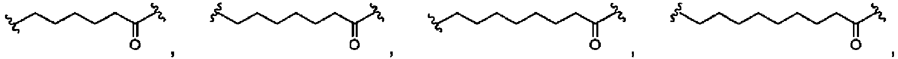

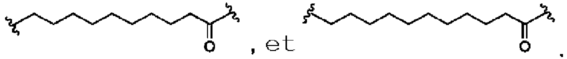

4. Composé bifonctionnel selon l'une quelconque des revendications 1 à 3, dans lequel le dégron a une structure représentée par la formule D1 :

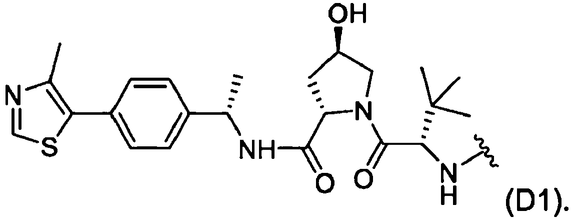

5. Composés bifonctionnels selon l'une quelconque des revendications 1 à 3, dans lesquels le dégron a une structure représentée par la formule D2 :

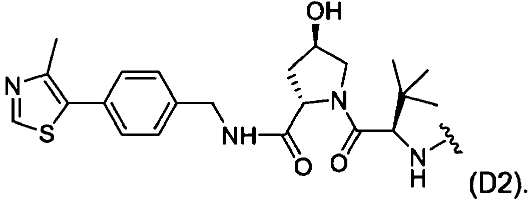

6. Composé bifonctionnel selon la revendication 1, qui est représenté par une structure choisie dans le groupe constitué de : 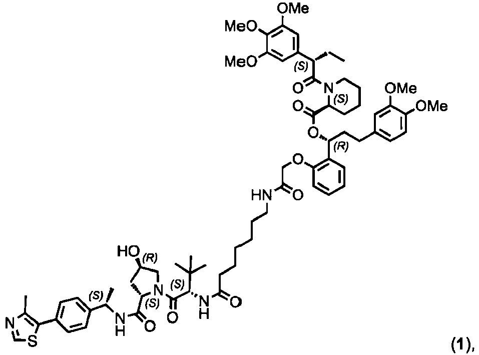 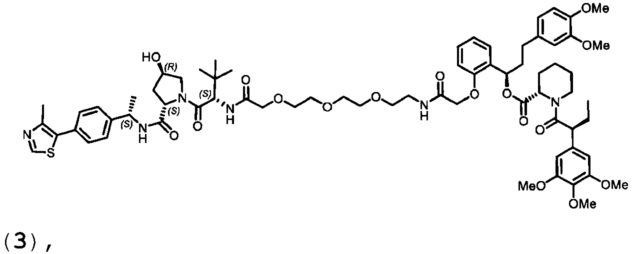 et 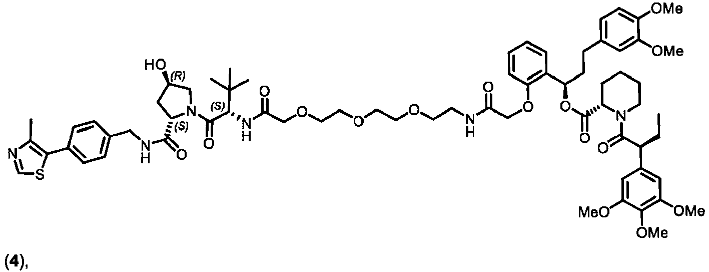 ou un sel et un stéréoisomère pharmaceutiquement acceptable de celui-ci.

7. Composé bifonctionnel selon la revendication 6, qui est : 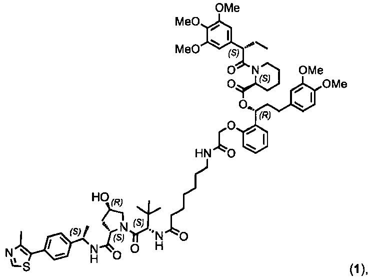 ou un sel et un stéréoisomère pharmaceutiquement acceptable de celui-ci.

8. Composé bifonctionnel selon la revendication 6, qui est : 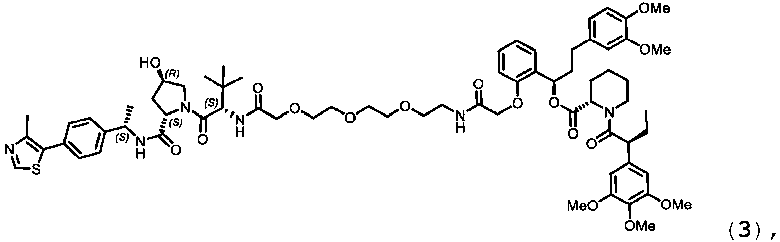 ou un sel et un stéréoisomère pharmaceutiquement acceptable de celui-ci.

9. Composé bifonctionnel selon la revendication 6, qui est : 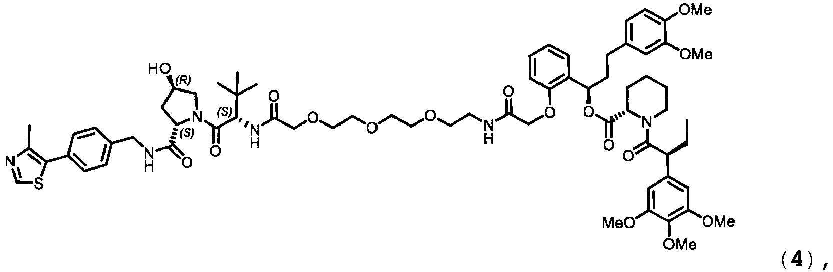 ou un sel et un stéréoisomère pharmaceutiquement acceptable de celui-ci.

10. Composition pharmaceutique, comprenant une quantité thérapeutiquement efficace du composé bifonctionnel selon l'une quelconque des revendications 1 à 9 ou un sel ou stéréoisomère pharmaceutiquement acceptable de celui-ci, et un support pharmaceutiquement acceptable.

11. Composé bifonctionnel selon l'une quelconque des revendications 1 à 9 pour une utilisation dans un procédé d'identification de la fonction protéique, comprenant la modification génétique d'une cellule par introduction d'un acide nucléique exogène comprenant une séquence qui code pour FKBP12(F36V) au niveau d'un locus génétique d'une protéine endogène, dans lequel le locus ainsi modifié exprime la protéine avec FKBP12(F36V) sous forme de fusion N-terminale ou C-terminale en phase ; la mise en contact de la cellule modifiée avec le composé bifonctionnel ; et la détection d'un changement dans la cellule modifiée par rapport à une cellule non modifiée, éventuellement dans lequel
la protéine endogène est mutée ;
éventuellement dans lequel
le procédé est mis en œuvre *in vitro* ou *in vivo* sur un animal non humain,
en outre éventuellement dans lequel
le procédé est mis en œuvre *in vivo* dans un modèle murin de maladie ou
de trouble ; ou
la cellule est une lignée cellulaire cancéreuse humaine ou une lignée cellulaire non cancéreuse.

12. Composé bifonctionnel selon l'une quelconque des revendications 1 à 69 ou un sel ou stéréoisomère pharmaceutiquement acceptable de celui-ci, pour une utilisation dans un procédé de dégradation d'une protéine CAR, comprenant :
l'administration à un sujet d'une quantité efficace du composé bifonctionnel, dans lequel le sujet a préalablement été traité par des cellules effectrices immunitaires allogéniques ou autologues qui expriment un acide nucléique codant pour une protéine de fusion comprenant le CAR et un dTAG FKBP12(F36V), dans lequel la protéine de fusion comprend, de l'extrémité N-terminale à l'extrémité C-terminale :
a) un domaine de liaison de ligand extracellulaire qui se lie à un antigène associé à une tumeur ;
b) un domaine transmembranaire ;
c) un domaine cytoplasmique comprenant au moins un domaine de signalisation intracellulaire ; et
d) un dTAG FKBP12 (F36V) de l'un des SEQ ID NOs: 1-4 qui est disposé à l'extrémité N-terminale ou entre le domaine de liaison extracellulaire et le domaine transmembranaire, à condition qu'il n'y ait pas de perturbation de la liaison à l'antigène ou de l'insertion dans la membrane ; ou à l'extrémité C-terminale, entre le domaine transmembranaire et le domaine intracellulaire ou entre les domaines de signalisation lorsqu'il y en a plus d'un, à condition qu'il n'y ait pas de perturbation de la signalisation intracellulaire ou de l'insertion dans la membrane.

13. Composé, qui est :
